# EUROPEAN PATENT SPECIFICATION

(11) **EP 2 986 629 B1**
(45) Date of publication and mention of the grant of the patent: **08.11.2017**
(21) Application number: 14723894.3
(22) Date of filing: 10.04.2014
(51) Int. Cl.: C07K 14/435, C12N 1/00, A61K 48/00

(54) **MITOCHONDRIAL PROTEINS CONSTRUCTS AND USES THEREOF**
MITOCHONDRIALE PROTEINKONSTRUKTE UND VERWENDUNGEN DAVON
CONSTRUCTIONS DE PROTÉINES MITOCHONDRIALES ET LEURS UTILISATIONS

(30) Priority: 15.04.2013 US 201361811934 P; 26.08.2013 US 201361869981 P; 23.09.2013 US 201314034224
(43) Date of publication of application: 24.02.2016
(73) Proprietor: Bio Blast Pharma Ltd., 6522042 Tel Aviv (IL)
(72) Inventor: LORBERBOUM-GALSKI, Haya, 9789017 Jerusalem (IL); GREIF, Hagar, 7407232 Ness Ziona (IL)
(74) Representative: Cooley (UK) LLP
(86) International application number: PCT/IL2014/050354
(87) International publication number: WO 2014/170896

(56) References cited:
- WO-A2-2009/098682
- C. A. KOCZOR ET AL: "Mitochondrial DNA Damage Initiates a Cell Cycle Arrest by a Chk2-associated Mechanism in Mammalian Cells", JOURNAL OF BIOLOGICAL CHEMISTRY, vol. 284, no. 52, 19 October 2009 (2009-10-19), pages 36191-36201, XP055127240, ISSN: 0021-9258, DOI: 10.1074/jbc.M109.036020
- DEL GAIZO V ET AL: "Targeting proteins to mitochondria using TAT", MOLECULAR GENETICS AND METABOLISM, ACADEMIC PRESS, AMSTERDAM, NL, vol. 80, no. 1-2, 1 September 2003 (2003-09-01), pages 170-180, XP002986585, ISSN: 1096-7192, DOI: 10.1016/J.YMGME.2003.08.017

### SEQUENCE LISTING

<110> Bioblast Pharma Ltd.
<120> Mitochondrial Proteins Constructs and Uses Thereof
<130> 2266197
<150> US 61/811,934
   <151> 2013-04-15
<150> US 14/034,224
   <151> 2013-09-23
<150> US 61/869,981
   <151> 2013-08-26
<160> 54
<170> PatentIn version 3.5
<210> 1
   <211> 27
   <212> DNA
   <213> Human immunodeficiency virus type 1
<400> 1
   aggaagaagc ggagacagcg acgaaga 27
<210> 2
   <211> 237
   <212> DNA
   <213> Homo sapiens
<400> 2
   tggactctcg ggcgccgcgc agtagccggc ctcctggcgt cacccagccc ggcccaggcc 60
   cagaccctca cccgggtccc gcggccggca gagttggccc cactctgcgg ccgccgtggc 120
   ctgcgcaccg acatcgatgc gacctgcacg ccccgccgcg caagttcgaa ccaacgtggc 180
   ctcaaccaga tttggaatgt caaaaagcag agtgtctatt tgatgaattt gaggaaa 237
<210> 3
   <211> 78
   <212> DNA
   <213> Homo sapiens
<400> 3
   gctttactta ctgcggccgc ccggctcttg ggaaccaaga atgcatcttg tcttgttctt        60
   gcagcccggc atgccagt                                                      78
<210> 4
   <211> 102
   <212> DNA
   <213> Homo sapiens
<400> 4
   ggagcactag tgattcgcgg tatcaggaat ttcaacctag agaaccgagc ggaacgggaa        60
   atcagcaaga tgaagccctc tgtcgctccc agacacccct ct                          102
<210> 5
   <211> 105
   <212> DNA
   <213> Homo sapiens
<400> 5
   cagagctgga gtcgtgtgta ctgctccttg gccaagagag gccatttcaa tcgaatatct        60
   catggcctac agggactttc tgcagtgcct ctgagaactt acgca                       105
<210> 6
   <211> 393
   <212> DNA
   <213> Homo sapiens
<400> 6
   tctggaactt tgggccaccc aggctctcta gatgagacca cctatgaaag actagcagag        60
   gaaacgctgg actctttagc agagtttttt gaagaccttg cagacaagcc atacacgttt       120
   gaggactatg atgtctcctt tgggagtggt gtcttaactg tcaaactggg tggagatcta       180
   ggaacctatg tgatcaacaa gcagacgcca aacaagcaaa tctggctatc ttctccatcc       240
   agtggaccta agcgttatga ctggactggg aaaaactggg tgtactccca cgacggcgtg       300
   tccctccatg agctgctggc cgcagagctc actaaagcct taaaaaccaa actggacttg       360
   tcttccttgg cctattccgg aaaagatgct tga                                    393
<210> 7
   <211> 27
   <212> DNA
   <213> Artificial Sequence
<220>
   <223> forward primer for precursor frataxin cloning
<400> 7
   cgcggatccg tggactctcg ggcgccg 27
<210> 8
   <211> 33
   <212> DNA
   <213> Artificial Sequence
<220>
   <223> reverse primer for precursor frataxin cloning
<400> 8
   acgctcgagt caagcatctt ttccggaata ggc 33
<210> 9
   <211> 30
   <212> DNA
   <213> Artificial Sequence
<220>
   <223> forward primer for MTS lad cloning
<400> 9
   cgcggatcca cagagctgga gtcgtgtgta 30
<210> 10
   <211> 66
   <212> DNA
   <213> Artificial Sequence
<220>
   <223> reverse primer for MTS lad cloning
<400> 10
   cataggtggt ctcatctaga gagcctgggt ggcccaaagt tccagatgcg taagttctca 60
   gaggca 66
<210> 11
   <211> 28
   <212> DNA
   <213> Artificial Sequence
<220>
   <223> forward primer for MTS orf cloning
<400> 11
   cgcggatccg ggagcactag tgattcgc 28
<210> 12
   <211> 66
   <212> DNA
   <213> Artificial Sequence
<220>
   <223> reverse primer for MTS orf cloning
<400> 12
   cataggtggt ctcatctaga gagcctgggt ggcccaaagt tccagaagag gggtgtctgg 60
   gagcga 66
<210> 13
   <211> 112
   <212> DNA
   <213> Artificial Sequence
<220>
   <223> forward oligo for MTS cs cloning
<400> 13
   gatccggctt tacttactgc ggccgcccgg ctcttgggaa ccaagaatgc atcttgtctt 60
   gttcttgcag cccggcatgc cagttctgga actttgggcc acccaggctc tc 112
<210> 14
   <211> 112
   <212> DNA
   <213> Artificial Sequence
<220>
   <223> reverse primer for MTS cs cloning
<400> 14
   tctagagagc ctgggtggcc caaagttcca gaactggcat gccgggctgc aagaacaaga 60
   caagatgcat tcttggttcc caagagccgg gcggccgcag taagtaaagc cg 112
<210> 15
   <211> 957
   <212> DNA
   <213> Homo sapiens
<400> 15
   ctgaagggcc gtgaccttct cactctaaga aactttaccg gagaagaaat taaatatatg        60
   ctatggctat cagcagatct gaaatttagg ataaaacaga aaggagagta tttgccttta       120
   ttgcaaggga agtccttagg catgattttt gagaaaagaa gtactcgaac aagattgtct       180
   acagaaacag gctttgcact tctgggagga catccttgtt ttcttaccac acaagatatt       240
   catttgggtg tgaatgaaag tctcacggac acggcccgtg tattgtctag catggcagat       300
   gcagtattgg ctcgagtgta taaacaatca gatttggaca ccctggctaa agaagcatcc       360
   atcccaatta tcaatgggct gtcagatttg taccatccta tccagatcct ggctgattac       420
   ctcacgctcc aggaacacta tagctctctg aaaggtctta ccctcagctg gatcggggat       480
   gggaacaata tcctgcactc catcatgatg agcgcagcga aattcggaat gcaccttcag       540
   gcagctactc caaagggtta tgagccggat gctagtgtaa ccaagttggc agagcagtat       600
   gccaaagaga atggtaccaa gctgttgctg acaaatgatc cattggaagc agcgcatgga       660
   ggcaatgtat taattacaga cacttggata agcatgggac aagaagagga gaagaaaaag       720
   cggctccagg ctttccaagg ttaccaggtt acaatgaaga ctgctaaagt tgctgcctct       780
   gactggacat ttttacactg cttgcccaga aagccagaag aagtggatga tgaagtcttt        840
   tattctcctc gatcactagt gttcccagag gcagaaaaca gaaagtggac aatcatggct       900
   gtcatggtgt ccctgctgac agattactca cctcagctcc agaagcctaa attttga         957
<210> 16
   <211> 159
   <212> DNA
   <213> Homo sapiens
<400> 16
   atgtaccgct acctggccaa agcgctgctg ccgtcccggg ccgggcccgc tgccctgggc        60
   tccgcggcca accactcggc cgcgttgctg ggccggggcc gcggacagcc cgccgccgcc       120
   tcgcagccgg ggctcgcatt ggccgcccgg cgccactac                              159
<210> 17
   <211> 190
   <212> PRT
   <213> Artificial Sequence
<220>
   <223> His TAT MTS(cs) 81-210 FRA
<400> 17
<210> 18
   <211> 243
   <212> PRT
   <213> Artificial Sequence
<220>
   <223> HTFrataxin [His TAT MTS(fra)81-210 FRA]
<400> 18
   Met Gly Ser Ser His His His His His His Ser Ser Gly Leu Val Pro
   1               5                   10                  15
   Arg Gly Ser His Met Arg Lys Lys Arg Arg Gln Arg Arg Arg Gly Ser
               20                  25                  30
   Asp Pro Trp Thr Leu Gly Arg Arg Ala Val Ala Gly Leu Leu Ala Ser
           35                  40                  45
   Pro Ser Pro Ala Gln Ala Gln Thr Leu Thr Arg Val Pro Arg Pro Ala
       50                  55                  60
   Glu Leu Ala Pro Leu Cys Gly Arg Arg Gly Leu Arg Thr Asp Ile Asp
   65                  70                  75                  80
   Ala Thr Cys Thr Pro Arg Arg Ala Ser Ser Asn Gln Arg Gly Leu Asn
                   85                  90                  95
   Gln Ile Trp Asn Val Lys Lys Gln Ser Val Tyr Leu Met Asn Leu Arg
               100                 105                 110
   Lys Ser Gly Thr Leu Gly His Pro Gly Ser Leu Asp Glu Thr Thr Tyr
           115                 120                 125
   Glu Arg Leu Ala Glu Glu Thr Leu Asp Ser Leu Ala Glu Phe Phe Glu
       130                 135                 140
   Asp Leu Ala Asp Lys Pro Tyr Thr Phe Glu Asp Tyr Asp Val Ser Phe
   145                 150                 155                 160
   Gly Ser Gly Val Leu Thr Val Lys Leu Gly Gly Asp Leu Gly Thr Tyr
                   165                 170                 175
   Val Ile Asn Lys Gln Thr Pro Asn Lys Gln Ile Trp Leu Ser Ser Pro
               180                 185                 190
   Ser Ser Gly Pro Lys Arg Tyr Asp Trp Thr Gly Lys Asn Trp Val Tyr
           195                 200                 205
   Ser His Asp Gly Val Ser Leu His Glu Leu Leu Ala Ala Glu Leu Thr
       210                 215                 220
   Lys Ala Leu Lys Thr Lys Leu Asp Leu Ser Ser Leu Ala Tyr Ser Gly
   225                 230                 235                 240
   Lys Asp Ala
<210> 19
   <211> 199
   <212> PRT
   <213> Artificial Sequence
<220>
   <223> His TAT MTS(lad) 81-210 FRA
<400> 19
   Met Gly Ser Ser His His His His His His Ser Ser Gly Leu Val Pro
   1               5                   10                  15
   Arg Gly Ser His Met Arg Lys Lys Arg Arg Gln Arg Arg Arg Gly Ser
               20                  25                  30
   Asp Pro Gln Ser Trp Ser Arg Val Tyr Cys Ser Leu Ala Lys Arg Gly
           35                  40                  45
   His Phe Asn Arg Ile Ser His Gly Leu Gln Gly Leu Ser Ala Val Pro
       50                  55                  60
   Leu Arg Thr Tyr Ala Ser Gly Thr Leu Gly His Pro Gly Ser Leu Asp
   65                  70                  75                  80
   Glu Thr Thr Tyr Glu Arg Leu Ala Glu Glu Thr Leu Asp Ser Leu Ala
                   85                  90                  95
   Glu Phe Phe Glu Asp Leu Ala Asp Lys Pro Tyr Thr Phe Glu Asp Tyr
               100                 105                 110
   Asp Val Ser Phe Gly Ser Gly Val Leu Thr Val Lys Leu Gly Gly Asp
           115                 120                 125
   Leu Gly Thr Tyr Val Ile Asn Lys Gln Thr Pro Asn Lys Gln Ile Trp
       130                 135                 140
   Leu Ser Ser Pro Ser Ser Gly Pro Lys Arg Tyr Asp Trp Thr Gly Lys
   145                 150                 155                 160
   Asn Trp Val Tyr Ser His Asp Gly Val Ser Leu His Glu Leu Leu Ala
                   165                 170                 175
   Ala Glu Leu Thr Lys Ala Leu Lys Thr Lys Leu Asp Leu Ser Ser Leu
               180                 185                 190
   Ala Tyr Ser Gly Lys Asp Ala
           195
<210> 20
   <211> 198
   <212> PRT
   <213> Artificial Sequence
<220>
   <223> His TAT MTS(orf) 81-210 FRA
<400> 20
   Met Gly Ser Ser His His His His His His Ser Ser Gly Leu Val Pro
   1               5                   10                  15
   Arg Gly Ser His Met Arg Lys Lys Arg Arg Gln Arg Arg Arg Gly Ser
               20                  25                  30
   Asp Pro Gly Ala Leu Val Ile Arg Gly Ile Arg Asn Phe Asn Leu Glu
           35                  40                  45
   Asn Arg Ala Glu Arg Glu Ile Ser Lys Met Lys Pro Ser Val Ala Pro
       50                  55                  60
   Arg His Pro Ser Ser Gly Thr Leu Gly His Pro Gly Ser Leu Asp Glu
   65                  70                  75                  80
   Thr Thr Tyr Glu Arg Leu Ala Glu Glu Thr Leu Asp Ser Leu Ala Glu
                   85                  90                  95
   Phe Phe Glu Asp Leu Ala Asp Lys Pro Tyr Thr Phe Glu Asp Tyr Asp
               100                 105                 110
   Val Ser Phe Gly Ser Gly Val Leu Thr Val Lys Leu Gly Gly Asp Leu
           115                 120                 125
   Gly Thr Tyr Val Ile Asn Lys Gln Thr Pro Asn Lys Gln Ile Trp Leu
       130                 135                 140
   Ser Ser Pro Ser Ser Gly Pro Lys Arg Tyr Asp Trp Thr Gly Lys Asn
   145                 150                 155                 160
   Trp Val Tyr Ser His Asp Gly Val Ser Leu His Glu Leu Leu Ala Ala
                   165                 170                 175
   Glu Leu Thr Lys Ala Leu Lys Thr Lys Leu Asp Leu Ser Ser Leu Ala
   180 185 190
   Tyr Ser Gly Lys Asp Ala
   195
<210> 21
   <211> 11
   <212> PRT
   <213> Human immunodeficiency virus type 1
<400> 21
   Tyr Gly Arg Lys Lys Arg Arg Gln Arg Arg Arg
   1 5 10
<210> 22
   <211> 79
   <212> PRT
   <213> Homo sapiens
<400> 22
   Trp Thr Leu Gly Arg Arg Ala Val Ala Gly Leu Leu Ala Ser Pro Ser
   1 5 10 15
   Pro Ala Gln Ala Gln Thr Leu Thr Arg Val Pro Arg Pro Ala Glu Leu
   20 25 30
   Ala Pro Leu Cys Gly Arg Arg Gly Leu Arg Thr Asp Ile Asp Ala Thr
   35 40 45
   Cys Thr Pro Arg Arg Ala Ser Ser Asn Gln Arg Gly Leu Asn Gln Ile
   50 55 60
   Trp Asn Val Lys Lys Gln Ser Val Tyr Leu Met Asn Leu Arg Lys
   65 70 75
<210> 23
   <211> 26
   <212> PRT
   <213> Homo sapiens
<400> 23
   Ala Leu Leu Thr Ala Ala Ala Arg Leu Leu Gly Thr Lys Asn Ala Ser
   1 5 10 15
   Cys Leu Val Leu Ala Ala Arg His Ala Ser
   20 25
<210> 24
   <211> 35
   <212> PRT
   <213> Homo sapiens
<400> 24
   Gln Ser Trp Ser Arg Val Tyr Cys Ser Leu Ala Lys Arg Gly His Phe
   1               5                   10                  15
   Asn Arg Ile Ser His Gly Leu Gln Gly Leu Ser Ala Val Pro Leu Arg
               20                  25                  30
   Thr Tyr Ala
           35
<210> 25
   <211> 34
   <212> PRT
   <213> Homo sapiens
<400> 25
   Gly Ala Leu Val Ile Arg Gly Ile Arg Asn Phe Asn Leu Glu Asn Arg
   1               5                   10                  15
   Ala Glu Arg Glu Ile Ser Lys Met Lys Pro Ser Val Ala Pro Arg His
               20                  25                  30
   Pro Ser
<210> 26
   <211> 130
   <212> PRT
   <213> Homo sapiens
<400> 26
   Ser Gly Thr Leu Gly His Pro Gly Ser Leu Asp Glu Thr Thr Tyr Glu
   1               5                   10                  15
   Arg Leu Ala Glu Glu Thr Leu Asp Ser Leu Ala Glu Phe Phe Glu Asp
               20                  25                  30
   Leu Ala Asp Lys Pro Tyr Thr Phe Glu Asp Tyr Asp Val Ser Phe Gly
           35                  40                  45
   Ser Gly Val Leu Thr Val Lys Leu Gly Gly Asp Leu Gly Thr Tyr Val
       50                  55                  60
   Ile Asn Lys Gln Thr Pro Asn Lys Gln Ile Trp Leu Ser Ser Pro Ser
   65                  70                  75                  80
   Ser Gly Pro Lys Arg Tyr Asp Trp Thr Gly Lys Asn Trp Val Tyr Ser
                   85                  90                  95
   His Asp Gly Val Ser Leu His Glu Leu Leu Ala Ala Glu Leu Thr Lys
               100                 105                 110
   Ala Leu Lys Thr Lys Leu Asp Leu Ser Ser Leu Ala Tyr Ser Gly Lys
           115                 120                 125
   Asp Ala
       130
<210> 27
   <211> 9
   <212> PRT
   <213> Human immunodeficiency virus type 2
<400> 27
   Arg Lys Lys Arg Arg Gln Arg Arg Arg
   1               5
<210> 28
   <211> 170
   <212> PRT
   <213> Artificial Sequence
<220>
   <223> TAT MTS(cs) 81-210 FRA
<400> 28
   Met Arg Lys Lys Arg Arg Gln Arg Arg Arg Gly Ser Asp Pro Ala Leu
   1               5                   10                  15
   Leu Thr Ala Ala Ala Arg Leu Leu Gly Thr Lys Asn Ala Ser Cys Leu
               20                  25                  30
   Val Leu Ala Ala Arg His Ala Ser Ser Gly Thr Leu Gly His Pro Gly
           35                  40                  45
   Ser Leu Asp Glu Thr Thr Tyr Glu Arg Leu Ala Glu Glu Thr Leu Asp
       50                  55                  60
   Ser Leu Ala Glu Phe Phe Glu Asp Leu Ala Asp Lys Pro Tyr Thr Phe
   65                  70                  75                  80
   Glu Asp Tyr Asp Val Ser Phe Gly Ser Gly Val Leu Thr Val Lys Leu
                   85                  90                  95
   Gly Gly Asp Leu Gly Thr Tyr Val Ile Asn Lys Gln Thr Pro Asn Lys
               100                 105                 110
   Gln Ile Trp Leu Ser Ser Pro Ser Ser Gly Pro Lys Arg Tyr Asp Trp
           115                 120                 125
   Thr Gly Lys Asn Trp Val Tyr Ser His Asp Gly Val Ser Leu His Glu
       130                 135                 140
   Leu Leu Ala Ala Glu Leu Thr Lys Ala Leu Lys Thr Lys Leu Asp Leu
   145                 150                 155                 160
   Ser Ser Leu Ala Tyr Ser Gly Lys Asp Ala
                   165                 170
<210> 29
   <211> 223
   <212> PRT
   <213> Artificial Sequence
<220>
   <223> TAT MTS(fra)81-210 FRA
<400> 29
   Met Arg Lys Lys Arg Arg Gln Arg Arg Arg Gly Ser Asp Pro Trp Thr
   1               5                   10                  15
   Leu Gly Arg Arg Ala Val Ala Gly Leu Leu Ala Ser Pro Ser Pro Ala
               20                  25                  30
   Gln Ala Gln Thr Leu Thr Arg Val Pro Arg Pro Ala Glu Leu Ala Pro
           35                  40                  45
   Leu Cys Gly Arg Arg Gly Leu Arg Thr Asp Ile Asp Ala Thr Cys Thr
       50                  55                  60
   Pro Arg Arg Ala Ser Ser Asn Gln Arg Gly Leu Asn Gln Ile Trp Asn
   65                  70                  75                  80
   Val Lys Lys Gln Ser Val Tyr Leu Met Asn Leu Arg Lys Ser Gly Thr
                   85                  90                  95
   Leu Gly His Pro Gly Ser Leu Asp Glu Thr Thr Tyr Glu Arg Leu Ala
               100                 105                 110
   Glu Glu Thr Leu Asp Ser Leu Ala Glu Phe Phe Glu Asp Leu Ala Asp
           115                 120                 125
   Lys Pro Tyr Thr Phe Glu Asp Tyr Asp Val Ser Phe Gly Ser Gly Val
       130                 135                 140
   Leu Thr Val Lys Leu Gly Gly Asp Leu Gly Thr Tyr Val Ile Asn Lys
   145                 150                 155                 160
   Gln Thr Pro Asn Lys Gln Ile Trp Leu Ser Ser Pro Ser Ser Gly Pro
                   165                 170                 175
   Lys Arg Tyr Asp Trp Thr Gly Lys Asn Trp Val Tyr Ser His Asp Gly
               180                 185                 190
   Val Ser Leu His Glu Leu Leu Ala Ala Glu Leu Thr Lys Ala Leu Lys
           195                 200                 205
   Thr Lys Leu Asp Leu Ser Ser Leu Ala Tyr Ser Gly Lys Asp Ala
       210                 215                 220
<210> 30
   <211> 179
   <212> PRT
   <213> Artificial Sequence
<220>
   <223> TAT MTS(lad) 81-210 FRA
<400> 30
   Met Arg Lys Lys Arg Arg Gln Arg Arg Arg Gly Ser Asp Pro Gln Ser
   1               5                   10                  15
   Trp Ser Arg Val Tyr Cys Ser Leu Ala Lys Arg Gly His Phe Asn Arg
               20                  25                  30
   Ile Ser His Gly Leu Gln Gly Leu Ser Ala Val Pro Leu Arg Thr Tyr
           35                  40                  45
   Ala Ser Gly Thr Leu Gly His Pro Gly Ser Leu Asp Glu Thr Thr Tyr
       50                  55                  60
   Glu Arg Leu Ala Glu Glu Thr Leu Asp Ser Leu Ala Glu Phe Phe Glu
   65                  70                  75                  80
   Asp Leu Ala Asp Lys Pro Tyr Thr Phe Glu Asp Tyr Asp Val Ser Phe
                   85                  90                  95
   Gly Ser Gly Val Leu Thr Val Lys Leu Gly Gly Asp Leu Gly Thr Tyr
               100                 105                 110
   Val Ile Asn Lys Gln Thr Pro Asn Lys Gln Ile Trp Leu Ser Ser Pro
           115                 120                 125
   Ser Ser Gly Pro Lys Arg Tyr Asp Trp Thr Gly Lys Asn Trp Val Tyr
       130                 135                 140
   Ser His Asp Gly Val Ser Leu His Glu Leu Leu Ala Ala Glu Leu Thr
   145                 150                 155                 160
   Lys Ala Leu Lys Thr Lys Leu Asp Leu Ser Ser Leu Ala Tyr Ser Gly
                   165                 170                 175
   Lys Asp Ala
<210> 31
   <211> 178
   <212> PRT
   <213> Artificial Sequence
<220>
   <223> TAT MTS(orf) 81-210 FRA
<400> 31
   Met Arg Lys Lys Arg Arg Gln Arg Arg Arg Gly Ser Asp Pro Gly Ala
   1 5 10 15
   Leu Val Ile Arg Gly Ile Arg Asn Phe Asn Leu Glu Asn Arg Ala Glu
   20 25 30
   Arg Glu Ile Ser Lys Met Lys Pro Ser Val Ala Pro Arg His Pro Ser
   35 40 45
   Ser Gly Thr Leu Gly His Pro Gly Ser Leu Asp Glu Thr Thr Tyr Glu
   50 55 60
   Arg Leu Ala Glu Glu Thr Leu Asp Ser Leu Ala Glu Phe Phe Glu Asp
   65 70 75 80
   Leu Ala Asp Lys Pro Tyr Thr Phe Glu Asp Tyr Asp Val Ser Phe Gly
   85 90 95
   Ser Gly Val Leu Thr Val Lys Leu Gly Gly Asp Leu Gly Thr Tyr Val
   100 105 110
   Ile Asn Lys Gln Thr Pro Asn Lys Gln Ile Trp Leu Ser Ser Pro Ser
   115 120 125
   Ser Gly Pro Lys Arg Tyr Asp Trp Thr Gly Lys Asn Trp Val Tyr Ser
   130 135 140
   His Asp Gly Val Ser Leu His Glu Leu Leu Ala Ala Glu Leu Thr Lys
   145 150 155 160
   Ala Leu Lys Thr Lys Leu Asp Leu Ser Ser Leu Ala Tyr Ser Gly Lys
   165 170 175
   Asp Ala
<210> 32
   <211> 4
   <212> PRT
   <213> Artificial Sequence
<220>
   <223> linker
<400> 32
   Gly Ser Asp Pro
   1
<210> 33
   <211> 166
   <212> PRT
   <213> Artificial Sequence
<220>
   <223> TAT MTS(cs) 81-210 FRA delta linker
<400> 33
   Met Arg Lys Lys Arg Arg Gln Arg Arg Arg Ala Leu Leu Thr Ala Ala
   1               5                   10                  15
   Ala Arg Leu Leu Gly Thr Lys Asn Ala Ser Cys Leu Val Leu Ala Ala
               20                  25                  30
   Arg His Ala Ser Ser Gly Thr Leu Gly His Pro Gly Ser Leu Asp Glu
           35                  40                  45
   Thr Thr Tyr Glu Arg Leu Ala Glu Glu Thr Leu Asp Ser Leu Ala Glu
       50                  55                  60
   Phe Phe Glu Asp Leu Ala Asp Lys Pro Tyr Thr Phe Glu Asp Tyr Asp
   65                  70                  75                  80
   Val Ser Phe Gly Ser Gly Val Leu Thr Val Lys Leu Gly Gly Asp Leu
                   85                  90                  95
   Gly Thr Tyr Val Ile Asn Lys Gln Thr Pro Asn Lys Gln Ile Trp Leu
               100                 105                 110
   Ser Ser Pro Ser Ser Gly Pro Lys Arg Tyr Asp Trp Thr Gly Lys Asn
           115                 120                 125
   Trp Val Tyr Ser His Asp Gly Val Ser Leu His Glu Leu Leu Ala Ala
       130                 135                 140
   Glu Leu Thr Lys Ala Leu Lys Thr Lys Leu Asp Leu Ser Ser Leu Ala
   145                 150                 155                 160
   Tyr Ser Gly Lys Asp Ala
                   165
<210> 34
   <211> 219
   <212> PRT
   <213> Artificial Sequence
<220>
   <223> TAT MTS(fra)81-210 FRA delta linker
<400> 34
   Met Arg Lys Lys Arg Arg Gln Arg Arg Arg Trp Thr Leu Gly Arg Arg
   1               5                   10                  15
   Ala Val Ala Gly Leu Leu Ala Ser Pro Ser Pro Ala Gln Ala Gln Thr
               20                  25                  30
   Leu Thr Arg Val Pro Arg Pro Ala Glu Leu Ala Pro Leu Cys Gly Arg
           35                  40                  45
   Arg Gly Leu Arg Thr Asp Ile Asp Ala Thr Cys Thr Pro Arg Arg Ala
       50                  55                  60
   Ser Ser Asn Gln Arg Gly Leu Asn Gln Ile Trp Asn Val Lys Lys Gln
   65                  70                  75                  80
   Ser Val Tyr Leu Met Asn Leu Arg Lys Ser Gly Thr Leu Gly His Pro
                   85                  90                  95
   Gly Ser Leu Asp Glu Thr Thr Tyr Glu Arg Leu Ala Glu Glu Thr Leu
               100                 105                 110
   Asp Ser Leu Ala Glu Phe Phe Glu Asp Leu Ala Asp Lys Pro Tyr Thr
           115                 120                 125
   Phe Glu Asp Tyr Asp Val Ser Phe Gly Ser Gly Val Leu Thr Val Lys
       130                 135                 140
   Leu Gly Gly Asp Leu Gly Thr Tyr Val Ile Asn Lys Gln Thr Pro Asn
   145                 150                 155                 160
   Lys Gln Ile Trp Leu Ser Ser Pro Ser Ser Gly Pro Lys Arg Tyr Asp
                   165                 170                 175
   Trp Thr Gly Lys Asn Trp Val Tyr Ser His Asp Gly Val Ser Leu His
               180                 185                 190
   Glu Leu Leu Ala Ala Glu Leu Thr Lys Ala Leu Lys Thr Lys Leu Asp
           195                 200                 205
   Leu Ser Ser Leu Ala Tyr Ser Gly Lys Asp Ala
       210                 215
<210> 35
   <211> 175
   <212> PRT
   <213> Artificial Sequence
<220>
   <223> TAT MTS(lad) 81-210 FRA delta linker
<400> 35
   Met Arg Lys Lys Arg Arg Gln Arg Arg Arg Gln Ser Trp Ser Arg Val
   1               5                   10                  15
   Tyr Cys Ser Leu Ala Lys Arg Gly His Phe Asn Arg Ile Ser His Gly
               20                  25                  30
   Leu Gln Gly Leu Ser Ala Val Pro Leu Arg Thr Tyr Ala Ser Gly Thr
           35                  40                  45
   Leu Gly His Pro Gly Ser Leu Asp Glu Thr Thr Tyr Glu Arg Leu Ala
       50                  55                  60
   Glu Glu Thr Leu Asp Ser Leu Ala Glu Phe Phe Glu Asp Leu Ala Asp
   65                  70                  75                  80
   Lys Pro Tyr Thr Phe Glu Asp Tyr Asp Val Ser Phe Gly Ser Gly Val
                   85                  90                  95
   Leu Thr Val Lys Leu Gly Gly Asp Leu Gly Thr Tyr Val Ile Asn Lys
               100                 105                 110
   Gln Thr Pro Asn Lys Gln Ile Trp Leu Ser Ser Pro Ser Ser Gly Pro
           115                 120                 125
   Lys Arg Tyr Asp Trp Thr Gly Lys Asn Trp Val Tyr Ser His Asp Gly
       130                 135                 140
   Val Ser Leu His Glu Leu Leu Ala Ala Glu Leu Thr Lys Ala Leu Lys
   145                 150                 155                 160
   Thr Lys Leu Asp Leu Ser Ser Leu Ala Tyr Ser Gly Lys Asp Ala
                   165                 170                 175
<210> 36
   <211> 174
   <212> PRT
   <213> Artificial Sequence
<220>
   <223> TAT MTS(orf) 81-210 FRA delta linker
<400> 36
   Met Arg Lys Lys Arg Arg Gln Arg Arg Arg Gly Ala Leu Val Ile Arg
   1               5                   10                  15
   Gly Ile Arg Asn Phe Asn Leu Glu Asn Arg Ala Glu Arg Glu Ile Ser
               20                  25                  30
   Lys Met Lys Pro Ser Val Ala Pro Arg His Pro Ser Ser Gly Thr Leu
           35                  40                  45
   Gly His Pro Gly Ser Leu Asp Glu Thr Thr Tyr Glu Arg Leu Ala Glu
       50                  55                  60
   Glu Thr Leu Asp Ser Leu Ala Glu Phe Phe Glu Asp Leu Ala Asp Lys
   65                  70                  75                  80
   Pro Tyr Thr Phe Glu Asp Tyr Asp Val Ser Phe Gly Ser Gly Val Leu
                   85                  90                  95
   Thr Val Lys Leu Gly Gly Asp Leu Gly Thr Tyr Val Ile Asn Lys Gln
               100                 105                 110
   Thr Pro Asn Lys Gln Ile Trp Leu Ser Ser Pro Ser Ser Gly Pro Lys
           115                 120                 125
   Arg Tyr Asp Trp Thr Gly Lys Asn Trp Val Tyr Ser His Asp Gly Val
       130                 135                 140
   Ser Leu His Glu Leu Leu Ala Ala Glu Leu Thr Lys Ala Leu Lys Thr
   145                 150                 155                 160
   Lys Leu Asp Leu Ser Ser Leu Ala Tyr Ser Gly Lys Asp Ala
                   165                 170
<210> 37
   <211> 105
   <212> DNA
   <213> Homo sapiens
<400> 37
   ctgtttaacc tgcgcattct gctgaacaat gcggccttcc gtaacggcca taattttatg      60
   gtccgcaact tccgttgcgg tcagccgctg caaaataaag tgcag                     105
<210> 38
   <211> 35
   <212> PRT
   <213> Homo sapiens
<400> 38
   Leu Phe Asn Leu Arg Ile Leu Leu Asn Asn Ala Ala Phe Arg Asn Gly
   1               5                   10                  15
   His Asn Phe Met Val Arg Asn Phe Arg Cys Gly Gln Pro Leu Gln Asn
               20                  25                  30
   Lys Val Gln
           35
<210> 39
   <211> 318
   <212> PRT
   <213> Homo sapiens
<400> 39
   Leu Lys Gly Arg Asp Leu Leu Thr Leu Arg Asn Phe Thr Gly Glu Glu
   1               5                   10                  15
   Ile Lys Tyr Met Leu Trp Leu Ser Ala Asp Leu Lys Phe Arg Ile Lys
               20                  25                  30
   Gln Lys Gly Glu Tyr Leu Pro Leu Leu Gln Gly Lys Ser Leu Gly Met
           35                  40                  45
   Ile Phe Glu Lys Arg Ser Thr Arg Thr Arg Leu Ser Thr Glu Thr Gly
       50                  55                  60
   Phe Ala Leu Leu Gly Gly His Pro Cys Phe Leu Thr Thr Gln Asp Ile
   65                  70                  75                  80
   His Leu Gly Val Asn Glu Ser Leu Thr Asp Thr Ala Arg Val Leu Ser
                   85                  90                  95
   Ser Met Ala Asp Ala Val Leu Ala Arg Val Tyr Lys Gln Ser Asp Leu
               100                 105                 110
   Asp Thr Leu Ala Lys Glu Ala Ser Ile Pro Ile Ile Asn Gly Leu Ser
   115 120 125
   Asp Leu Tyr His Pro Ile Gln Ile Leu Ala Asp Tyr Leu Thr Leu Gln
   130 135 140
   Glu His Tyr Ser Ser Leu Lys Gly Leu Thr Leu Ser Trp Ile Gly Asp
   145 150 155 160
   Gly Asn Asn Ile Leu His Ser Ile Met Met Ser Ala Ala Lys Phe Gly
   165 170 175
   Met His Leu Gln Ala Ala Thr Pro Lys Gly Tyr Glu Pro Asp Ala Ser
   180 185 190
   Val Thr Lys Leu Ala Glu Gln Tyr Ala Lys Glu Asn Gly Thr Lys Leu
   195 200 205
   Leu Leu Thr Asn Asp Pro Leu Glu Ala Ala His Gly Gly Asn Val Leu
   210 215 220
   Ile Thr Asp Thr Trp Ile Ser Met Gly Gln Glu Glu Glu Lys Lys Lys
   225 230 235 240
   Arg Leu Gln Ala Phe Gln Gly Tyr Gln Val Thr Met Lys Thr Ala Lys
   245 250 255
   Val Ala Ala Ser Asp Trp Thr Phe Leu His Cys Leu Pro Arg Lys Pro
   260 265 270
   Glu Glu Val Asp Asp Glu Val Phe Tyr Ser Pro Arg Ser Leu Val Phe
   275 280 285
   Pro Glu Ala Glu Asn Arg Lys Trp Thr Ile Met Ala Val Met Val Ser
   290 295 300
   Leu Leu Thr Asp Tyr Ser Pro Gln Leu Gln Lys Pro Lys Phe
   305 310 315
<210> 40
   <211> 1170
   <212> DNA
   <213> Artificial Sequence
<220>
   <223> His-TAT-MTS(otc)-OTC delta inker
<400> 40
   catatgggct catcgcatca tcatcatcat cactcatcag gtctggttcc gcgtggctcg      60
   cacatgtatg gtcgcaaaaa acgtcgtcaa cgtcgccgtc tgtttaacct gcgcattctg     120
   ctgaacaatg cggccttccg taacggccat aattttatgg tccgcaactt ccgttgcggt     180
   cagccgctgc aaaataaagt gcagctgaaa ggccgcgatc tgctgaccct gcgtaacttc     240
   acgggtgaag aaatcaaata catgctgtgg ctgagcgcag acctgaaatt ccgcatcaaa     300
   caaaaaggcg aatacctgcc gctgctgcag ggcaaatctc tgggtatgat ttttgaaaaa     360
   cgtagtaccc gcacgcgtct gtccaccgaa acgggctttg ccctgctggg cggtcatccg     420
   tgtttcctga ccacgcaaga tatccacctg ggtgtgaacg aaagtctgac cgatacggca     480
   cgcgttctga gctctatggc agacgctgtg ctggctcgtg tttataaaca gtccgatctg     540
   gacaccctgg cgaaagaagc ctcaattccg attatcaatg gcctgtcgga tctgtaccat     600
   ccgattcaaa tcctggcgga ctatctgacc ctgcaggaac actacagttc cctgaaaggt     660
   ctgaccctga gttggatcgg cgatggtaac aatattctgc atagcatcat gatgtctgca     720
   gctaaatttg gcatgcacct gcaagcggcc accccgaaag gttatgaacc ggatgccagc     780
   gttacgaaac tggcagaaca gtacgctaaa gaaaacggta ccaaactgct gctgacgaat     840
   gatccgctgg aagcagctca tggcggtaac gtcctgatta ccgacacgtg gatctctatg     900
   ggccaggaag aagaaaagaa aaaacgtctg caggcgtttc aaggttatca ggttaccatg     960
   aaaacggcca aagtcgcggc cagcgattgg accttcctgc actgcctgcc gcgtaaaccg    1020
   gaagaagtcg atgacgaagt gttttactca ccgcgctcgc tggtgttccc ggaagcagaa    1080
   aatcgtaaat ggaccatcat ggctgttatg gtgtccctgc tgaccgacta ttccccgcaa    1140
   ctgcaaaaac cgaaattcta atgaaagctt                                      1170
<210> 41
   <211> 1155
   <212> DNA
   <213> Artificial Sequence
<220>
   <223> His-TAT-MTS(cs)-OTC
<400> 41
   catatgggct catctcatca tcatcatcat cattcgtcag gtctggtccc gcgtggctct        60
   cacatgcgta aaaaacgtcg tcagcgtcgt cgtggcagtg atccggcact gctgaccgca       120
   gcagcacgtc tgctgggtac gaaaaacgct agctgcctgg tgctggctgc gcgtcatgcg       180
   tctgaatttc tgaaaggccg tgatctgctg accctgcgca acttcacggg tgaagaaatc       240
   aaatacatgc tgtggctgag tgccgacctg aaatttcgta tcaaacaaaa aggcgaatac       300
   ctgccgctgc tgcagggcaa atccctgggt atgattttcg aaaaacgcag tacccgtacg       360
   cgcctgtcca ccgaaacggg ctttgcactg ctgggcggtc atccgtgttt cctgaccacg       420
   caagatatcc acctgggtgt gaacgaatca ctgaccgata cggctcgtgt tctgagctct       480
   atggcagacg cagtgctggc acgtgtttat aaacagtcgg atctggacac cctggctaaa       540
   gaagcgtcaa ttccgattat caatggcctg tcggatctgt accatccgat tcaaatcctg       600
   gcggactatc tgaccctgca ggaacactac agttccctga aaggtctgac cctgagctgg       660
   atcggcgatg gtaacaatat tctgcatagc atcatgatgt ctgccgcaaa atttggcatg       720
   cacctgcaag ctgcgacccc gaaaggttat gaaccggacg ccagcgtcac gaaactggcc       780
   gaacagtacg caaaagaaaa cggtaccaaa ctgctgctga cgaatgatcc gctggaagcc       840
   gcacatggcg gtaacgttct gattaccgac acgtggatca gcatgggcca ggaagaagaa       900
   aagaaaaaac gtctgcaggc ctttcaaggt tatcaggtta ccatgaaaac ggcaaaagtc       960
   gctgcgtctg attggacctt cctgcactgc ctgccgcgca aaccggaaga agtcgatgac      1020
   gaagtgtttt actcaccgcg ttcgctggtt ttcccggaag cggaaaatcg caaatggacc      1080
   attatggctg tgatggtctc tctgctgacg gactactcgc cgcaactgca aaaaccgaaa      1140
   ttctaatgaa agctt                                                        1155
<210> 42
   <211> 1179
   <212> DNA
   <213> Artificial Sequence
<220>
   <223> His-TAT-MTS(orf)-OTC
<400> 42
   catatgggtt catcacatca tcatcatcat cattcatcag gtctggtccc gcgtggttca      60
   cacatgcgta aaaaacgtcg tcagcgtcgt cgtggcagtg atccgggtgc gctggtcatt     120
   cgtggcatcc gcaactttaa tctggaaaac cgtgcggaac gcgaaattag taaaatgaaa     180
   ccgtccgtgg caccgcgtca tccgtctgaa tttctgaaag gccgtgatct gctgaccctg     240
   cgcaacttca cgggtgaaga aatcaaatac atgctgtggc tgagtgcaga cctgaaattc     300
   cgtatcaaac aaaagggtga atacctgccg ctgctgcagg gcaaatccct gggtatgatt     360
   ttcgaaaaac gctcaacccg tacgcgcctg tcgaccgaaa cgggctttgc cctgctgggc     420
   ggtcatccgt gcttcctgac cacgcaagat atccacctgg gtgtgaacga atcactgacc     480
   gatacggcac gtgttctgag ctctatggca gacgcagtgc tggctcgtgt ttataaacag     540
   tcggatctgg acaccctggc aaaagaagct agcattccga ttatcaatgg cctgtctgat     600
   ctgtaccatc cgattcaaat cctggcggac tatctgaccc tgcaggaaca ctacagttcc     660
   ctgaaaggtc tgaccctgag ctggatcggc gatggtaaca atattctgca tagcatcatg     720
   atgtctgcgg ccaaattcgg catgcacctg caagcagcta ccccgaaagg ttatgaaccg     780
   gacgcctccg ttacgaaact ggcggaacag tacgccaaag aaaacggcac caaactgctg     840
   ctgacgaatg atccgctgga agcggcccat ggcggtaacg tcctgattac cgacacgtgg     900
   atcagcatgg gccaggaaga agaaaagaaa aaacgtctgc aggcatttca aggttatcag     960
   gttaccatga aaacggctaa agtcgcagct tctgattgga ccttcctgca ctgtctgccg    1020
   cgcaaaccgg aagaagtcga tgacgaagtg ttttactcac cgcgttcgct ggtgttcccg    1080
   gaagcggaaa atcgcaaatg gaccattatg gctgtgatgg tgtcgctgct gacggactac    1140
   tcgccgcaac tgcaaaaacc gaaattctaa tgaaagctt                           1179
<210> 43
   <211> 1182
   <212> DNA
   <213> Artificial Sequence
<220>
   <223> His-TAT-MTS(lad)-OTC
<400> 43
   catatgggta gttcacatca tcatcatcat cactcgtcgg gtctggtgcc gcgtggctca      60
   cacatgcgta aaaaacgtcg tcagcgtcgt cgtggctcag atccgcaatc atggtcgcgc     120
   gtctattgct cgctggcgaa acgtggtcat tttaaccgca ttagccacgg cctgcagggt     180
   ctgtctgcag tgccgctgcg tacctacgct gaatttctga aaggccgtga tctgctgacc     240
   ctgcgcaact tcacgggtga agaaatcaaa tacatgctgt ggctgagcgc agacctgaaa     300
   tttcgtatca aacaaaaagg cgaatacctg ccgctgctgc agggcaaatc tctgggtatg     360
   attttcgaaa aacgctcaac ccgtacgcgc ctgtcgaccg aaacgggctt tgccctgctg     420
   ggcggtcatc cgtgtttcct gaccacgcag gatatccacc tgggtgtgaa cgaaagtctg     480
   accgatacgg cacgtgttct gagctctatg gcagacgcag tgctggctcg tgtttataaa     540
   cagtccgatc tggacaccct ggcaaaagaa gctagtattc cgattatcaa tggcctgtcc     600
   gatctgtacc atccgattca aatcctggcg gactatctga ccctgcagga acactacagt     660
   tccctgaaag gtctgacgct gagctggatc ggcgatggta acaatattct gcatagtatc     720
   atgatgtccg cggccaaatt cggcatgcac ctgcaagcag ctaccccgaa aggttatgaa     780
   ccggacgcct ctgttacgaa actggcggaa cagtacgcca aagaaaacgg taccaaactg     840
   ctgctgacga atgatccgct ggaagcggcc catggcggta acgtcctgat taccgacacg     900
   tggatcagta tgggccagga agaagaaaag aaaaaacgtc tgcaggcgtt tcaaggttat     960
   caggttacca tgaaaacggc caaagtcgca gctagcgatt ggaccttcct gcactgcctg    1020
   ccgcgcaaac cggaagaagt cgatgacgaa gtgttttata gcccgcgttc tctggtgttc    1080
   ccggaagcgg aaaatcgcaa atggaccatc atggccgtta tggtgtcgct gctgaccgat    1140
   tactccccgc aactgcaaaa accgaaattc taatgaaagc tt                       1182
<210> 44
   <211> 385
   <212> PRT
   <213> Artificial Sequence
<220>
   <223> His-TAT-MTS(otc)-OTC delta linker
<400> 44
   Met Gly Ser Ser His His His His His His Ser Ser Gly Leu Val Pro
   1               5                   10                  15
   Arg Gly Ser His Met Tyr Gly Arg Lys Lys Arg Arg Gln Arg Arg Arg
               20                  25                  30
   Leu Phe Asn Leu Arg Ile Leu Leu Asn Asn Ala Ala Phe Arg Asn Gly
           35                  40                  45
   His Asn Phe Met Val Arg Asn Phe Arg Cys Gly Gln Pro Leu Gln Asn
       50                  55                  60
   Lys Val Gln Leu Lys Gly Arg Asp Leu Leu Thr Leu Arg Asn Phe Thr
   65                  70                  75                  80
   Gly Glu Glu Ile Lys Tyr Met Leu Trp Leu Ser Ala Asp Leu Lys Phe
                   85                  90                  95
   Arg Ile Lys Gln Lys Gly Glu Tyr Leu Pro Leu Leu Gln Gly Lys Ser
               100                 105                 110
   Leu Gly Met Ile Phe Glu Lys Arg Ser Thr Arg Thr Arg Leu Ser Thr
           115                 120                 125
   Glu Thr Gly Phe Ala Leu Leu Gly Gly His Pro Cys Phe Leu Thr Thr
       130                 135                 140
   Gln Asp Ile His Leu Gly Val Asn Glu Ser Leu Thr Asp Thr Ala Arg
   145                 150                 155                 160
   Val Leu Ser Ser Met Ala Asp Ala Val Leu Ala Arg Val Tyr Lys Gln
                   165                 170                 175
   Ser Asp Leu Asp Thr Leu Ala Lys Glu Ala Ser Ile Pro Ile Ile Asn
               180                 185                 190
   Gly Leu Ser Asp Leu Tyr His Pro Ile Gln Ile Leu Ala Asp Tyr Leu
           195                 200                 205
   Thr Leu Gln Glu His Tyr Ser Ser Leu Lys Gly Leu Thr Leu Ser Trp
   210 215 220
   Ile Gly Asp Gly Asn Asn Ile Leu His Ser Ile Met Met Ser Ala Ala
   225 230 235 240
   Lys Phe Gly Met His Leu Gln Ala Ala Thr Pro Lys Gly Tyr Glu Pro
   245 250 255
   Asp Ala Ser Val Thr Lys Leu Ala Glu Gln Tyr Ala Lys Glu Asn Gly
   260 265 270
   Thr Lys Leu Leu Leu Thr Asn Asp Pro Leu Glu Ala Ala His Gly Gly
   275 280 285
   Asn Val Leu Ile Thr Asp Thr Trp Ile Ser Met Gly Gln Glu Glu Glu
   290 295 300
   Lys Lys Lys Arg Leu Gln Ala Phe Gln Gly Tyr Gln Val Thr Met Lys
   305 310 315 320
   Thr Ala Lys Val Ala Ala Ser Asp Trp Thr Phe Leu His Cys Leu Pro
   325 330 335
   Arg Lys Pro Glu Glu Val Asp Asp Glu Val Phe Tyr Ser Pro Arg Ser
   340 345 350
   Leu Val Phe Pro Glu Ala Glu Asn Arg Lys Trp Thr Ile Met Ala Val
   355 360 365
   Met Val Ser Leu Leu Thr Asp Tyr Ser Pro Gln Leu Gln Lys Pro Lys
   370 375 380
   Phe
   385
<210> 45
   <211> 380
   <212> PRT
   <213> Artificial Sequence
<220>
   <223> His-TAT-MTS(cs)-OTC
<400> 45
   Met Gly Ser Ser His His His His His His Ser Ser Gly Leu Val Pro
   1 5 10 15
   Arg Gly Ser His Met Arg Lys Lys Arg Arg Gln Arg Arg Arg Gly Ser
               20                  25                  30
   Asp Pro Ala Leu Leu Thr Ala Ala Ala Arg Leu Leu Gly Thr Lys Asn
           35                  40                  45
   Ala Ser Cys Leu Val Leu Ala Ala Arg His Ala Ser Glu Phe Leu Lys
       50                  55                  60
   Gly Arg Asp Leu Leu Thr Leu Arg Asn Phe Thr Gly Glu Glu Ile Lys
   65                  70                  75                  80
   Tyr Met Leu Trp Leu Ser Ala Asp Leu Lys Phe Arg Ile Lys Gln Lys
                   85                  90                  95
   Gly Glu Tyr Leu Pro Leu Leu Gln Gly Lys Ser Leu Gly Met Ile Phe
               100                 105                 110
   Glu Lys Arg Ser Thr Arg Thr Arg Leu Ser Thr Glu Thr Gly Phe Ala
           115                 120                 125
   Leu Leu Gly Gly His Pro Cys Phe Leu Thr Thr Gln Asp Ile His Leu
       130                 135                 140
   Gly Val Asn Glu Ser Leu Thr Asp Thr Ala Arg Val Leu Ser Ser Met
   145                 150                 155                 160
   Ala Asp Ala Val Leu Ala Arg Val Tyr Lys Gln Ser Asp Leu Asp Thr
                   165                 170                 175
   Leu Ala Lys Glu Ala Ser Ile Pro Ile Ile Asn Gly Leu Ser Asp Leu
               180                 185                 190
   Tyr His Pro Ile Gln Ile Leu Ala Asp Tyr Leu Thr Leu Gln Glu His
           195                 200                 205
   Tyr Ser Ser Leu Lys Gly Leu Thr Leu Ser Trp Ile Gly Asp Gly Asn
       210                 215                 220
   Asn Ile Leu His Ser Ile Met Met Ser Ala Ala Lys Phe Gly Met His
   225                 230                 235                 240
   Leu Gln Ala Ala Thr Pro Lys Gly Tyr Glu Pro Asp Ala Ser Val Thr
                   245                 250                 255
   Lys Leu Ala Glu Gln Tyr Ala Lys Glu Asn Gly Thr Lys Leu Leu Leu
               260                 265                 270
   Thr Asn Asp Pro Leu Glu Ala Ala His Gly Gly Asn Val Leu Ile Thr
           275                 280                 285
   Asp Thr Trp Ile Ser Met Gly Gln Glu Glu Glu Lys Lys Lys Arg Leu
       290                 295                 300
   Gln Ala Phe Gln Gly Tyr Gln Val Thr Met Lys Thr Ala Lys Val Ala
   305                 310                 315                 320
   Ala Ser Asp Trp Thr Phe Leu His Cys Leu Pro Arg Lys Pro Glu Glu
                   325                 330                 335
   Val Asp Asp Glu Val Phe Tyr Ser Pro Arg Ser Leu Val Phe Pro Glu
               340                 345                 350
   Ala Glu Asn Arg Lys Trp Thr Ile Met Ala Val Met Val Ser Leu Leu
           355                 360                 365
   Thr Asp Tyr Ser Pro Gln Leu Gln Lys Pro Lys Phe
       370                 375                 380
<210> 46
   <211> 388
   <212> PRT
   <213> Artificial Sequence
<220>
   <223> His-TAT-MTS(orf)-OTC
<400> 46
   Met Gly Ser Ser His His His His His His Ser Ser Gly Leu Val Pro
   1               5                   10                  15
   Arg Gly Ser His Met Arg Lys Lys Arg Arg Gln Arg Arg Arg Gly Ser
               20                  25                  30
   Asp Pro Gly Ala Leu Val Ile Arg Gly Ile Arg Asn Phe Asn Leu Glu
           35                  40                  45
   Asn Arg Ala Glu Arg Glu Ile Ser Lys Met Lys Pro Ser Val Ala Pro
       50                  55                  60
   Arg His Pro Ser Glu Phe Leu Lys Gly Arg Asp Leu Leu Thr Leu Arg
   65                  70                  75                  80
   Asn Phe Thr Gly Glu Glu Ile Lys Tyr Met Leu Trp Leu Ser Ala Asp
                   85                  90                  95
   Leu Lys Phe Arg Ile Lys Gln Lys Gly Glu Tyr Leu Pro Leu Leu Gln
               100                 105                 110
   Gly Lys Ser Leu Gly Met Ile Phe Glu Lys Arg Ser Thr Arg Thr Arg
           115                 120                 125
   Leu Ser Thr Glu Thr Gly Phe Ala Leu Leu Gly Gly His Pro Cys Phe
       130                 135                 140
   Leu Thr Thr Gln Asp Ile His Leu Gly Val Asn Glu Ser Leu Thr Asp
   145                 150                 155                 160
   Thr Ala Arg Val Leu Ser Ser Met Ala Asp Ala Val Leu Ala Arg Val
                   165                 170                 175
   Tyr Lys Gln Ser Asp Leu Asp Thr Leu Ala Lys Glu Ala Ser Ile Pro
               180                 185                 190
   Ile Ile Asn Gly Leu Ser Asp Leu Tyr His Pro Ile Gln Ile Leu Ala
           195                 200                 205
   Asp Tyr Leu Thr Leu Gln Glu His Tyr Ser Ser Leu Lys Gly Leu Thr
       210                 215                 220
   Leu Ser Trp Ile Gly Asp Gly Asn Asn Ile Leu His Ser Ile Met Met
   225                 230                 235                 240
   Ser Ala Ala Lys Phe Gly Met His Leu Gln Ala Ala Thr Pro Lys Gly
                   245                 250                 255
   Tyr Glu Pro Asp Ala Ser Val Thr Lys Leu Ala Glu Gln Tyr Ala Lys
               260                 265                 270
   Glu Asn Gly Thr Lys Leu Leu Leu Thr Asn Asp Pro Leu Glu Ala Ala
           275                 280                 285
   His Gly Gly Asn Val Leu Ile Thr Asp Thr Trp Ile Ser Met Gly Gln
       290                 295                 300
   Glu Glu Glu Lys Lys Lys Arg Leu Gln Ala Phe Gln Gly Tyr Gln Val
   305                 310                 315                 320
   Thr Met Lys Thr Ala Lys Val Ala Ala Ser Asp Trp Thr Phe Leu His
                   325                 330                 335
   Cys Leu Pro Arg Lys Pro Glu Glu Val Asp Asp Glu Val Phe Tyr Ser
               340                 345                 350
   Pro Arg Ser Leu Val Phe Pro Glu Ala Glu Asn Arg Lys Trp Thr Ile
   355 360 365
   Met Ala Val Met Val Ser Leu Leu Thr Asp Tyr Ser Pro Gln Leu Gln
   370 375 380
   Lys Pro Lys Phe
   385
<210> 47
   <211> 389
   <212> PRT
   <213> Artificial Sequence
<220>
   <223> His-TAT-MTS(lad)-OTC
<400> 47
   Met Gly Ser Ser His His His His His His Ser Ser Gly Leu Val Pro
   1 5 10 15
   Arg Gly Ser His Met Arg Lys Lys Arg Arg Gln Arg Arg Arg Gly Ser
   20 25 30
   Asp Pro Gln Ser Trp Ser Arg Val Tyr Cys Ser Leu Ala Lys Arg Gly
   35 40 45
   His Phe Asn Arg Ile Ser His Gly Leu Gln Gly Leu Ser Ala Val Pro
   50 55 60
   Leu Arg Thr Tyr Ala Glu Phe Leu Lys Gly Arg Asp Leu Leu Thr Leu
   65 70 75 80
   Arg Asn Phe Thr Gly Glu Glu Ile Lys Tyr Met Leu Trp Leu Ser Ala
   85 90 95
   Asp Leu Lys Phe Arg Ile Lys Gln Lys Gly Glu Tyr Leu Pro Leu Leu
   100 105 110
   Gln Gly Lys Ser Leu Gly Met Ile Phe Glu Lys Arg Ser Thr Arg Thr
   115 120 125
   Arg Leu Ser Thr Glu Thr Gly Phe Ala Leu Leu Gly Gly His Pro Cys
   130 135 140
   Phe Leu Thr Thr Gln Asp Ile His Leu Gly Val Asn Glu Ser Leu Thr
   145 150 155 160
   Asp Thr Ala Arg Val Leu Ser Ser Met Ala Asp Ala Val Leu Ala Arg
                   165                 170                 175
   Val Tyr Lys Gln Ser Asp Leu Asp Thr Leu Ala Lys Glu Ala Ser Ile
               180                 185                 190
   Pro Ile Ile Asn Gly Leu Ser Asp Leu Tyr His Pro Ile Gln Ile Leu
           195                 200                 205
   Ala Asp Tyr Leu Thr Leu Gln Glu His Tyr Ser Ser Leu Lys Gly Leu
       210                 215                 220
   Thr Leu Ser Trp Ile Gly Asp Gly Asn Asn Ile Leu His Ser Ile Met
   225                 230                 235                 240
   Met Ser Ala Ala Lys Phe Gly Met His Leu Gln Ala Ala Thr Pro Lys
                   245                 250                 255
   Gly Tyr Glu Pro Asp Ala Ser Val Thr Lys Leu Ala Glu Gln Tyr Ala
               260                 265                 270
   Lys Glu Asn Gly Thr Lys Leu Leu Leu Thr Asn Asp Pro Leu Glu Ala
           275                 280                 285
   Ala His Gly Gly Asn Val Leu Ile Thr Asp Thr Trp Ile Ser Met Gly
       290                 295                 300
   Gln Glu Glu Glu Lys Lys Lys Arg Leu Gln Ala Phe Gln Gly Tyr Gln
   305                 310                 315                 320
   Val Thr Met Lys Thr Ala Lys Val Ala Ala Ser Asp Trp Thr Phe Leu
                   325                 330                 335
   His Cys Leu Pro Arg Lys Pro Glu Glu Val Asp Asp Glu Val Phe Tyr
               340                 345                 350
   Ser Pro Arg Ser Leu Val Phe Pro Glu Ala Glu Asn Arg Lys Trp Thr
           355                 360                 365
   Ile Met Ala Val Met Val Ser Leu Leu Thr Asp Tyr Ser Pro Gln Leu
       370                 375                 380
   Gln Lys Pro Lys Phe
   385
<210> 48
   <211> 362
   <212> PRT
   <213> Artificial Sequence
<220>
   <223> TAT-MTS(otc)-OTC delta linker
<400> 48
   Arg Lys Lys Arg Arg Gln Arg Arg Arg Leu Phe Asn Leu Arg Ile Leu
   1               5                   10                  15
   Leu Asn Asn Ala Ala Phe Arg Asn Gly His Asn Phe Met Val Arg Asn
               20                  25                  30
   Phe Arg Cys Gly Gln Pro Leu Gln Asn Lys Val Gln Leu Lys Gly Arg
           35                  40                  45
   Asp Leu Leu Thr Leu Arg Asn Phe Thr Gly Glu Glu Ile Lys Tyr Met
       50                  55                  60
   Leu Trp Leu Ser Ala Asp Leu Lys Phe Arg Ile Lys Gln Lys Gly Glu
   65                  70                  75                  80
   Tyr Leu Pro Leu Leu Gln Gly Lys Ser Leu Gly Met Ile Phe Glu Lys
                   85                  90                  95
   Arg Ser Thr Arg Thr Arg Leu Ser Thr Glu Thr Gly Phe Ala Leu Leu
               100                 105                 110
   Gly Gly His Pro Cys Phe Leu Thr Thr Gln Asp Ile His Leu Gly Val
           115                 120                 125
   Asn Glu Ser Leu Thr Asp Thr Ala Arg Val Leu Ser Ser Met Ala Asp
       130                 135                 140
   Ala Val Leu Ala Arg Val Tyr Lys Gln Ser Asp Leu Asp Thr Leu Ala
   145                 150                 155                 160
   Lys Glu Ala Ser Ile Pro Ile Ile Asn Gly Leu Ser Asp Leu Tyr His
                   165                 170                 175
   Pro Ile Gln Ile Leu Ala Asp Tyr Leu Thr Leu Gln Glu His Tyr Ser
               180                 185                 190
   Ser Leu Lys Gly Leu Thr Leu Ser Trp Ile Gly Asp Gly Asn Asn Ile
           195                 200                 205
   Leu His Ser Ile Met Met Ser Ala Ala Lys Phe Gly Met His Leu Gln
       210                 215                 220
   Ala Ala Thr Pro Lys Gly Tyr Glu Pro Asp Ala Ser Val Thr Lys Leu
   225                 230                 235                 240
   Ala Glu Gln Tyr Ala Lys Glu Asn Gly Thr Lys Leu Leu Leu Thr Asn
                   245                 250                 255
   Asp Pro Leu Glu Ala Ala His Gly Gly Asn Val Leu Ile Thr Asp Thr
               260                 265                 270
   Trp Ile Ser Met Gly Gln Glu Glu Glu Lys Lys Lys Arg Leu Gln Ala
           275                 280                 285
   Phe Gln Gly Tyr Gln Val Thr Met Lys Thr Ala Lys Val Ala Ala Ser
       290                 295                 300
   Asp Trp Thr Phe Leu His Cys Leu Pro Arg Lys Pro Glu Glu Val Asp
   305                 310                 315                 320
   Asp Glu Val Phe Tyr Ser Pro Arg Ser Leu Val Phe Pro Glu Ala Glu
                   325                 330                 335
   Asn Arg Lys Trp Thr Ile Met Ala Val Met Val Ser Leu Leu Thr Asp
               340                 345                 350
   Tyr Ser Pro Gln Leu Gln Lys Pro Lys Phe
           355                 360
<210> 49
   <211> 360
   <212> PRT
   <213> Artificial Sequence
<220>
   <223> TAT-MTS(cs)-OTC
<400> 49
   Met Arg Lys Lys Arg Arg Gln Arg Arg Arg Gly Ser Asp Pro Ala Leu
   1               5                   10                  15
   Leu Thr Ala Ala Ala Arg Leu Leu Gly Thr Lys Asn Ala Ser Cys Leu
               20                  25                  30
   Val Leu Ala Ala Arg His Ala Ser Glu Phe Leu Lys Gly Arg Asp Leu
           35                  40                  45
   Leu Thr Leu Arg Asn Phe Thr Gly Glu Glu Ile Lys Tyr Met Leu Trp
       50                  55                  60
   Leu Ser Ala Asp Leu Lys Phe Arg Ile Lys Gln Lys Gly Glu Tyr Leu
   65                  70                  75                  80
   Pro Leu Leu Gln Gly Lys Ser Leu Gly Met Ile Phe Glu Lys Arg Ser
                   85                  90                  95
   Thr Arg Thr Arg Leu Ser Thr Glu Thr Gly Phe Ala Leu Leu Gly Gly
               100                 105                 110
   His Pro Cys Phe Leu Thr Thr Gln Asp Ile His Leu Gly Val Asn Glu
           115                 120                 125
   Ser Leu Thr Asp Thr Ala Arg Val Leu Ser Ser Met Ala Asp Ala Val
       130                 135                 140
   Leu Ala Arg Val Tyr Lys Gln Ser Asp Leu Asp Thr Leu Ala Lys Glu
   145                 150                 155                 160
   Ala Ser Ile Pro Ile Ile Asn Gly Leu Ser Asp Leu Tyr His Pro Ile
                   165                 170                 175
   Gln Ile Leu Ala Asp Tyr Leu Thr Leu Gln Glu His Tyr Ser Ser Leu
               180                 185                 190
   Lys Gly Leu Thr Leu Ser Trp Ile Gly Asp Gly Asn Asn Ile Leu His
           195                 200                 205
   Ser Ile Met Met Ser Ala Ala Lys Phe Gly Met His Leu Gln Ala Ala
       210                 215                 220
   Thr Pro Lys Gly Tyr Glu Pro Asp Ala Ser Val Thr Lys Leu Ala Glu
   225                 230                 235                 240
   Gln Tyr Ala Lys Glu Asn Gly Thr Lys Leu Leu Leu Thr Asn Asp Pro
                   245                 250                 255
   Leu Glu Ala Ala His Gly Gly Asn Val Leu Ile Thr Asp Thr Trp Ile
               260                 265                 270
   Ser Met Gly Gln Glu Glu Glu Lys Lys Lys Arg Leu Gln Ala Phe Gln
           275                 280                 285
   Gly Tyr Gln Val Thr Met Lys Thr Ala Lys Val Ala Ala Ser Asp Trp
       290                 295                 300
   Thr Phe Leu His Cys Leu Pro Arg Lys Pro Glu Glu Val Asp Asp Glu
   305                 310                 315                 320
   Val Phe Tyr Ser Pro Arg Ser Leu Val Phe Pro Glu Ala Glu Asn Arg
   325 330 335
   Lys Trp Thr Ile Met Ala Val Met Val Ser Leu Leu Thr Asp Tyr Ser
   340 345 350
   Pro Gln Leu Gln Lys Pro Lys Phe
   355 360
<210> 50
   <211> 368
   <212> PRT
   <213> Artificial Sequence
<220>
   <223> TAT-MTS(orf)-OTC
<400> 50
   Met Arg Lys Lys Arg Arg Gln Arg Arg Arg Gly Ser Asp Pro Gly Ala
   1               5                   10                  15
   Leu Val Ile Arg Gly Ile Arg Asn Phe Asn Leu Glu Asn Arg Ala Glu
               20                  25                  30
   Arg Glu Ile Ser Lys Met Lys Pro Ser Val Ala Pro Arg His Pro Ser
           35                  40                  45
   Glu Phe Leu Lys Gly Arg Asp Leu Leu Thr Leu Arg Asn Phe Thr Gly
       50                  55                  60
   Glu Glu Ile Lys Tyr Met Leu Trp Leu Ser Ala Asp Leu Lys Phe Arg
   65                  70                  75                  80
   Ile Lys Gln Lys Gly Glu Tyr Leu Pro Leu Leu Gln Gly Lys Ser Leu
                   85                  90                  95
   Gly Met Ile Phe Glu Lys Arg Ser Thr Arg Thr Arg Leu Ser Thr Glu
               100                 105                 110
   Thr Gly Phe Ala Leu Leu Gly Gly His Pro Cys Phe Leu Thr Thr Gln
           115                 120                 125
   Asp Ile His Leu Gly Val Asn Glu Ser Leu Thr Asp Thr Ala Arg Val
       130                 135                 140
   Leu Ser Ser Met Ala Asp Ala Val Leu Ala Arg Val Tyr Lys Gln Ser
   145                 150                 155                 160
   Asp Leu Asp Thr Leu Ala Lys Glu Ala Ser Ile Pro Ile Ile Asn Gly
   165 170 175
   Leu Ser Asp Leu Tyr His Pro Ile Gln Ile Leu Ala Asp Tyr Leu Thr
   180 185 190
   Leu Gln Glu His Tyr Ser Ser Leu Lys Gly Leu Thr Leu Ser Trp Ile
   195 200 205
   Gly Asp Gly Asn Asn Ile Leu His Ser Ile Met Met Ser Ala Ala Lys
   210 215 220
   Phe Gly Met His Leu Gln Ala Ala Thr Pro Lys Gly Tyr Glu Pro Asp
   225 230 235 240
   Ala Ser Val Thr Lys Leu Ala Glu Gln Tyr Ala Lys Glu Asn Gly Thr
   245 250 255
   Lys Leu Leu Leu Thr Asn Asp Pro Leu Glu Ala Ala His Gly Gly Asn
   260 265 270
   Val Leu Ile Thr Asp Thr Trp Ile Ser Met Gly Gln Glu Glu Glu Lys
   275 280 285
   Lys Lys Arg Leu Gln Ala Phe Gln Gly Tyr Gln Val Thr Met Lys Thr
   290 295 300
   Ala Lys Val Ala Ala Ser Asp Trp Thr Phe Leu His Cys Leu Pro Arg
   305 310 315 320
   Lys Pro Glu Glu Val Asp Asp Glu Val Phe Tyr Ser Pro Arg Ser Leu
   325 330 335
   Val Phe Pro Glu Ala Glu Asn Arg Lys Trp Thr Ile Met Ala Val Met
   340 345 350
   Val Ser Leu Leu Thr Asp Tyr Ser Pro Gln Leu Gln Lys Pro Lys Phe
   355 360 365
<210> 51
   <211> 369
   <212> PRT
   <213> Artificial Sequence
<220>
   <223> TAT-MTS(lad)-OTC
<400> 51
   Met Arg Lys Lys Arg Arg Gln Arg Arg Arg Gly Ser Asp Pro Gln Ser
   1               5                   10                  15
   Trp Ser Arg Val Tyr Cys Ser Leu Ala Lys Arg Gly His Phe Asn Arg
               20                  25                  30
   Ile Ser His Gly Leu Gln Gly Leu Ser Ala Val Pro Leu Arg Thr Tyr
           35                  40                  45
   Ala Glu Phe Leu Lys Gly Arg Asp Leu Leu Thr Leu Arg Asn Phe Thr
       50                  55                  60
   Gly Glu Glu Ile Lys Tyr Met Leu Trp Leu Ser Ala Asp Leu Lys Phe
   65                  70                  75                  80
   Arg Ile Lys Gln Lys Gly Glu Tyr Leu Pro Leu Leu Gln Gly Lys Ser
                   85                  90                  95
   Leu Gly Met Ile Phe Glu Lys Arg Ser Thr Arg Thr Arg Leu Ser Thr
               100                 105                 110
   Glu Thr Gly Phe Ala Leu Leu Gly Gly His Pro Cys Phe Leu Thr Thr
           115                 120                 125
   Gln Asp Ile His Leu Gly Val Asn Glu Ser Leu Thr Asp Thr Ala Arg
       130                 135                 140
   Val Leu Ser Ser Met Ala Asp Ala Val Leu Ala Arg Val Tyr Lys Gln
   145                 150                 155                 160
   Ser Asp Leu Asp Thr Leu Ala Lys Glu Ala Ser Ile Pro Ile Ile Asn
                   165                 170                 175
   Gly Leu Ser Asp Leu Tyr His Pro Ile Gln Ile Leu Ala Asp Tyr Leu
               180                 185                 190
   Thr Leu Gln Glu His Tyr Ser Ser Leu Lys Gly Leu Thr Leu Ser Trp
           195                 200                 205
   Ile Gly Asp Gly Asn Asn Ile Leu His Ser Ile Met Met Ser Ala Ala
       210                 215                 220
   Lys Phe Gly Met His Leu Gln Ala Ala Thr Pro Lys Gly Tyr Glu Pro
   225                 230                 235                 240
   Asp Ala Ser Val Thr Lys Leu Ala Glu Gln Tyr Ala Lys Glu Asn Gly
                   245                 250                 255
   Thr Lys Leu Leu Leu Thr Asn Asp Pro Leu Glu Ala Ala His Gly Gly
   260 265 270
   Asn Val Leu Ile Thr Asp Thr Trp Ile Ser Met Gly Gln Glu Glu Glu
   275 280 285
   Lys Lys Lys Arg Leu Gln Ala Phe Gln Gly Tyr Gln Val Thr Met Lys
   290 295 300
   Thr Ala Lys Val Ala Ala Ser Asp Trp Thr Phe Leu His Cys Leu Pro
   305 310 315 320
   Arg Lys Pro Glu Glu Val Asp Asp Glu Val Phe Tyr Ser Pro Arg Ser
   325 330 335
   Leu Val Phe Pro Glu Ala Glu Asn Arg Lys Trp Thr Ile Met Ala Val
   340 345 350
   Met Val Ser Leu Leu Thr Asp Tyr Ser Pro Gln Leu Gln Lys Pro Lys
   355 360 365
   Phe
<210> 52
   <211> 356
   <212> PRT
   <213> Artificial Sequence
<220>
   <223> TAT-MTS(cs)-OTC delta linker
<400> 52
   Met Arg Lys Lys Arg Arg Gln Arg Arg Arg Ala Leu Leu Thr Ala Ala
   1               5                   10                  15
   Ala Arg Leu Leu Gly Thr Lys Asn Ala Ser Cys Leu Val Leu Ala Ala
               20                  25                  30
   Arg His Ala Ser Glu Phe Leu Lys Gly Arg Asp Leu Leu Thr Leu Arg
           35                  40                  45
   Asn Phe Thr Gly Glu Glu Ile Lys Tyr Met Leu Trp Leu Ser Ala Asp
       50                  55                  60
   Leu Lys Phe Arg Ile Lys Gln Lys Gly Glu Tyr Leu Pro Leu Leu Gln
   65                  70                  75                  80
   Gly Lys Ser Leu Gly Met Ile Phe Glu Lys Arg Ser Thr Arg Thr Arg
                   85                  90                  95
   Leu Ser Thr Glu Thr Gly Phe Ala Leu Leu Gly Gly His Pro Cys Phe
               100                 105                 110
   Leu Thr Thr Gln Asp Ile His Leu Gly Val Asn Glu Ser Leu Thr Asp
           115                 120                 125
   Thr Ala Arg Val Leu Ser Ser Met Ala Asp Ala Val Leu Ala Arg Val
       130                 135                 140
   Tyr Lys Gln Ser Asp Leu Asp Thr Leu Ala Lys Glu Ala Ser Ile Pro
   145                 150                 155                 160
   Ile Ile Asn Gly Leu Ser Asp Leu Tyr His Pro Ile Gln Ile Leu Ala
                   165                 170                 175
   Asp Tyr Leu Thr Leu Gln Glu His Tyr Ser Ser Leu Lys Gly Leu Thr
               180                 185                 190
   Leu Ser Trp Ile Gly Asp Gly Asn Asn Ile Leu His Ser Ile Met Met
           195                 200                 205
   Ser Ala Ala Lys Phe Gly Met His Leu Gln Ala Ala Thr Pro Lys Gly
       210                 215                 220
   Tyr Glu Pro Asp Ala Ser Val Thr Lys Leu Ala Glu Gln Tyr Ala Lys
   225                 230                 235                 240
   Glu Asn Gly Thr Lys Leu Leu Leu Thr Asn Asp Pro Leu Glu Ala Ala
                   245                 250                 255
   His Gly Gly Asn Val Leu Ile Thr Asp Thr Trp Ile Ser Met Gly Gln
               260                 265                 270
   Glu Glu Glu Lys Lys Lys Arg Leu Gln Ala Phe Gln Gly Tyr Gln Val
           275                 280                 285
   Thr Met Lys Thr Ala Lys Val Ala Ala Ser Asp Trp Thr Phe Leu His
       290                 295                 300
   Cys Leu Pro Arg Lys Pro Glu Glu Val Asp Asp Glu Val Phe Tyr Ser
   305                 310                 315                 320
   Pro Arg Ser Leu Val Phe Pro Glu Ala Glu Asn Arg Lys Trp Thr Ile
                   325                 330                 335
   Met Ala Val Met Val Ser Leu Leu Thr Asp Tyr Ser Pro Gln Leu Gln
   340 345 350
   Lys Pro Lys Phe
   355
<210> 53
   <211> 364
   <212> PRT
   <213> Artificial Sequence
<220>
   <223> TAT-MTS(orf)-OTC delta linker
<400> 53
   Met Arg Lys Lys Arg Arg Gln Arg Arg Arg Gly Ala Leu Val Ile Arg
   1               5                   10                  15
   Gly Ile Arg Asn Phe Asn Leu Glu Asn Arg Ala Glu Arg Glu Ile Ser
               20                  25                  30
   Lys Met Lys Pro Ser Val Ala Pro Arg His Pro Ser Glu Phe Leu Lys
           35                  40                  45
   Gly Arg Asp Leu Leu Thr Leu Arg Asn Phe Thr Gly Glu Glu Ile Lys
       50                  55                  60
   Tyr Met Leu Trp Leu Ser Ala Asp Leu Lys Phe Arg Ile Lys Gln Lys
   65                  70                  75                  80
   Gly Glu Tyr Leu Pro Leu Leu Gln Gly Lys Ser Leu Gly Met Ile Phe
                   85                  90                  95
   Glu Lys Arg Ser Thr Arg Thr Arg Leu Ser Thr Glu Thr Gly Phe Ala
               100                 105                 110
   Leu Leu Gly Gly His Pro Cys Phe Leu Thr Thr Gln Asp Ile His Leu
           115                 120                 125
   Gly Val Asn Glu Ser Leu Thr Asp Thr Ala Arg Val Leu Ser Ser Met
       130                 135                 140
   Ala Asp Ala Val Leu Ala Arg Val Tyr Lys Gln Ser Asp Leu Asp Thr
   145                 150                 155                 160
   Leu Ala Lys Glu Ala Ser Ile Pro Ile Ile Asn Gly Leu Ser Asp Leu
                   165                 170                 175
   Tyr His Pro Ile Gln Ile Leu Ala Asp Tyr Leu Thr Leu Gln Glu His
               180                 185                 190
   Tyr Ser Ser Leu Lys Gly Leu Thr Leu Ser Trp Ile Gly Asp Gly Asn
   195 200 205
   Asn Ile Leu His Ser Ile Met Met Ser Ala Ala Lys Phe Gly Met His
   210 215 220
   Leu Gln Ala Ala Thr Pro Lys Gly Tyr Glu Pro Asp Ala Ser Val Thr
   225 230 235 240
   Lys Leu Ala Glu Gln Tyr Ala Lys Glu Asn Gly Thr Lys Leu Leu Leu
   245 250 255
   Thr Asn Asp Pro Leu Glu Ala Ala His Gly Gly Asn Val Leu Ile Thr
   260 265 270
   Asp Thr Trp Ile Ser Met Gly Gln Glu Glu Glu Lys Lys Lys Arg Leu
   275 280 285
   Gln Ala Phe Gln Gly Tyr Gln Val Thr Met Lys Thr Ala Lys Val Ala
   290 295 300
   Ala Ser Asp Trp Thr Phe Leu His Cys Leu Pro Arg Lys Pro Glu Glu
   305 310 315 320
   Val Asp Asp Glu Val Phe Tyr Ser Pro Arg Ser Leu Val Phe Pro Glu
   325 330 335
   Ala Glu Asn Arg Lys Trp Thr Ile Met Ala Val Met Val Ser Leu Leu
   340 345 350
   Thr Asp Tyr Ser Pro Gln Leu Gln Lys Pro Lys Phe
   355 360
<210> 54
   <211> 365
   <212> PRT
   <213> Artificial Sequence
<220>
   <223> TAT-MTS(lad)-OTC delta linker
<400> 54
   Met Arg Lys Lys Arg Arg Gln Arg Arg Arg Gln Ser Trp Ser Arg Val
   1 5 10 15
   Tyr Cys Ser Leu Ala Lys Arg Gly His Phe Asn Arg Ile Ser His Gly
   20 25 30
   Leu Gln Gly Leu Ser Ala Val Pro Leu Arg Thr Tyr Ala Glu Phe Leu
           35                  40                  45
   Lys Gly Arg Asp Leu Leu Thr Leu Arg Asn Phe Thr Gly Glu Glu Ile
       50                  55                  60
   Lys Tyr Met Leu Trp Leu Ser Ala Asp Leu Lys Phe Arg Ile Lys Gln
   65                  70                  75                  80
   Lys Gly Glu Tyr Leu Pro Leu Leu Gln Gly Lys Ser Leu Gly Met Ile
                   85                  90                  95
   Phe Glu Lys Arg Ser Thr Arg Thr Arg Leu Ser Thr Glu Thr Gly Phe
               100                 105                 110
   Ala Leu Leu Gly Gly His Pro Cys Phe Leu Thr Thr Gln Asp Ile His
           115                 120                 125
   Leu Gly Val Asn Glu Ser Leu Thr Asp Thr Ala Arg Val Leu Ser Ser
       130                 135                 140
   Met Ala Asp Ala Val Leu Ala Arg Val Tyr Lys Gln Ser Asp Leu Asp
   145                 150                 155                 160
   Thr Leu Ala Lys Glu Ala Ser Ile Pro Ile Ile Asn Gly Leu Ser Asp
                   165                 170                 175
   Leu Tyr His Pro Ile Gln Ile Leu Ala Asp Tyr Leu Thr Leu Gln Glu
               180                 185                 190
   His Tyr Ser Ser Leu Lys Gly Leu Thr Leu Ser Trp Ile Gly Asp Gly
           195                 200                 205
   Asn Asn Ile Leu His Ser Ile Met Met Ser Ala Ala Lys Phe Gly Met
       210                 215                 220
   His Leu Gln Ala Ala Thr Pro Lys Gly Tyr Glu Pro Asp Ala Ser Val
   225                 230                 235                 240
   Thr Lys Leu Ala Glu Gln Tyr Ala Lys Glu Asn Gly Thr Lys Leu Leu
                   245                 250                 255
   Leu Thr Asn Asp Pro Leu Glu Ala Ala His Gly Gly Asn Val Leu Ile
               260                 265                 270
   Thr Asp Thr Trp Ile Ser Met Gly Gln Glu Glu Glu Lys Lys Lys Arg
           275                 280                 285
   Leu Gln Ala Phe Gln Gly Tyr Gln Val Thr Met Lys Thr Ala Lys Val
       290                 295                 300
   Ala Ala Ser Asp Trp Thr Phe Leu His Cys Leu Pro Arg Lys Pro Glu
   305                 310                 315                 320
   Glu Val Asp Asp Glu Val Phe Tyr Ser Pro Arg Ser Leu Val Phe Pro
                   325                 330                 335
   Glu Ala Glu Asn Arg Lys Trp Thr Ile Met Ala Val Met Val Ser Leu
               340                 345                 350
   Leu Thr Asp Tyr Ser Pro Gln Leu Gln Lys Pro Lys Phe
           355                 360                 365

## Description

### TECHNOLOGICAL FIELD

Disclosed are novel fusion protein constructs comprising a functional mitochondrial protein. Further, the disclosed fusion proteins and compositions therefor are disclosed for use in treating mitochondrial disorders.

### PRIOR ART

References considered to be relevant as background to the presently disclosed subject matter are listed below:
[1] Chinnery PF, Schon EA (2003) Mitochondria. J Neurol Neurosurg Psychiatry 74: 1188-1199.
[2] DiMauro S, Schon EA (2003) Mitochondrial respiratory-chain diseases. N Engl J Med 348: 2656-2668.
[3] Brautigam CA, Chuang JL, Tomchick DR, Machius M, Chuang DT (2005) Crystal structure of human dihydrolipoamide dehydrogenase: NAD+/NADH binding and the structural basis of disease-causing mutations. J Mol Biol 350: 543-552.
[4] Brady RO, Schiffmann R (2004) Enzyme-replacement therapy for metabolic storage disorders. Lancet Neurol 3: 752-756.
[5] Wang D, Bonten EJ, Yogalingam G, Mann L, d'Azzo A (2005) Short-term, high dose enzyme replacement therapy in sialidosis mice. Mol Genet Metab 85: 181-189.
[6] Luft FC (2003) Transducing proteins to manipulate intracellular targets. J Mol Med (Berl) 81: 521-523.
[7] Kabouridis PS (2003) Biological applications of protein transduction technology. Trends Biotechnol 21: 498-503.
[8] Green M, Loewenstein PM (1988) Autonomous functional domains of chemically synthesized human immunodeficiency virus tat trans-activator protein. Cell 55: 1179-1188.
[9] Frankel AD, Pabo CO (1988) Cellular uptake of the tat protein from human immunodeficiency virus. Cell 55: 1189-1193.
[10] Futaki S, Suzuki T, Ohashi W, Yagami T, Tanaka S, et al. (2001) Arginine-rich peptides. An abundant source of membrane-permeable peptides having potential as carriers for intracellular protein delivery. J Biol Chem 276: 5836-5840.
[11] Schwarze SR, Ho A, Vocero-Akbani A, Dowdy SF (1999) In vivo protein transduction: delivery of a biologically active protein into the mouse. Science 285: 1569-1572.
[12] Guo X, Hutcheon AE, Zieske JD (2004) Transduction of functionally active TAT fusion proteins into cornea. Exp Eye Res 78: 997-1005.
[13] Del Gaizo V, MacKenzie JA, Payne RM (2003) Targeting proteins to mitochondria using TAT. Mol Genet Metab 80: 170-180.
[14] Harding AE (1981) Friedreich's ataxia: a clinical and genetic study of 90 families with an analysis of early diagnostic criteria and intrafamilial clustering of clinical features. Brain 104: 589-620.
[15] Schulz JB, Boesch S, Burk K, Durr A, Giunti P, et al. (2009) Diagnosis and treatment of Friedreich ataxia: a European perspective. Nat Rev Neurol 5: 222-234.
[16] Durr A, Cossee M, Agid Y, Campuzano V, Mignard C, et al. (1996) Clinical and genetic abnormalities in patients with Friedreich's ataxia. N Engl J Med 335: 1169-1175.
[17] Campuzano V, Montermini L, Lutz Y, Cova L, Hindelang C, et al. (1997) Frataxin is reduced in Friedreich ataxia patients and is associated with mitochondrial membranes. Hum Mol Genet 6: 1771-1780.
[18] Rotig A, de Lonlay P, Chretien D, Foury F, Koenig M, et al. (1997) Aconitase and mitochondrial iron-sulphur protein deficiency in Friedreich ataxia. Nat Genet 17: 215-217.
[19] Lodi R, Cooper JM, Bradley JL, Manners D, Styles P, et al. (1999) Deficit of in vivo mitochondrial ATP production in patients with Friedreich ataxia. Proc Natl Acad Sci U S A 96: 11492-11495.
[20] Delatycki MB, Camakaris J, Brooks H, Evans-Whipp T, Thorburn DR, et al. (1999) Direct evidence that mitochondrial iron accumulation occurs in Friedreich ataxia. Ann Neurol 45: 673-675.
[21] Tsou AY, Friedman LS, Wilson RB, Lynch DR (2009) Pharmacotherapy for Friedreich ataxia. CNS Drugs 23: 213-223.
[22] Perlman SL (2012) A review of Friedreich ataxia clinical trial results. J Child Neurol 27: 1217-1222.
[23] Rapoport M, Saada A, Elpeleg O, Lorberboum-Galski H (2008) TAT-mediated delivery of LAD restores pyruvate dehydrogenase complex activity in the mitochondria of patients with LAD deficiency. Mol Ther 16: 691-697.
[24] Rapoport M, Salman L, Sabag O, Patel MS, Lorberboum-Galski H (2011) Successful TAT-mediated enzyme replacement therapy in a mouse model of mitochondrial E3 deficiency. J Mol Med (Berl) 89: 161-170.
[25] Vyas PM, Tomamichel WJ, Pride PM, Babbey CM, Wang Q, et al. (2012) A TAT-frataxin fusion protein increases lifespan and cardiac function in a conditional Friedreich's ataxia mouse model. Hum Mol Genet 21: 1230-1247.
[26] Gakh O, Cavadini P, Isaya G (2002) Mitochondrial processing peptidases. Biochim Biophys Acta 1592: 63-77.
[27] Cavadini P, Adamec J, Taroni F, Gakh O, Isaya G (2000) Two-step processing of human frataxin by mitochondrial processing peptidase. Precursor and intermediate forms are cleaved at different rates. J Biol Chem 275: 41469-41475.
[28] Schmucker S, Argentini M, Carelle-Calmels N, Martelli A, Puccio H (2008) The in vivo mitochondrial two-step maturation of human frataxin. Hum Mol Genet 17: 3521-3531.
[29] Gakh O, Bedekovics T, Duncan SF, Smith DYt, Berkholz DS, et al. (2010) Normal and Friedreich ataxia cells express different isoforms of frataxin with complementary roles in iron-sulfur cluster assembly. J Biol Chem 285: 38486-38501.
[30] Gavel Y, von Heijne G (1990) Cleavage-site motifs in mitochondrial targeting peptides. Protein Eng 4: 33-37.
[31] Braun HP, Schmitz UK (1997) The mitochondrial processing peptidase. Int J Biochem Cell Biol 29: 1043-1045.
[32] Horwich A (1990) Protein import into mitochondria and peroxisomes. Curr Opin Cell Biol 2: 625-633.
[33] Saada A, Edvardson S, Rapoport M, Shaag A, Amry K, et al. (2008) C6ORF66 is an assembly factor of mitochondrial complex I. Am J Hum Genet 82: 32-38.
[34] Cheng TL, Liao CC, Tsai WH, Lin CC, Yeh CW, et al. (2009) Identification and characterization of the mitochondrial targeting sequence and mechanism in human citrate synthase. J Cell Biochem 107: 1002-1015.
[35] Santos R, Lefevre S, Sliwa D, Seguin A, Camadro JM, et al. (2010) Friedreich ataxia: molecular mechanisms, redox considerations, and therapeutic opportunities. Antioxid Redox Signal 13: 651-690.
[36] Bulteau AL, O'Neill HA, Kennedy MC, Ikeda-Saito M, Isaya G, et al. (2004) Frataxin acts as an iron chaperone protein to modulate mitochondrial aconitase activity. Science 305: 242-245.
[37] Richardson TE, Yu AE, Wen Y, Yang SH, Simpkins JW (2012) Estrogen prevents oxidative damage to the mitochondria in Friedreich's ataxia skin fibroblasts. PLoS One 7: e34600.
[38] WO 2009/098682.
[39] http:// www.curefa.org/pipeline.html.
[40] Tang N, et al. (2012) Stable Overexpression of Arginase I and Ornithine Transcarbamylase in HepG2 Cells Improves Its Ammonia Detoxification. Journal of Cellular Biochemistry 113: 518-527.
[41] Cunningham, S. C. et al. (2011) Induction and Prevention of Severe Hyperammonemia in the spfash Mouse Model of Ornithine Transcarbamylase Deficiency Using shRNA and rAAV-mediated Gene Delivery. Molecular Therapy 19(5):854-9.

Acknowledgement of the above references herein is not to be inferred as meaning that these are in any way relevant to the patentability of the presently disclosed subject matter.

### BACKGROUND

Mitochondria play a major and critical role in cellular homeostasis - they participate in intracellular signaling and apoptosis, and perform numerous biochemical tasks, such as in pyruvate oxidation, in the citric acid cycle (also referred to as the Krebs cycle), and in metabolism of amino acids, fatty acids, nucleotides and steroids. However, the most crucial task of mitochondria is their role in cellular energy metabolism. This includes β-oxidation of fatty acids and production of ATP by means of the electron-transport chain and the oxidative-phosphorylation system [1,2].

Most of the approximately 900 gene products in the mitochondria are encoded by nuclear DNA (nDNA) while mitochondrial DNA (mtDNA) only contains 13 protein encoding genes. Most of the polypeptides encoded by nDNA genes are synthesized with a mitochondrial targeting sequence (MTS), allowing their import from the cytoplasm into mitochondria through the translocation machinery (TOM/TIM). Upon entering the mitochondria, the MTS is recognized and cleaved off, allowing for proper processing and, if necessary, assembly into mitochondrial enzymatic complexes [3].

Currently, there is no cure for genetic mitochondrial metabolic disorders and treatment is mostly palliative.

Enzyme or Protein Replacement Therapy (E/PRT) is a therapeutic approach for metabolic disorders, whereby the deficient or absent protein/enzyme is artificially manufactured, purified and administered intravenously to the patient in need thereof on a regular basis.

After many years of extensive research, E/PRT has been successfully accepted as the treatment of choice for metabolic lysosomal storage diseases, including Gaucher disease, Fabry disease and attenuated variants of mucopolysaccaridoses type 1 (MPS 1). However, the inability of the intravenously administered enzymes to penetrate the blood-brain barrier severely limits the application of this approach for the treatment of other metabolic disorders that involve the central nervous system (CNS) [4, 5].

One approach for delivering proteins into cells is their fusion with protein transduction domains (PTDs), a group of short peptides that serve as delivery vectors for large molecules. Generally, PTDs are defined as short, water-soluble and partly hydrophobic, and/or polybasic peptides (at most 30 - 35 amino acids residues) with a net positive charge at physiological pH [6, 7]. The main feature of PTDs is that they are able to penetrate the cell membrane at low micromolar concentrations both *in vitro* and *in vivo* without using any chiral receptors and without causing significant membrane damage.

Furthermore, and even more importantly, these peptides are capable of internalizing electrostatically or covalently bound biologically active cargoes (such as drugs) with high efficiency and with low toxicity. The mechanism(s) by which PTDs enter the cells has not been completely understood. One of the well-characterized PTDs is the transactivator of transcription (TAT) peptide originating from the HIV-1 virus. TAT is an 11-amino-acid (residues 47-57) arginine- and lysine-rich portion of the Tat protein encoded by HIV-1 virus [8, 9]. TAT-fusion proteins have been previously shown to be rapidly and efficiently introduced into cultured cells, intact tissue and live tissues when injected into mice [10-12]. It has also been demonstrated that TAT fusion proteins traverse mitochondrial membranes [13, 38].

There has been great progress in the use of PTD-fusion proteins for the delivery of different macromolecules into cells both *in vitro* and *in vivo.* This system can be used even for the delivery of cargoes into the brain across the blood-brain barrier. In addition, the ability to target specific intracellular sub-localizations, such as the nuclei, the mitochondria and lysosomes, further expands the possibilities of this delivery system to the development of sub-cellular organelle-targeted therapy. The therapeutic applications seem almost unlimited, and the use of the TAT-based delivery system has extended from proteins to a large variety of cargoes such as oligonucleotides, imaging agents, low molecular mass drugs, nanoparticles, micelles and liposomes. As will be shown, this PTD system is used for developing fusion constructs of functional mitochondrial proteins, for treatment of mitochondrial disorders, for example Friedreich ataxia.

Friedreich ataxia is an autosomal recessive degenerative disorder characterized by ataxia, areflexia, sensory loss, weakness, scoliosis, and cardiomyopathy. Diabetes mellitus, optic neuropathy, and hearing loss are also seen in patients suffering from this disease [14, 15]. Most patients with Friedreich ataxia (97%) have expansions of a GAA repeat in the first intron on both alleles of the gene encoding the mitochondrial protein frataxin [15, 16] whose expression is reduced in Friedreich ataxia [17]. The size of the GAA repeat expansion inversely correlates with frataxin expression and with the age of disease onset [16]. A deficiency of frataxin in cells leads to decreased activities of mitochondrial iron-sulfur cluster-containing enzymes, to an accumulation of iron in the mitochondrial matrix, increased sensitivity to oxidative stress, as well as to impaired adenosine triphosphate (ATP) production [18-20].

Current targets for disease-modifying drug development include agents targeting the mitochondria, aimed to (1) reduce oxidative stress and free-radical generation; (2) improve ATP production; (3) reduce iron accumulation; and (4) increase frataxin production and the assembly of iron-sulfur clusters [21]. There are presently 21 agents or classes of therapeutic agents enrolled in the research pipeline of Friedreich ataxia disease [39]. Millions of dollars from public, private, and industry-based initiatives have been dedicated to research of Friedreich ataxia therapeutics. Despite this vigorous international effort, there is as yet no proven disease-modifying therapy for Friedreich ataxia [22].

Development of E/PRT using the TAT delivery system in mitochondrial disorders was previously reported for lipoamide dehydrogenase (LAD) mitochondrial deficiency [23, 38]. LAD is the E3 subunit of the three α-ketoacid dehydrogenase complexes in the mitochondrial matrix, which are crucial for the metabolism of carbohydrates and amino acids. These complexes are the pyruvate dehydrogenase complex (PDHC), the α-ketoglutarate dehydrogenase complex (KGDHC) and the branched chain ketoacid dehydrogenase complex (BCKDHC). This previously reported TAT delivery system was based on a TAT-LAD fusion protein comprising the natural precursor sequence of the human LAD containing the N-terminal 35 amino acid mitochondrial targeting sequence (MTS). The natural MTS of LAD was used to facilitate processing of the TAT-LAD construct upon delivery into the mitochondria, thus allowing the incorporation of the delivered LAD into the α-ketoacid dehydrogenase complexes. This TAT-LAD construct was demonstrated to enter patients' cells rapidly, and efficiently reaching the mitochondria. Inside the mitochondria, TAT-LAD was shown to be processed and to restore LAD activity [23]. Moreover, delivery of TAT-LAD into E3-deficient mice tissues was also demonstrated [24]. In mice tissues, a single administration of TAT-LAD resulted in a significant increase in the enzymatic activity of the mitochondrial multienzyme complex pyruvate-dehydrogenase complex within the liver, heart and, most importantly, brain of TAT-LAD-treated E3-deficient mice [24].

Notably, TAT-LAD was shown to be able to restore the activity of the pyruvate dehydrogenase complex (PDHC) within treated patients' cells almost back to its normal levels. PDHC is a 9.5×10⁶ Da macromolecular machine whose multipart structure assembly process involves numerous different subunits: a pentagonal core of 60 units of the E2 component (dihydrolipoamide), attached to 30 tetramers of the E1 component (α2β2) (pyruvate decarboxylase), 12 dimers of the E3 (LAD, dihydrolipoamide) component and 12 units of the E3 binding protein. The structure of all α-ketoacid dehydrogenase complexes is similar to that of PDHC. The complexity of this structure emphasizes the significance in showing that TAT-mediated replacement of one mutated component restores the activity of an essential mitochondrial multi-component enzymatic complex in cells of enzyme-deficient patients.

Previous studies of mitochondria delivery system primarily used the native MTS of mitochondrial proteins (e.g. LAD) and showed that the native MTS was necessary for maximal restoration of LAD enzymatic function. Deleting the MTS restored a significantly smaller amount of LAD activity within the mitochondria. Since TAT can move both ways across a membrane and thus pull the therapeutic cargo out of the mitochondria, when MTS is included, the matrix processing peptidases recognizes the sequence and clips it, and the cargo (e.g. mature LAD) is left in the mitochondrial matrix while the TAT peptide can transduce out of the mitochondrion. Repeated dosing should therefore result in accumulating amounts of cargo in the mitochondria over time.

A TAT-FRATAXIN (TAT-FRA, also referred to as TAT-FXN) fusion protein for putative treatment of Friedreich's ataxia was recently reported [25]. This TAT-FXN fusion protein was shown to bind iron *in vitro,* transduce into the mitochondria of Friedreich ataxia deficient fibroblasts and also reduce caspase-3 activation in response to an exogenous iron-oxidant stress. In this TAT-FXN fusion protein, the authors used the native MTS of frataxin that consists of 80 amino acid residues (aa) for preparing their TAT-FXN fusion protein [26].

It is known that FXN mRNA is translated to a precursor polypeptide that is transported to the mitochondrial matrix and processed to at least two forms, namely FXN42-210 and FXN81-210. FXN42-210 is a transient processing intermediate, whereas FXN81-210 represents the mature protein [27, 28]. However, it was found that both FXN42-210 and FXN81-210 are present in control cell lines and tissues at steady-state, and that FXN42-210 is consistently more depleted than FXN81-210 in samples from Friedreich's ataxia patients [29].

Most nuclear-encoded mitochondrial proteins contain a cleavable N-terminal MTS that directs mitochondrial targeting of the protein; as detailed above, the N-terminal MTS is cleaved off by matrix processing proteases at a well-conserved RXY ↓ (S/A) motif, which is a three amino acid (aa) motif, where X can be any aa, followed by serine or alanine and cleavage is performed after the three first amino acids [26, 30-31]. These N-terminal MTSs are typically 15-30 amino acids in length including 3-5 nonconsecutive basic amino acid (arginine/lysine) residues, often with several serine/threonine residues but without acidic amino acid (asparate/glutamate) residues. In their molecular structure, these MTSs are able to form strong basic amphipathic α-helices that are essential for efficient mitochondrial transportation [32]. Thus, by way of example, the long 80-aa native MTS of frataxin as well as its two-step processing can reduce its efficiency in the delivery of cargos into the matrix of the mitochondria.

### SUMMARY OF THE INVENTION

The invention is defined by the appended claims.

Provided is a fusion protein comprising a HIV-1 transactivator of transcription (TAT) domain, a functional human mitochondrial protein and a human mitochondria targeting sequence (MTS) situated between said TAT domain and said functional human mitochondrial protein and wherein said human MTS is heterologous to said functional human mitochondrial protein, and wherein said functional mitochondrial protein is frataxin.

In the disclosed fusion protein said functional human mitochondrial protein can be situated C-terminal to said human MTS.

In the disclosed fusion protein said human mitochondrial protein can be a functional human mitochondrial protein *per se* and/or a component of a mitochondrial multi-component complex, for example human frataxin.

In the disclosed fusion protein, said MTS can comprise from 15 to 40 amino acid residues, including from 3 to 5 nonconsecutive basic amino acid residues. Optionally, said MTS may comprise from 1 to 3 or 4 or 5 serine/threonine residues.

Non-limiting examples of the MTS comprised in the disclosed fusion protein are any one of human mitochondrial citrate synthase MTS (the amino acid and the nucleic acid sequence encoding therefor are denoted by SEQ ID NO. 23 and SEQ ID NO. 3, respectively), the human lipoamide dehydrogenase MTS (the amino acid and the nucleic acid sequence encoding therefor are denoted by SEQ ID NO. 24 and SEQ ID NO. 5, respectively), the MTS of the human C6ORF66 gene product (the amino acid and the nucleic acid sequence encoding therefor are denoted by SEQ ID NO. 25 and SEQ ID NO. 4, respectively) and the MTS of human mitochondrial GLUD2 (encoded by the nucleic acid sequence denoted by SEQ ID NO. 16).

Further provided is a fusion protein comprising a HIV-1 transactivator of transcription (TAT) domain fused to a functional human mitochondrial protein and a human mitochondria targeting sequence (MTS) of a human mitochondrial protein selected from citrate synthase (CS) and lipoamide dehydrogenase (LAD) situated between said TAT domain and said functional human mitochondrial protein, wherein said functional human mitochondrial protein is C-terminal to said MTS of human lipoamide dehydrogenase or human citrate synthase, and wherein said MTS is frataxin.

The disclosed fusion protein as herein defined may further comprise a linker covalently linking said TAT domain to said MTS sequence.

In the disclosed fusion protein, the fusion protein may have the amino acid sequence denoted by SEQ ID NO. 30, comprising a HIV-1 transactivator of transcription (TAT) domain having the amino acid sequence denoted by SEQ ID NO. 27 fused to human frataxin having the amino acid sequence denoted by SEQ ID NO. 26 and a mitochondria targeting sequence (MTS) of human lipoamide dehydrogenase having the amino acid sequence denoted by SEQ ID NO. 24, said MTS situated between said TAT domain and said frataxin, and is linked to said TAT domain via a linker having the amino acid sequence denoted by SEQ ID NO. 32, and wherein said frataxin is C-terminal to said MTS of human lipoamide dehydrogenase.

In further embodiments of the disclosed fusion protein, the fusion protein may have the amino acid sequence denoted by SEQ ID NO. 28, comprising a HIV-1 transactivator of transcription (TAT) domain having the amino acid sequence denoted by SEQ ID NO. 27 fused to human frataxin having the amino acid sequence denoted by SEQ ID NO. 26 and a mitochondria targeting sequence (MTS) of human citrate synthase having the amino acid sequence denoted by SEQ ID NO. 23, said MTS situated between said TAT domain and said frataxin, and is linked to said TAT domain via a linker having the amino acid sequence denoted by SEQ ID NO. 32, and wherein said frataxin is C-terminal to said MTS of human citrate synthase.

Further disclosed is a composition comprising a physiologically acceptable carrier and as an active ingredient a fusion protein as disclosed herein, and a pharmaceutical composition comprising a physiologically or a pharmaceutically acceptable carrier and as an active ingredient a fusion protein as disclosed herein.

Further disclosed is a pharmaceutical composition for use as a medicament.

Further disclosed is a composition comprising a physiologically acceptable carrier and as an active ingredient a fusion protein comprising a HIV-1 transactivator of transcription (TAT) domain fused to human frataxin and a human mitochondria targeting sequence (MTS) of a human mitochondrial protein selected from citrate synthase (CS) and lipoamide dehydrogenase (LAD) situated between said TAT domain and said frataxin, wherein said frataxin is C-terminal to said MTS of human lipoamide dehydrogenase or human citrate synthase, as disclosed herein.

The present disclosure further provides a composition comprising as an active ingredient a fusion protein having the amino acid sequence denoted by SEQ ID NO. 30 or a fusion protein having the amino acid sequence denoted by SEQ ID NO. 28 and a physiologically acceptable carrier.

The pharmaceutical composition disclosed herein can be intended for use in treating or alleviating Friedreich's ataxia.

Further disclosed is a pharmaceutical composition for use in the treatment of Friedreich's Ataxia, wherein the composition is for intravenous administration to a subject in need thereof, said composition comprising a therapeutically effective amount of a fusion protein having the amino acid sequence denoted by SEQ ID NO. 30 or a fusion protein having the amino acid sequence denoted by SEQ ID NO. 28 and at least one of pharmaceutically acceptable carrier, diluent, additive and excipient.

A non-limiting example of a pharmaceutical composition as herein defined is wherein the therapeutically effective amount administered is from 0.5 mg/Kg to 2 mg/Kg body weight of the subject.

The disclosed medical use can further comprise administering an additional therapeutic agent.

In the disclosed medical use said introduced functional human mitochondrial protein may restore at least partial activity of a wild type human mitochondrial protein, i.e., frataxin, at least 5%, at least 10%, at least 20%, at least 30%, at least 40%, at least 50%, at least 60%, at least 70%, at least 80%, at least 90% or up to 100% of the activity of a wild type human mitochondrial protein.

Still further, in the disclosed medical use, a therapeutically effective amount of the fusion protein disclosed herein alleviates oxidative stress in said subject.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Figure 1A-1D****: Schematic structures of the various TAT-MTS-FRA fusion proteins and the expected molecular weights thereof**
   Abbreviations: H, His tag; TAT, transactivator of transcription; FRA (or fra), Frataxin; MTS, mitochondrial translocation sequence; cs, Citrate synthase; orf, C6ORF66; lad, LAD and kDa, kilo Dalton.
**Figure 2A-2D****: Expression and sub-cellular localization of TAT-MTSfra-FRA and of TAT-MTSorf FRA fusion proteins**

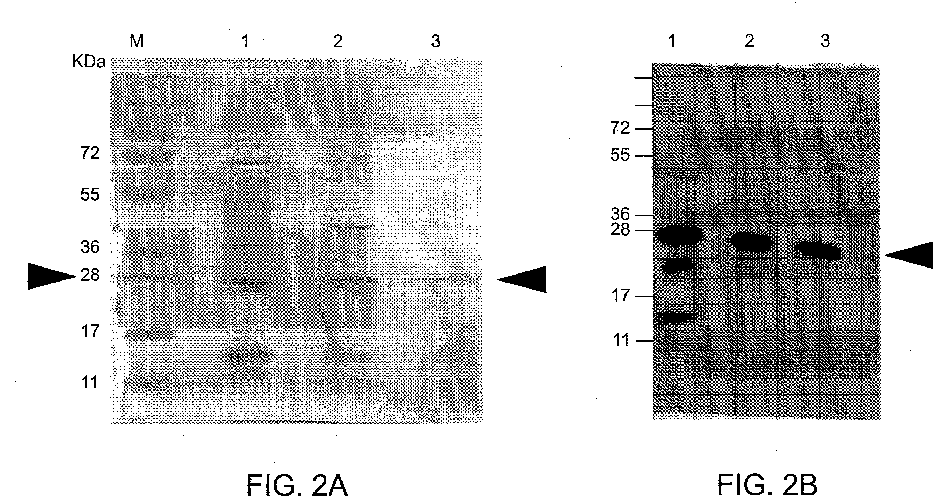

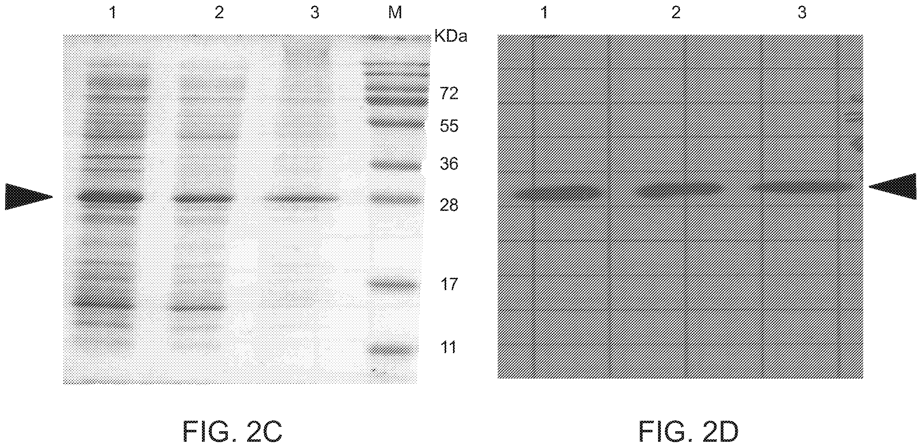

   Figure 2 presents an image of SDS-PAGE analysis of bacterial sub-fractions expressing the TAT-MTSfra-FRA fusion protein **(****Fig. 2A****)** and an immunoblot of Western blot analysis thereof **(****Fig. 2B****)** using anti-His antibody; an image of SDS-PAGE analysis of bacterial sub-fractions expressing TAT-MTSorf-FRA fusion protein **(****Fig. 2C****)** and an immunoblot of Western blot analysis thereof using anti-His antibody is presented in **Fig. 2D****.**
   Abbreviations (for Figs. 2A-2D): 1, whole cell extract; 2, soluble fraction; 3, insoluble fraction; kDa, kilo Dalton; and M = marker. Arrow heads indicate the fusion proteins.

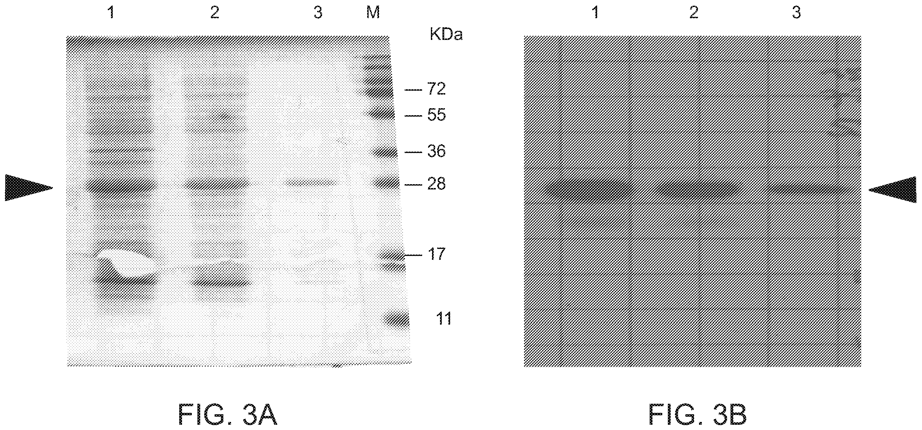

**Figure 3A-3D****: Expression and sub-cellular localization of TAT-MTSlad-FRA and TAT-MTScs-FRA fusion proteins**

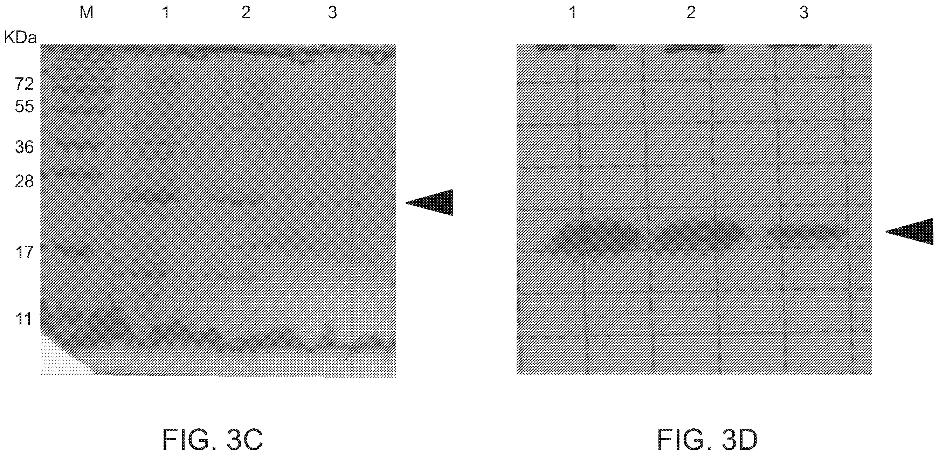

   Figure 3 presents an image of SDS-PAGE analysis of bacterial sub-fractions expressing the TAT-MTSlad-FRA fusion protein **(****Fig. 3A****)** and an immunoblot of Western blot analysis thereof **(****Fig. 3B****)** using anti-His antibody; an image of SDS-PAGE analysis of bacterial sub-fractions expressing TAT-MTScs-FRA fusion protein **(****Fig. 3C****)** and an immunoblot of Western blot analysis thereof using anti-His antibody is presented in **Fig. 3D****.**
   Abbreviations (for Fig. 3A-3D): 1, whole cell extract; 2, soluble fraction; 3, insoluble fraction; KDa, kilo Dalton; and M=marker. Arrow heads indicate the fusion proteins.

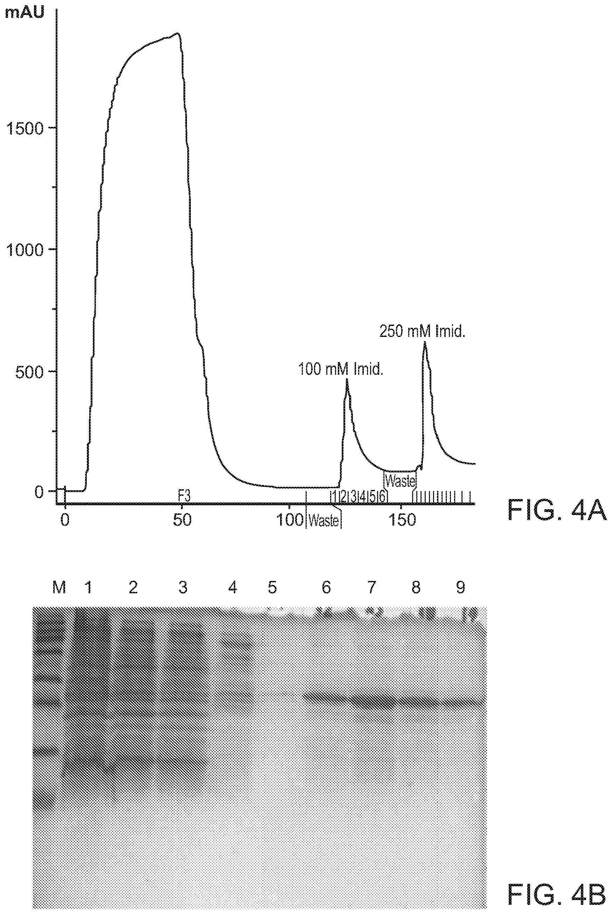

**Figure 4A-4D****: Purification of TAT-MTSfra-FRA and of TAT-MTSorf-FRA fusion proteins**

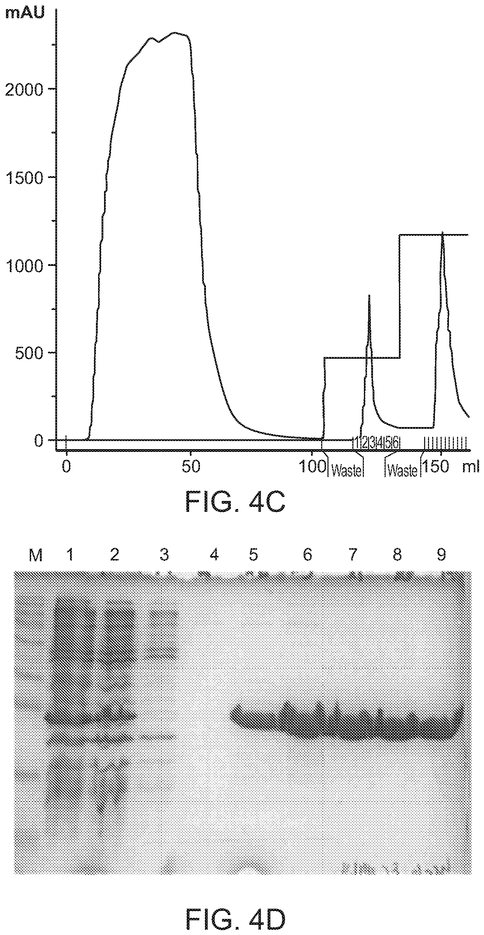

   Figure 4 presents an image of an affinity chromatography purification profile of the fusion protein TAT-MTSfra-FRA **(****Fig. 4A****)** and an image of SDS-PAGE analysis of the purification steps obtained for TAT-MTSfra-FRA is presented in **Fig. 4B****.** An image of an affinity chromatography purification profile of the fusion protein TAT-MTSorf-FRA is presented in **Fig. 4C** and an image of SDS-PAGE analysis of the purification steps obtained for TAT-MTSorf-FRA is presented in **Fig. 4D****.**
   Abbreviations: M, marker; 1, whole cell extract; 2, pre-run fraction (the soluble sub-fraction of bacterial cells expressing the fusion protein); 3, flow through; 4, elution with 100 mM imidazole; and 5-9, elution with 250 mM imidazole.

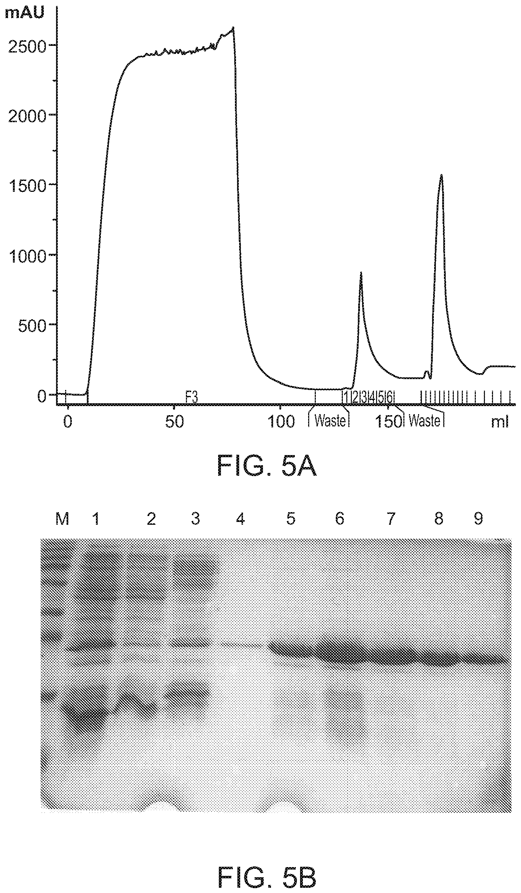

**Figure 5A-5D****: Purification of TAT-MTSlad-FRA and of TAT-MTScs-FRA fusion proteins**

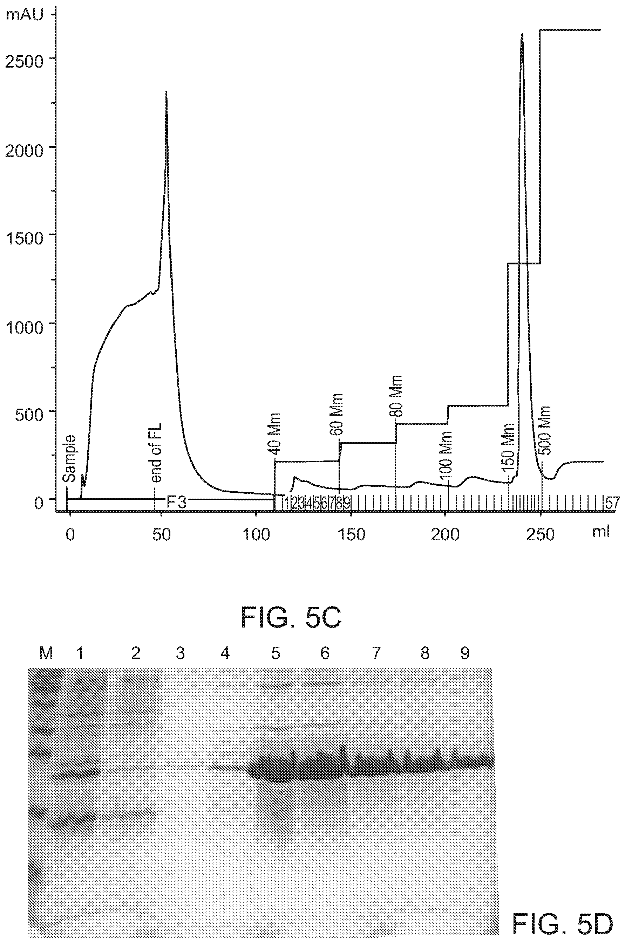

   Figure 5 presents an image of an affinity chromatography purification profile of the fusion protein TAT-MTSlad-FRA **(****Fig. 5A****)** and an image of SDS-PAGE analysis of the purification steps obtained for TAT-MTSlad-FRA is presented in **Fig. 5B****.** An image of an affinity chromatography purification profile of the fusion protein TAT-MTScs-FRA is presented in **Fig. 5C** and an image of SDS-PAGE analysis of the purification steps obtained for TAT-MTScs-FRA is presented in **Fig. 5D****.**
   Abbreviations: M, marker; 1, whole cell extract; 2, pre-run fraction (the soluble sub-fraction of bacterial cells expressing the fusion protein); 3, flow through; 4, elution with 100 mM imidazole; and 5-9, elution with 250 mM imidazole.

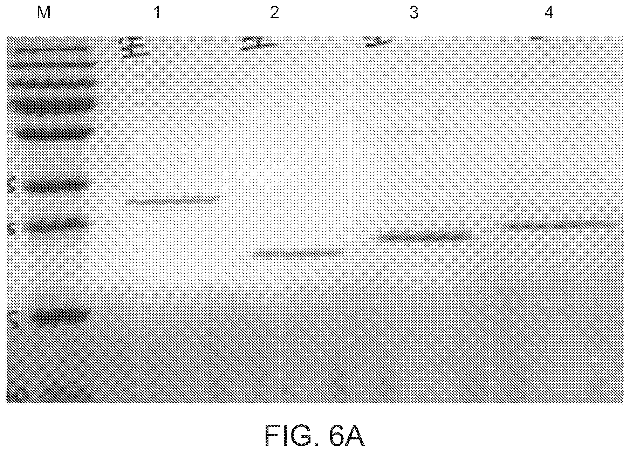

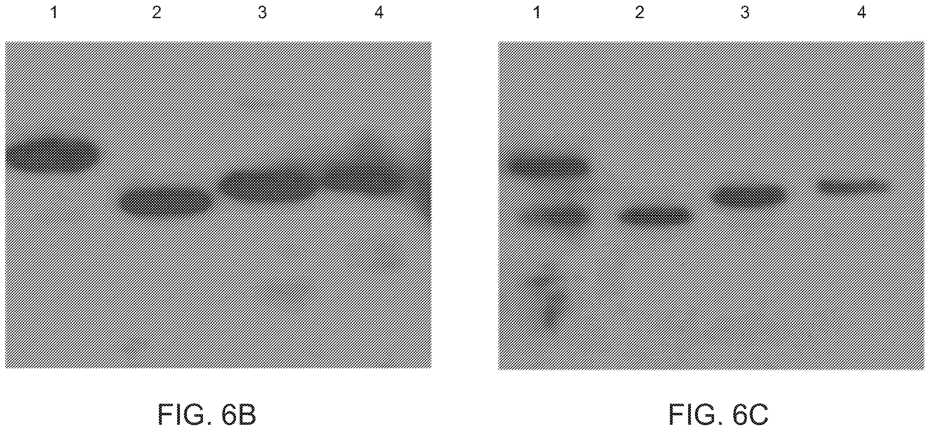

**Figure 6A-6C****: Characterization of TAT-MTS-FRA highly purified fusion proteins** The four highly purified TAT-MTS-FRA fusion proteins were characterized by a SDS-PAGE gel **(****Fig. 6A****)** and by Western blot analyses using anti-His **(****Fig. 6B****)** or anti-frataxin **(****Fig. 6C****)** antibodies.
   Abbreviations: 1, TAT-MTSfra-FRA; 2, TAT-MTScs-FRA; 3, TAT-TSlad-FRA; 4, TAT-MTSorf-FRA; and M, Marker.

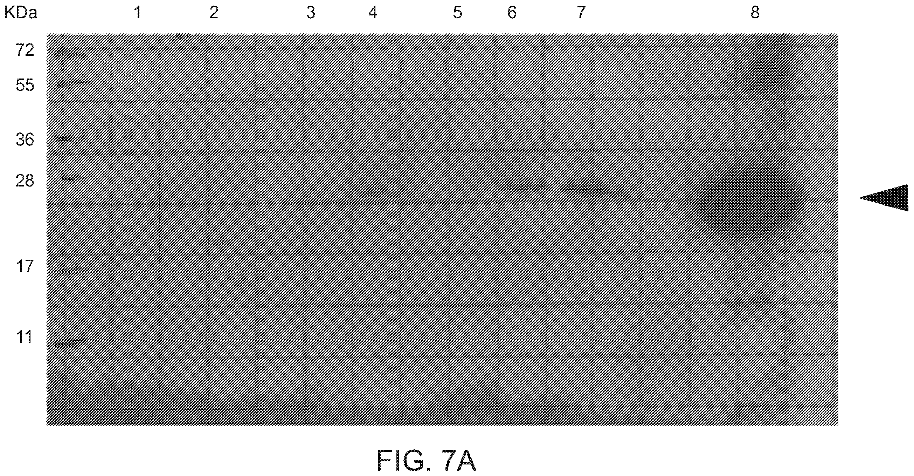

**Figure 7A-7B****: Internalization of TAT-MTSlad-FRA into cells and their mitochondria**

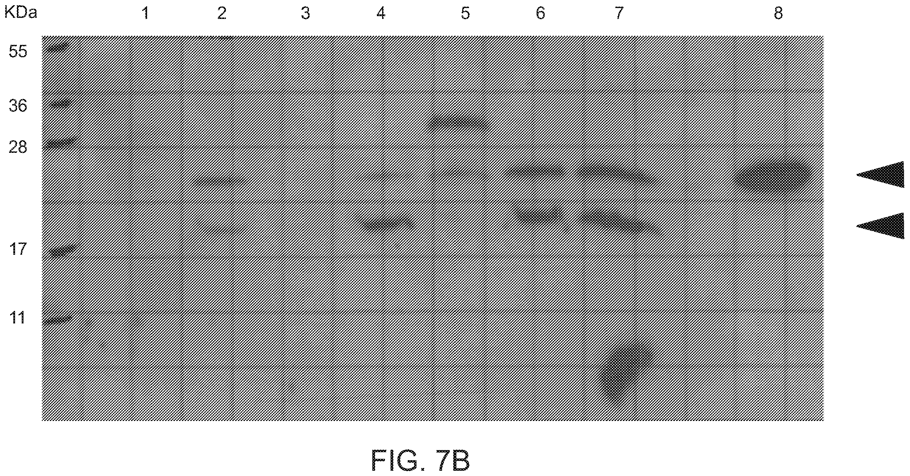

   **Fig. 7A** presents an immunoblot of a Western blot analysis using anti-His antibodies and **Fig. 7B** presents an immunoblot of a Western blot analysis using anti frataxin antibodies performed with BJAB cells incubated in the absence (lanes 1 & 2) or in the presence of TAT-MTSlad-FRA (lanes 3-7). At the end of the incubation period, sub-fractionation was performed, obtaining the cytoplasmic and mitochondrial fractions. Fractions were separated by SDS-PAGE and subjected to Western blot analysis. Abbreviations: control, untreated cells: cytoplasm (1), mitochondria (2); cells treated for 1hr with the fusion protein: cytoplasm (3), mitochondria (4); cells treated for 5hr: cytoplasm (5), mitochondria (6, 7; from two separate experiments); highly purified TAT-MTSlad-FRA fusion protein as a positive control is shown in (8). Arrow-heads indicate the fusion protein (or its processing products, indicated by the lower arrowhead in Figure 7B).

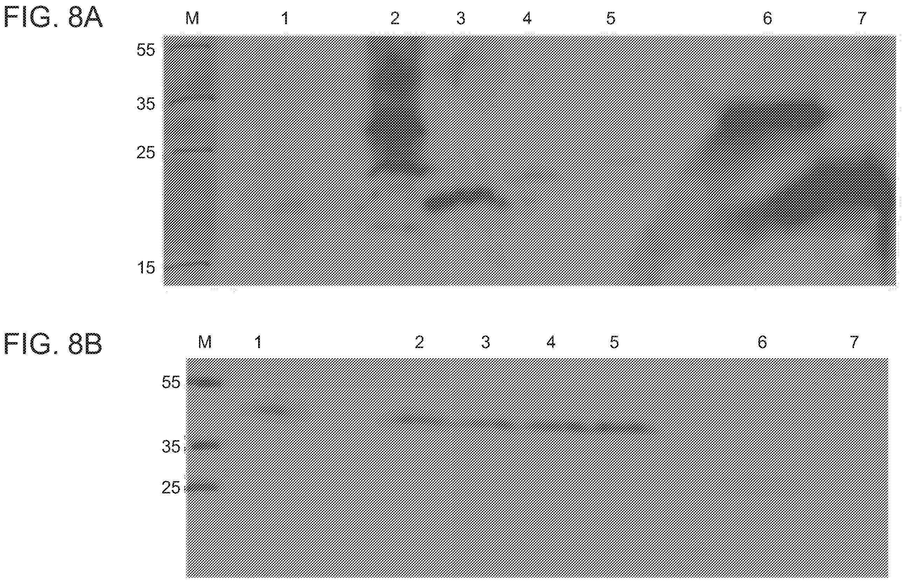

**Figure 8A-8B****: Internalization of TAT-MTS-FRA fusion proteins into mitochondria of cells**
   **Fig. 8A** presents a Western blot analysis using anti-frataxin antibodies of cells incubated for 3 hours with TAT-MTS-FRA fusion proteins, each fusion protein at a final concentration of 0.02 µg/µl. The cells were washed and their mitochondria were isolated.
   **Fig. 8B** presents a Western blot analysis using anti-E1α antibodies of cells as detailed above.
   Abbreviations: M, marker; mitochondria isolated from control cells without any treatment (1), cells incubated with TAT-MTSfra-FRA (2), cells incubated with TAT-MTScs-FRA (3), cells incubated with TAT-MTSlad-FRA (4), cells incubated with TAT-MTSorf-FRA (5), purified TAT-MTSfra-FRA fusion protein (6), and purified TAT-MTScs-FRA fusion protein (7).

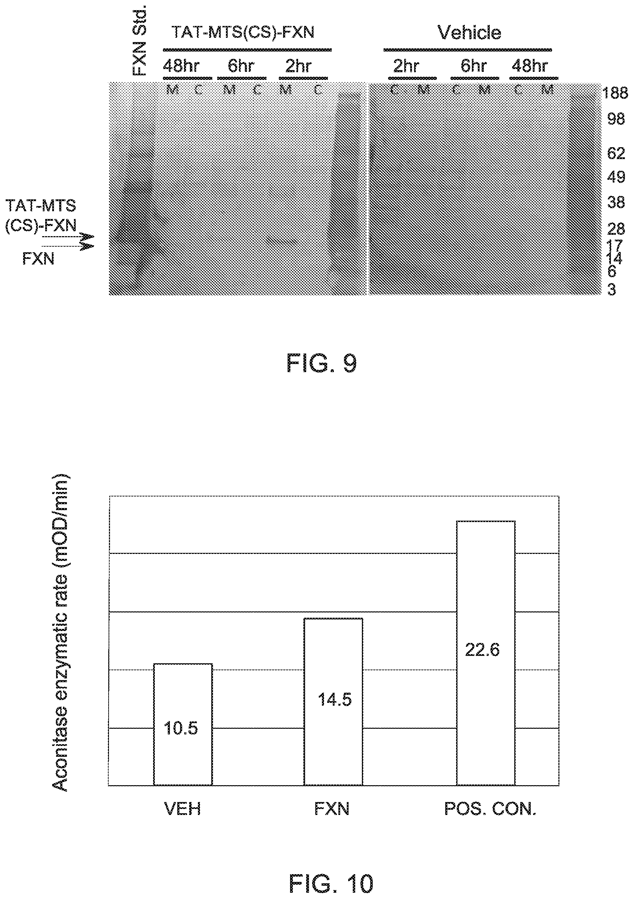

**Figure 9****: Internalization of TAT-MTScs-FRA to fibroblasts of FRA patients**
   An immunoblot of a Western blot analysis using an anti-Frataxin antibody performed for mitochondrial (M) and cytosolic (C) fractions of fibroblasts (F816) obtained from Friedreich's ataxia patients that were incubated in the presence of 20 µg/ml TAT-MTScs-FRA (or vehicle) for 2, 6 and 48 hours, with fresh addition of TAT-MTScs-FRA (at 20 µg/ml) after 24 hours. TAT-MTScs-FRA and processed Frataxin are marked with arrows. Abbreviations: kDa, kilodalton; TAT, transactivator of transcription; MTS, mitochondria targeting sequence; CS, citrate synthase; FXN, frataxin; Std, standard.
**Figure 10****: Aconitase activity in fibroblasts obtained from Friedreich's ataxia patients following 48 hours incubation with TAT-MTScs-FRA**
   A bar graph showing aconitase activity (mOD/min) of mitochondrial fractions obtained from Friedreich's ataxia patients' fibroblasts (F816) incubated for 48 hours with either 20µg/ml TAT-MTScs-FRA protein or vehicle (FXN or VEH, respectively). HepG2 whole cells homogenate served as positive control (POS.CON). Abbreviations: kDa, kilodalton; TAT, transactivator of transcription; MTS, mitochondria targeting sequence; CS, citrate synthase; FXN, frataxin; Std, standard; VEH, vehicle, POS. CON., positive control.

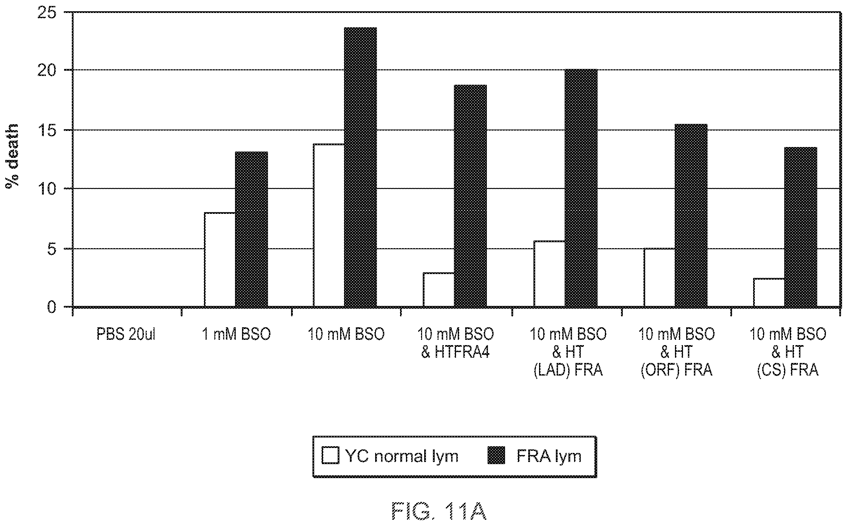

**Figure 11A-11B****: TAT-MTS-FRA fusion proteins partially rescue cells from BSO-induced oxidative stress**
   **Fig. 11A** presents a bar diagram showing percentage of cell death induced by L-Buthionine-sulfoximine (BSO). Normal lymphocytes or lymphocytes obtained from Friedreich's ataxia (FRDA) patients (Lym 43) were seeded, incubated for 5 hr with the various TAT-MTS-FRA fusion proteins, after which BSO at different concentrations was added for additional 48 hr. At the end of the incubation time, cell cultures were subjected to cell proliferation assays.

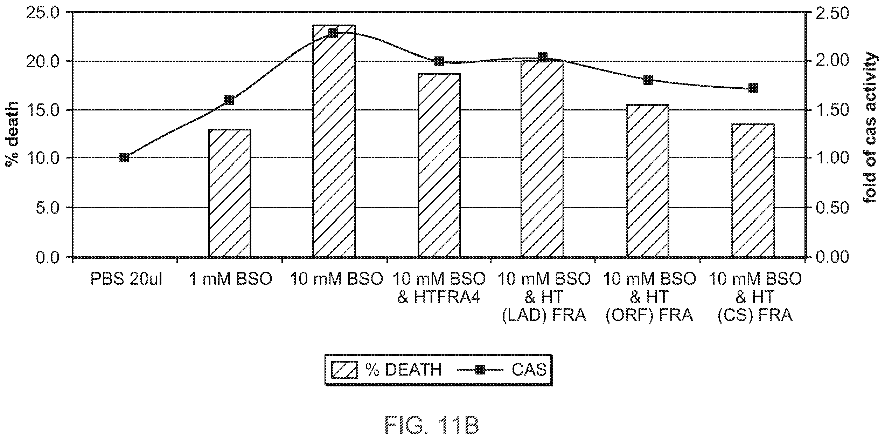

   **Fig. 11B** presents a bar diagram showing percentage of cell death of Lym 43 induced by L-Buthionine-sulfoximine (BSO) correlated with caspase 3 activity within the cells, assessed using the Apo-ONE Homogeneous Caspase 3/7 Assay Kit (Promega). Experiments were carried in parallel with cell viability assays. Abbreviations: %, percent; YC, normal lymphocytes; FRA lym, lymphocytes obtained from Friedreich's ataxia patients; HTFRA=TAT-MTSfra-FRA, HT(LAD)FRA=TAT-MTSlad-FRA, HT(ORF)FRA=TAT-MTSorf-FRA, HT(CS)FRA=TAT-MTScs-FRA; BSO, L-Buthionine-sulfoximine; and PBS, Phosphate buffered saline.

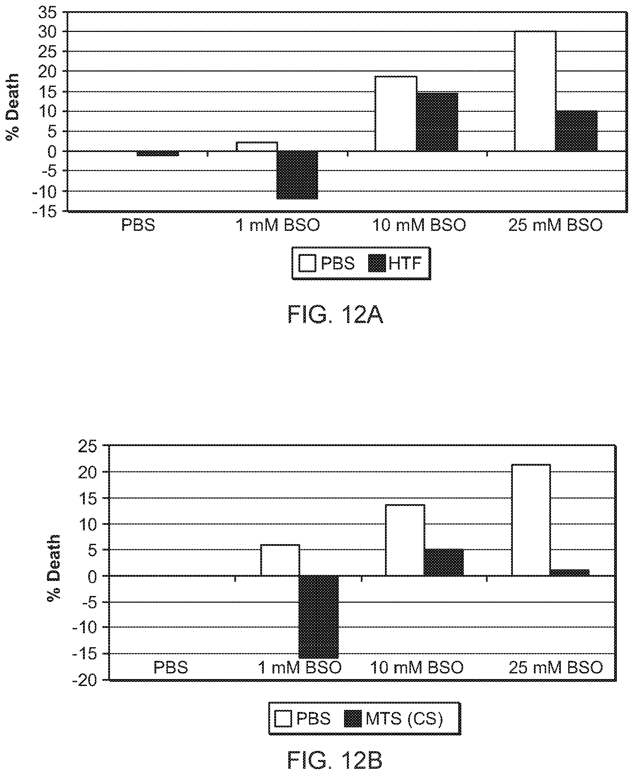

**Figure 12A-12D****: TAT-MTS-FRA fusion proteins partially rescue fibroblasts obtained from patients from BSO-induced oxidative stress, a comparison**

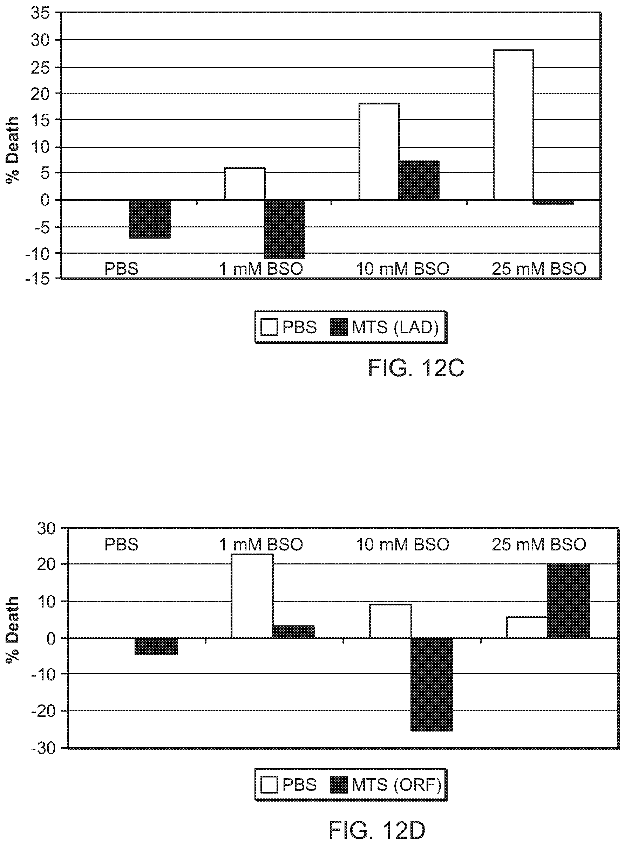

   **Fig. 12A** to **Fig. 12D** present bar diagrams of percentage of cell death induced by BSO in fibroblasts obtained from FRDA patients (Fib. 78). Cells were seeded, incubated for 24 hr with the TAT-MTSfra-FRA **(****Fig. 12A****),** TAT-MTScs-FRA **(****Fig. 12B****),** TAT-MTSlad-FRA **(****Fig. 12C****)** and with the TAT-MTSorf-FRA **(****Fig. 12D****)** fusion protein, after which BSO at different concentrations was added for additional 48 hr. At the end of the incubation time, cell cultures were subjected to cell proliferation assays. Abbreviations: %, percent; PBS, Phosphate buffered saline; BSO, L-Buthionine-sulfoximine; HTF, TAT-MTSfra-FRA; MTS (cs), TAT-MTScs-FRA; MTS (LAD), TAT-MTSlad-FRA; MTS (ORF), TAT-MTSorf-FRA.

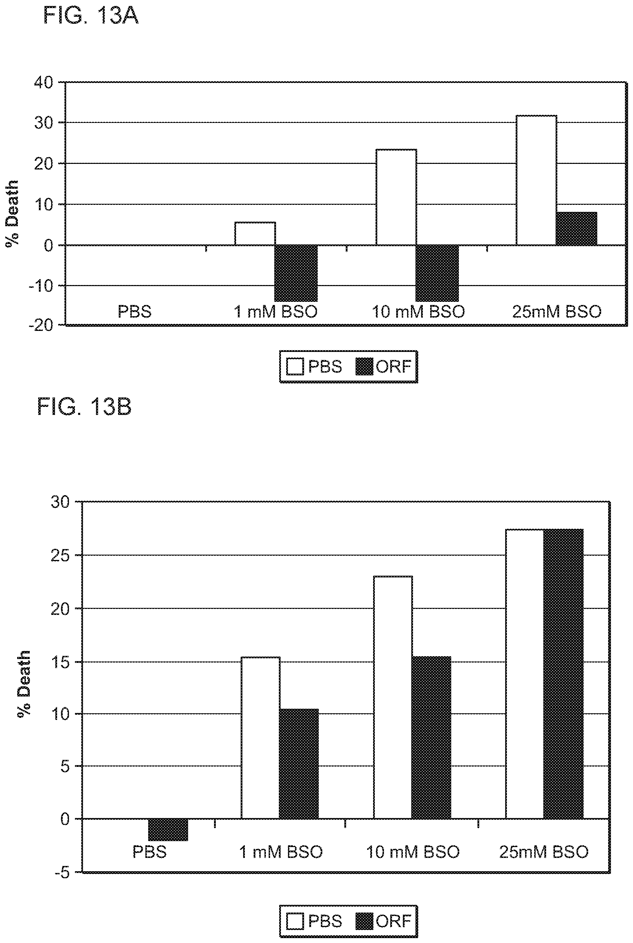

**Figure 13A-13B****: TAT-MTSorf-FRA rescues lymphocytes and fibroblasts obtained from Friedreich's ataxia patients from BSO-induced oxidative stress**
   **Fig. 13A** presents a bar diagram of percentage of cell death induced by BSO in fibroblasts obtained from FRDA patients (Fib. 78) and **Fig. 13B** presents a bar diagram of percentage of cell death induced by BSO in lymphocytes obtained from patients (Lym 43). Cells were seeded, incubated for 24 hr with the TAT-MTSorf-FRA fusion protein, after which BSO at different concentrations was added for additional 48 hr. At the of the incubation time, cell cultures were subjected to cell proliferation assays. Abbreviations: PBS, Phosphate buffered saline; %, percent; BSO, L-Buthionine-sulfoximine; and ORF, C6ORF66.

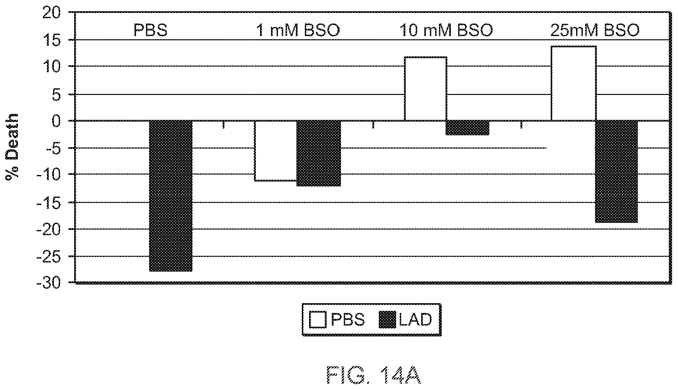

**Figure 14A-14B****: TAT-MTSlad-FRA rescues fibroblasts obtained from patients from BSO-induced oxidative stress**

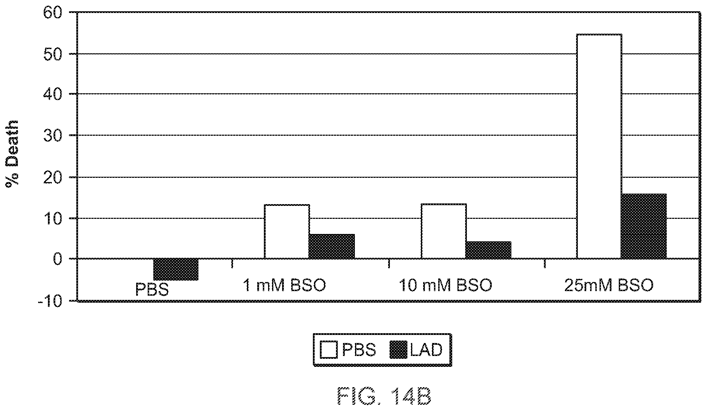

   **Fig. 14A** and **Fig. 14B** present bar diagrams of percentage of cell death induced by BSO in fibroblasts obtained from FRDA patients (Fib. 78) of two representative experiments. Cells were seeded, incubated for 24 hr with the TAT-MTSlad-FRA fusion protein, after which BSO at different concentrations was added for additional 48 hr. At the of the incubation time, cell cultures were subjected to cell proliferation assays. Abbreviations: %, percent; PBS, Phosphate buffered saline; BSO, L-Buthionine-sulfoximine; LAD, lipoamide dehydrogenase.

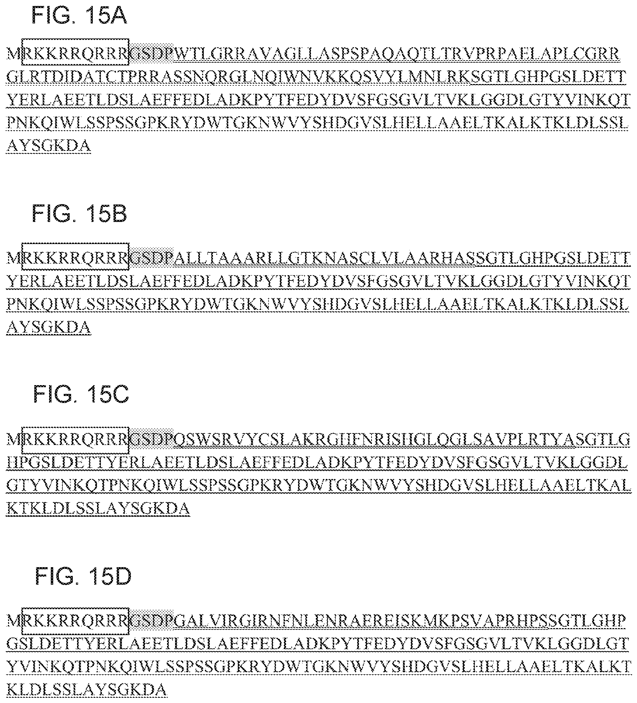

**Figure 15A-15D****: TAT-MTS-FRA fusion proteins constructs**
   **Fig. 15A** to **Fig. 15D** are schematic presentations of TAT-MTS-FRA fusion protein constructs comprising a HIV-1 transactivator of transcription (TAT) domain (boxed) fused to a GSDP linker (colored in grey) fused to a human mitochondria targeting sequence (MTS) of a human mitochondrial protein (double-underlined) selected from frataxin (MTSfra, **Fig. 15A**), citrate synthase (MTScs, **Fig. 15B**), lipoamide dehydrogenase (MTSlad, **Fig. 15C**) and C6ORF66 (MTSorf, **Fig. 15D**) fused to human frataxin (underlined).

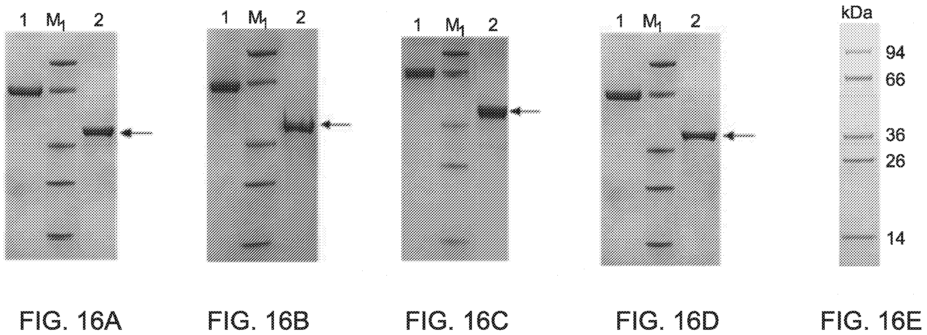

**Figure 16A-16E****: Expression and purification of TAT-MTS-OTC protein constructs**
   **Fig. 16A** to **Fig. 16D** are images of sodium dodecyl sulfate polyacrylamide gel electrophoresis (SDS PAGE) analysis of bacterially expressed TAT-MTS-OTC fusion protein constructs comprising ornithine transcarbamoylase (OTC, Fig. 16A), citrate synthase (CS, Fig. 16B), C6ORF66 (ORF, Fig. 16C) or lipoamide dehydrogenase (LAD Fig. 16D) as their MTS. The fusion protein construct is indicated with arrows. About 2µg of each protein and BSA were analyzed on 4-20% gels, followed by Coomassie blue staining. Marker sizes are presented in Fig. 16E. Abbreviations: M1, protein marker; kDa, kilodalton.

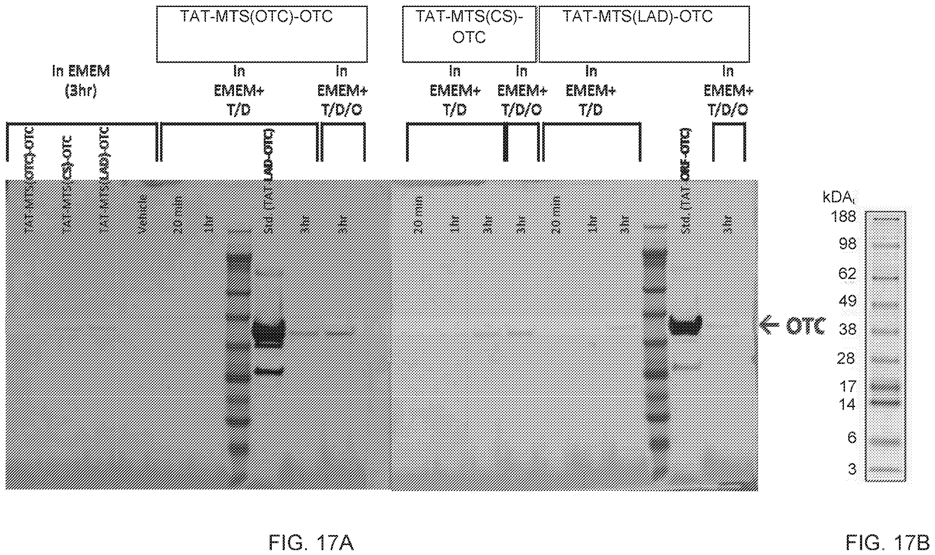

**Figure 17A-17B****: Internalization of TAT-MTS-OTC protein constructs into mitochondria**
   **Fig. 17A** is an image of a Western blot analysis performed for mitochondrial fractions of HepG2 cells incubated with 12 µg/ml of the TAT-MTS-OTC fusion protein TAT-MTSotc-OTC, TAT-MTScs-OTC, TAT-MTSlad-OTC or vehicle for 20 min, 1 and 3 hours in either Eagle's Minimum Essential Medium (EMEM) alone or EMEM supplemented with DMSO (D), Trehalose (T), or Ornithine (O), as indicated. The protein standards TAT-MTSlad-OTC or TAT-MTSorf-OTC (Std.) were run in parallel, sizes of the protein markers (M) are presented in **Fig. 17B****.** Migration of the OTC fusion protein is marked by an arrow. Abbreviations: EMEM, Eagle's Minimum Essential Medium; TAT, transactivator of transcription; MTS, mitochondria targeting sequence; OTC, ornithine transcarbamoylase; CS, citrate synthase; LAD, lipoamide dehydrogenase; Std., standard; T/D, Trehalose/DMSO; T/D/O, Trehalose/DMSO/Ornithine; M, marker.

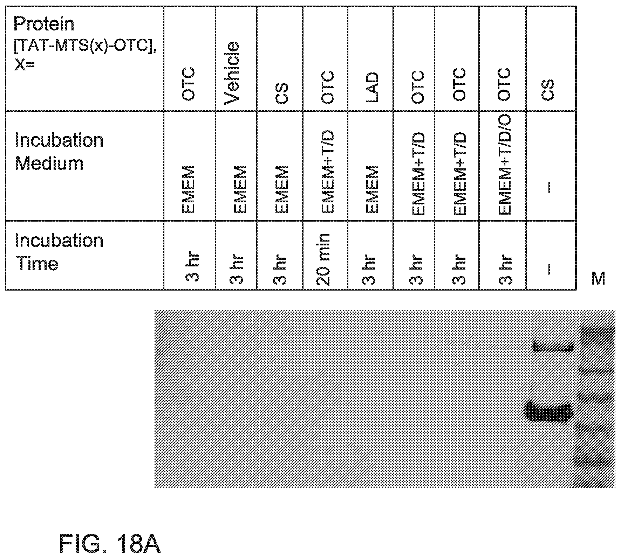

**Figure 18A-18B****: Western blot analysis of cytosolic fractions of HepG2 cells incubated with TAT-MTS-OTC protein constructs**

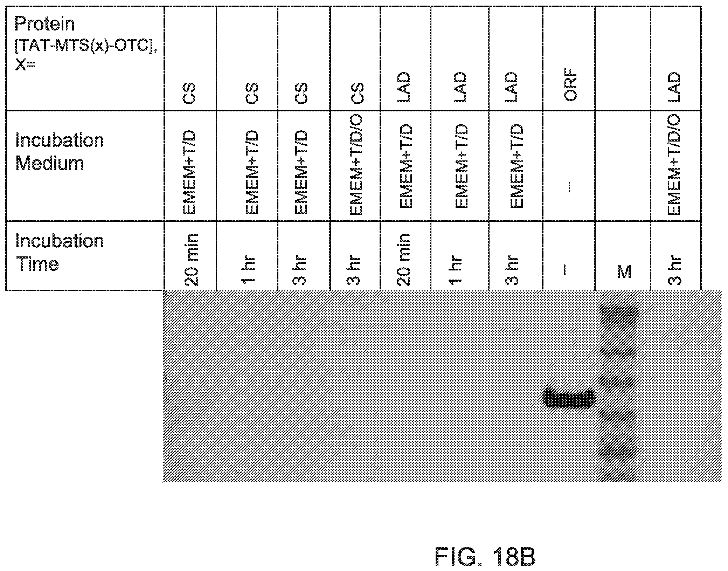

   **Fig. 18A** and **Fig. 18B** are images of Western blot analyses performed for cytosolic fractions of HepG2 cells incubated with 12 µg/ml of the TAT-MTS-OTC fusion protein TAT-MTSotc-OTC, TAT-MTScs-OTC, TAT-MTSlad-OTC or vehicle for 20 min, 1 and 3 hours in either EMEM alone or EMEM supplemented with DMSO (D), Trehalose (T), or Ornithine (O), as indicated. The protein standards TAT-MTScs-OTC or TAT-MTSorf-OTC (Std.) were run in parallel. Abbreviations: OTC, ornithine transcarbamoylase; CS, citrate synthase; LAD, lipoamide dehydrogenase; ORF, C6ORF66; EMEM, Eagle's Minimum Essential Medium; T/D, Trehalose/DMSO; T/D/O, Trehalose/DMSO/Ornithine; M, marker.

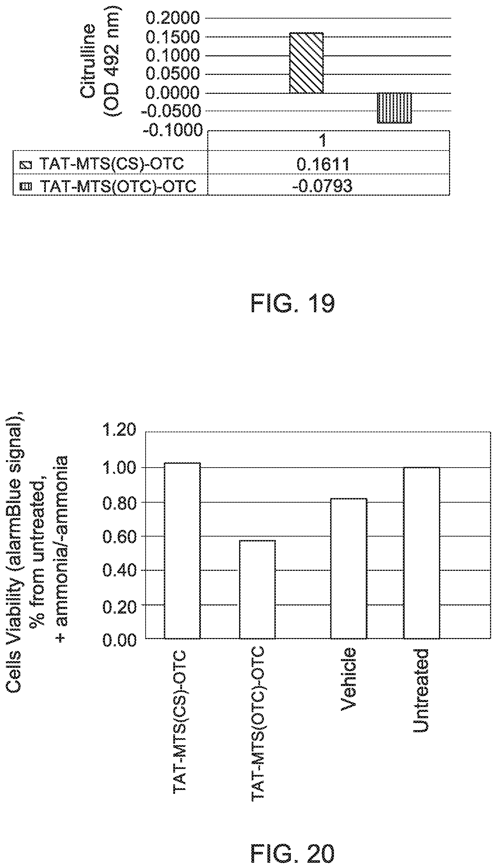

**Figure 19****: *In vitro* enzymatic activity of OTC**
   A bar graph showing the level of *in vitro* enzymatic activities of the OTC protein constructs TAT-MTScs-OTC and TAT-MTSotc-OTC measured by detecting the net absorbance of citrulline at an optical density (O.D.) of 492 nm compared to a control. Abbreviations: TAT, transactivator of transcription; MTS, mitochondria targeting sequence; OTC, ornithine transcarbamoylase; CS, citrate synthase.
**Figure 20****: Rescue from ammonia stress by OTC fusion proteins**
   A bar graph showing cell viability of HepG2 cells suffering from ammonia stress in the presence of 14µg/ml of the OTC protein constructs TAT-MTScs-OTC, and TAT-MTSotc-OTC, compared to vehicle-treated and untreated cells. Florescence signal was calculated as the ratio between Ammonium chloride treated and non-treated cells. Abbreviations: TAT, transactivator of transcription; MTS, mitochondria targeting sequence; CS, citrate synthase; OTC, ornithine transcarbamoylase.

### DETAILED DESCRIPTION OF EMBODIMENTS

The presently disclosed subject matter relates to the preparation of various plasmid constructs encoding TAT-MTS-Frataxin fusion proteins, providing a basis of a wide-range therapeutic tool for delivering mitochondrial proteins into mitochondria.

The protein constructs described herein, comprising a mitochondrial protein as well as TAT and a specific mitochondrial targeting sequence (MTS), enabling the mitochondrial protein to cross both cellular and mitochondrial membranes, were expressed and purified and their biological activity was verified. Remarkably, the protein yield obtained for fusion protein constructs comprising an MTS which was other than the native MTS of the mitochondrial protein present in the fusion construct (e.g., a frataxin fusion protein construct with MTS heterologous to frataxin), was superior to the yield obtained for a frataxin fusion protein construct comprising the native MTS of frataxin.

As demonstrated below, the inventors show that various fusion proteins are able to enter the mitochondria within intact cells. In addition, the inventors show that the fusion proteins exhibit biological activity. For example, the various TAT-MTS-FRA fusion proteins were shown to rescue cells obtained from Friedreich ataxia patients as well as normal cells from oxidative stress, as demonstrated in the Examples below. Surprisingly, a superior protective effect was observed for fusion proteins carrying an MTS, which was other than the native MTS of the functional mitochondrial protein present in the fusion construct (i.e. a heterologous MTS) as compared to the effect demonstrated by the fusion protein constructs carrying the native MTS.

The presently disclosed subject matter provides fusion protein constructs comprising heterologous MTSs of human nuclear-encoded mitochondrial proteins that are classical MTS sequences, which are known to be removed upon entry to the mitochondria. By a non-limiting example, a delivery system as herein described comprising frataxin may be used for the treatment or alleviation of Friedreich's ataxia or any other disorder associated with a deficiency of frataxin or defective frataxin.

Thus, the presently disclosed subject matter provides a fusion protein comprising a HIV-1 transactivator of transcription (TAT) domain, a functional human mitochondrial protein and a human mitochondria targeting sequence (MTS) situated between said TAT domain and said functional human mitochondrial protein and wherein said human MTS is heterologous to said functional human mitochondrial protein.

The term "*functional human mitochondrial protein*" as used herein refers to any protein which is essential for a biological activity of mitochondria. A functional human mitochondrial protein may be a protein, which is active when present in the mitochondria by itself (*per se*) or a protein that when present in the mitochondria functions as a component of a mithochondrial multi-component complex (i.e. with other enzymes, co-factors, or proteins). Typically, a functional human mithochondrial protein is a protein, which, when absent, deficient or mutated, causes a mitochondrial disorder or is associated with a mitochondrial disorder.

In some specific embodiments, the functional mitochondrial protein refers to the full-length amino acid sequence of the protein. In other embodiments, the functional mitochondrial protein is a fragment of the full-length amino acid sequence, sufficient to provide the mitochondrial protein activity, either alone or as part of a multi-component complex, as appropriate.

In further embodiments, the functional human mitochondrial protein is a mutated derivative of said protein, wherein one or more of the native amino acid residues has been deleted, replaced or modified while still maintaining the mitochondrial functionally of the protein (alone or as part of a multi-component complex).

In the above and other embodiments, the functional human mitochondrial protein (also denoted "*mature*" protein) refers to a protein devoid of its mitochondrial targeting sequence (MTS). In other words, the fusion protein construct herein provided comprises a functional mitochondrial protein, which, upon entry to the mitochondria is cleaved off from the fusion protein construct in its mature, active (functional) state.

By way of non-limiting example, in the above and other embodiments of the disclosed subject matter, the functional human mitochondrial protein whose activity is supplied by a fusion protein of the present invention may be human frataxin (the mature protein having the amino acid sequence denoted by SEQ ID NO. 26 and encoded by the nucleic acid sequence denoted by SEQ ID NO. 6).

In some embodiments, the human mitochondrial protein is a functional mitochondrial protein *per se* and/or is a component of a mitochondrial multi-component complex.

As indicated above, the functional human mitochondrial protein of the disclosed subject matter may be a protein which is active when present in the mitochondria by itself (i.e. the protein *per se* is active) or a protein that when present in the mitochondria functions as a component of a mitochondrial multi-component complex (i.e. with other enzymes, co-factors, or proteins). The term "*mitochondrial multi-component complex*" as used herein refers to an enzyme that forms a complex with other enzymes or proteins that is essential for a biological activity of mitochondria.

As shown in the Examples below (Figure 7), the fusion protein comprising a TAT and MTS sequences is cleaved upon entry into the mitochondria, and a mature active protein is obtained. The protein constructs provided by the presently disclosed subject matter thus allow a human mitochondrial protein, which is first covalently attached to TAT and MTS domains, to cross both cellular and mitochondrial membranes, and once inside the mitochondria, be processed by mitochondrial peptidases while retaining their biological activity and proper conformation. The delivery system described herein thus enables a human mitochondrial protein to retain its mitochondrial function *per se* or the integration thereof in a mitochondrial multi-component complex.

The mitochondrial multi-component complex encompassed by the present disclosure refers to a group of at least two different proteins assembled together in a specific ratio that functions in a coordinated fashion to catalyze a series of reactions. The function of a mitochondrial multi-component complex is dependent on its structure; thus, the proteins that compose the complex must properly fold and physically fit together in the proper configuration in order to efficiently catalyze the series of reactions.

In all embodiments, the functional human mitochondrial protein according to presently disclosed subject matter is cleaved off from the fusion protein construct upon entry to the mitochondria and resides therein at its mature, properly-folded active state. In some embodiments, the functional human mitochondrial protein may readily then integrate into a conformationally-sensitive mitochondrial multi-component complex.

By way of non-limiting example, the presently disclosed subject matter encompasses a mitochondrial multi-component complex which is any one of pyruvate dehydrogenase complex (PDHC), α-ketoglutarate dehydrogenase complex (KGDHC), and branched-chain keto-acid dehydrogenase complex (BCKDHC), the complexes of the respiratory chain, and those involved in fatty acid β-oxidation and the urea cycle. The complexes of the respiratory chain are complex I (NADH-ubiquinone oxidoreductase), complex II (succinate-ubiquinone oxidoreductase), complex III (ubiquinol-ferricytochrome C oxidoreductase), complex IV (Cytochrome C oxidoreductase), and complex V (FIFO ATPase) where each mitochondrial multicomponent complex represents a separate embodiment of the present invention.

As shown in Examples 1-3 below, the inventors have cloned, expressed and purified fusion protein constructs comprising the protein frataxin.

The mitochondrial protein human frataxin (FXN) is an essential and highly conserved protein expressed in most eukaryotic organisms that appears to function in mitochondrial iron homeostasis, notably the *de novo* biosynthesis of iron-sulfur (Fe-S) cluster proteins and heme biosynthesis. The exact function of FXN has not been defined but recent studies suggest that FXN functions as an allosteric activator with Fe²⁺ for Fe-S cluster biosynthesis. The absence of FXN is associated with a loss of activity in Fe-S-containing proteins, such as aconitase as well as with the disease Friedreich ataxia.

Precursor FXN protein (23.1 kDa, 210 amino acids) comprises an 80 amino acid mitochondrial targeting sequence (MTS) at its amino (N) terminus. Within mitochondria, the precursor FXN protein is processed in two steps by the mitochondrial matrix processing peptidase (MPP). It has been shown that the intermediate form of FXN is formed by cleavage at residue 42 by the MPP, and the resulting form of FXN (FXN42-210) has been shown to be cleaved at amino acid 81, yielding a mature, 130 amino acid protein, with a predicted molecular weight of 14.2 kDa.

As described above, Friedreich ataxia is an autosomal recessive degenerative disorder characterized by ataxia, areflexia, sensory loss, weakness, scoliosis, and cardiomyopathy. A deficiency of frataxin in cells leads to decreased activities of mitochondrial iron-sulfur cluster-containing enzymes, to an accumulation of iron in the mitochondrial matrix, increased sensitivity to oxidative stress, as well as to impaired adenosine triphosphate (ATP) production.

In the above and other embodiments of the presently disclosed subject matter, frataxin refers to human frataxin and any biologically active fragments and derivatives thereof, which is devoid of its natural (native) MTS sequence. Non limiting examples for mature human frataxin are given by the accession number Q16595[81-210] and as indicated in Table 1 below, where the amino acid sequence of mature human frataxin is as set forth in SEQ ID NO. 26 and the nucleic acid sequence encoding therefor is as set forth in SEQ ID NO. 6.

Notably, as shown in Example 4 below, a TAT-MTS-frataxin fusion protein was demonstrated by the inventors to enter mitochondria of human intact BJAB cells. Analysis of sub-cellular fractions of these cells, in order to separate the mitochondria from the cytosol, verified that the various frataxin fusion protein constructs (i.e. TAT-MTSlad-FRA, TAT-MTSfra-FRA, TAT-MTScs-FRA, and TAT-MTSorf-FRA) were indeed successfully delivered into the mitochondria. Surprisingly, among the fusion proteins carrying a heterologous MTS, the MTS of citrate synthase (MTScs) was shown by the inventors to be delivered into the mitochondria in the most efficient manner.

Delivery of fusion proteins comprising a mitochondrial protein into the mitochondria has far-reaching therapeutic beneficial implications for treatment of mitochondrial disorders in general, and delivery of frataxin into mitochondria has specific therapeutic benefit for treatment of Friedreich's ataxia in particular.

Fusion protein constructs comprising ornithine transcarbamoylase (OTC, also called ornithine carbamoyltransferase) are disclosed in addition to the invention. OTC is a protein having enzymatic activity that catalyzes the reaction between carbamoyl phosphate (CP) and ornithine (Orn) to form citrulline (Cit) and phosphate (Pᵢ). In mammals OTC is located in the mitochondria and is part of the urea cycle.

OTC is a trimer, and the active sites thereof are located at the interface between the protein monomers, emphasizing the importance of proper folding to the mitochondrial activity of the protein. Deficiency in OTC results in an increase in ammonia level, leading to neurological problems.

As demonstrated in the appended comparative Examples, fusion protein constructs comprising OTC and its native MTS as well as fusion protein constructs comprising OTC and a heterologous MTS were able to internalize into the mitochondria in HepG2 cells (Comparative Example 9). Interestingly, the internalization ability of the fusion protein constructs comprising an MTS that is heterologous to OTC was slightly higher than the internalization ability of the fusion protein construct comprising the native MTS of OTC. Fusion protein constructs comprising the protein OTC were also shown to be active. As shown in Figure 20, the level of cell viability in the presence of fusion protein constructs comprising OTC and citrate synthase as the MTS was similar to the level of cell viability for cells which were not exposed to ammonium chloride (which served as a model for ammonia stress conferred by defective or missing OTC).

Furthermore, the level of cell viability in the presence of a fusion protein construct comprising OTC and citrate synthase as the MTS was higher from the level of cell viability in cells treated with the fusion protein construct comprising the native MTS of OTC.

Thus, in the above and other embodiments of the invention, the functional human mitochondrial protein is specifically frataxin. Fusion protein constructs comprising the functional human mitochondrial protein ornithine transcarbamoylase (OTC) are also described herein. In some embodiments, the nucleic acid encoding the mature

OTC protein is denoted by SEQ ID NO. 15. In other embodiments the OTC mature protein of the present disclosure has the amino acid sequence denoted by SEQ ID NO. 39.

As indicated above, the presently disclosed subject matter provides a fusion protein comprising a HIV-1 transactivator of transcription (TAT) domain, a functional human mitochondrial protein and a human mitochondria targeting sequence (MTS) situated between said TAT domain and said functional mitochondrial protein and wherein said human MTS is heterologous to said functional protein.

Most of the proteins directed to the mitochondria are synthesized with a mitochondrial targeting (or translocation) sequence (MTS), which allows their import from the cytoplasm into mitochondria through the translocation machinery. Once entering the mitochondria, the MTS is recognized and cleaved off, allowing for proper processing and, if necessary, assembly into mitochondrial enzymatic complexes.

Thus, as used herein, the term "*mitochondria targeting sequence",* MTS or "*mitochondria translocation sequence"* refers to an amino acid sequence capable of causing the transport into the mitochondria of a protein, peptide, amino acid sequence, or compound attached thereto, and any biologically active fragments thereof. MTSs used in the fusion protein constructs in accordance with the presently disclosed subject matter, which are situated N-terminal to the functional mitochondrial protein, are typically from about 15 to about 40 amino acids in length, including from about 3 to about 5 nonconsecutive basic amino acid (arginine/lysine) residues, often with several serine/threonine residues but without acidic amino acid (asparate/glutamate) residues. In their molecular structure, these MTSs are able to form strong basic amphipathic α-helices that are essential for efficient mitochondrial transportation.

In other words, presently disclosed is a fusion protein as herein defined, wherein said functional human mitochondrial protein is C-terminal to said human mitochondria targeting sequence (MTS).

The term "*about*" as used herein indicates values that may deviate up to 1%, more specifically 5%, more specifically 10%, more specifically 15%, and in some cases up to 20% higher or lower than the value referred to, the deviation range including integer values, and, if applicable, non-integer values as well, constituting a continuous range.

In the above and other embodiments, the MTS is human MTS, namely MTS of a human mitochondrial protein.

In the above and other embodiments of the presently disclosed subject matter the MTS comprises from about 15 to about 40 amino acid residues, including from about 3 to about 5 nonconsecutive (i.e. which are not covalently linked one to the other in a sequential manner) basic amino acid residues, and optionally from about 1 to about 3 or 4 or 5 serine/threonine residues.

The term "*amino acid residues*" as used herein refers to naturally occurring and synthetic amino acids, as well as amino acid analogs and amino acid mimetics that can function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. "amino acid analogs and amino acid mimetics" refers to compounds that have the same fundamental chemical structure as a naturally occurring amino acid. Such analogs have modified R groups or modified peptide backbones, but retain the same basic chemical structure as a naturally occurring amino acid. Amino acids may be referred to herein by either their commonly known three letter symbols or by the one-letter symbols recommended by the IUPAC-IUB Biochemical Nomenclature Commission.

It is well known in the art that amino acid residues may be divided according to their chemical properties to various groups, *inter alia,* on the basis of similarity in polarity, charge, solubility, hydrophobicity, hydrophilicity, and/or the amphipathic nature of the residues involved.

For example, nonpolar *"hydrophobic"* amino acids are selected from the group consisting of Valine (V), Isoleucine (I), Leucine (L), Methionine (M), Phenylalanine (F), Tryptophan (W), Cysteine (C), Alanine (A), Tyrosine (Y), Histidine (H), Threonine (T), Serine (S), Proline (P), Glycine (G), Arginine (R) and Lysine (K); "*polar*" amino acids are selected from the group consisting of Arginine (R), Lysine (K), Aspartic acid (D), Glutamic acid (E), Asparagine (N), Glutamine (Q); "*positively charged*" amino acids are selected form the group consisting of Arginine (R), Lysine (K) and Histidine (H) and wherein "*acidic*" amino acids are selected from the group consisting of Aspartic acid (D), Asparagine (N), Glutamic acid (E) and Glutamine (Q). "*Basic*" amino acids are selected from the group consisting of Histidine (H), lysine (K) and Arginine (R), which are polar and positively charged at pH values below their pKa's, and are very hydrophilic.

As indicated above, the presently disclosed subject matter encompasses human mature frataxin and any biologically active fragments and derivatives thereof, which is devoid of its natural (native) MTS sequence. By the term "*biologically active fragments and derivatives*" it is meant any variations, including deletion, substitution and/or insertion of one or more amino acid residues in the amino acid sequences of mature frataxin (or in the nucleic acid encoding therefor), for example 1, 2, 3, 4, 5 or more amino acid residues, in accordance with the presently disclosed subject matter which would not alter the biological activity of frataxin.

The disclosure further relates to DNA constructs comprising the nucleic acid sequence of the presently disclosed subject matter or biologically functional fragments and derivatives thereof. The constructs of the presently disclosed subject matter may further comprise additional elements such as promoters, regulatory and control elements, translation, expression and other signals, operably linked to the nucleic acid sequence of the invention.

It is known that each mitochondrial enzyme produced in the cytoplasm and transported into the mitochondria is produced as a precursor protein, carrying its natural MTS. Thus, the precursor mitochondrial protein already has its native MTS. However, this naturally occurring sequence in the precursor protein may be exchanged with any other known MTS.

As exemplified herein, the fusion protein constructs comprising frataxin or ornithine transcarbamoylase further comprised the MTS of lipoamide dehydrogenase (MTSlad, of the amino acid sequence denoted by SEQ ID NO. 24 and encoded by the nucleic acid sequence denoted by SEQ ID NO. 5), C6ORF66 (MTSorf, of the amino acid sequence denoted by SEQ ID NO. 25 and encoded by the nucleic acid sequence denoted by SEQ ID NO. 4), or of citrate synthase (MTScs, of the amino acid sequence denoted by SEQ ID NO. 23 and encoded by the nucleic acid sequence denoted by SEQ ID NO. 3).

Alternatively, fusion protein constructs comprising frataxin or ornithine transcarbamoylase comprised respectively the native MTS of frataxin (MTSfra, of the amino acid sequence denoted by SEQ ID NO. 22 and encoded by the nucleic acid sequence denoted by SEQ ID NO. 2) or the native MTS of ornithine transcarbamoylase (MTSotc, of the amino acid sequence denoted by SEQ ID NO. 38 and encoded by the nucleic acid sequence denoted by SEQ ID NO. 37).

In addition, the inventors showed that fusion protein constructs comprising frataxin and a MTS sequence that is not the native MTS of frataxin (i.e. heterologous MTS) were superior to the frataxin fusion protein construct comprising the native MTS, based on the higher yield obtained for fusion protein constructs comprising heterologous MTS during the expression and purification stages.

Surprisingly, fusion protein constructs comprising frataxin and a heterologous MTS were also demonstrated by the inventors to have an enhanced biological activity as compared to the frataxin fusion protein construct comprising the native MTS (Example 5). In particular, a fusion protein construct comprising frataxin and citrate synthase MTS showed the highest effect in reducing toxicity of BSO (Figure 11A and Figure 11B). As shown in Example 4 below, the fusion protein construct comprising frataxin and citrate synthase MTS also showed the highest ability of being delivered into mitochondria among the exemplified constructs comprising heterologous MTS.

Consistent with the above results, a fusion protein construct comprising frataxin and another heterologous MTS, namely the MTS of lipoamide dehydrogenase (LAD), was also demonstrated by the inventors to have an enhanced biological activity as compared to the frataxin fusion protein construct comprising the frataxin native MTS, as demonstrated in Figure 12C. As evident from Figure 12, the biological activity of this fusion protein construct was comparable to the biological activity of the fusion protein construct comprising the citrate synthase MTS.

As detailed above, it is known that FXN mRNA is translated into a precursor polypeptide that is transported to the mitochondrial matrix and processed to at least two forms, namely FXN42-210 and FXN81-210, where FXN42-210 is the transient processing intermediate and FXN81-210 represents the mature protein. In other words, the transient frataxin polypeptide FXN42-210 includes a portion of the native frataxin MTS, whereas the FXN81-210 is the mature protein *per se,* devoid of its native MTS. Without wishing to be bound by theory, by using a heterologous MATS in fusion protein constructs comprising frataxin, mature frataxin is expected to be released from the fusion protein at a single step, thereby raising the biological availability of this protein in the mitochondria compared to fusion protein constructs comprising frataxin and its native MTS.

Thus, the MTS encompassed by the presently disclosed subject matter is any human MTS that is encoded by the nuclear DNA, translated (produced) in the cytoplasm and transported into the mitochondria and which is not the native N-terminal MTS sequence of the functional protein present in the fusion protein construct according to the invention. In other words, the MTS sequence is other than the native N-terminal MTS sequence of the functional protein, i.e. is heterologous thereto. The various MTS may be exchangeable for each mitochondrial enzyme among themselves. Each possibility represents a separate embodiment of the present invention.

As used herein, the term "*heterologous*" when referring to MTS fused to the functional human mitochondrial protein according to the present disclosure, is to be taken to mean MTS obtained from another (distinct) mitochondrial protein, i.e. MTS which is not the naturally occurring MTS of the said functional protein.

By way of a non-binding example, the heterologous MTS according to the present disclosure is a MTS which is heterologous to the mitochondrial protein frataxin, which may be, but is not limited to, the MTS of any human mitochondrial protein, e.g. lipoamide dehydrogenase (MTSlad, of the amino acid sequence denoted by SEQ ID NO. 24 and encoded by the nucleic acid sequence denoted by SEQ ID NO. 5), C6ORF66 (MTSorf, of the amino acid sequence denoted by SEQ ID NO. 25 and encoded by the nucleic acid sequence denoted by SEQ ID NO. 4), and of citrate synthase (MTScs, of the amino acid sequence denoted by SEQ ID NO. 23 and encoded by the nucleic acid sequence denoted by SEQ ID NO. 3), which are non-limiting examples.

Thus, in embodiments of the presently disclosed fusion protein constructs, the MTS can be any one of human mitochondrial citrate synthase MTS having the amino acid sequence denoted by SEQ ID NO. 23, the human lipoamide dehydrogenase MTS having the amino acid sequence denoted by SEQ ID NO. 24, the MTS of the human C6ORF66 gene product having the amino acid sequence denoted by SEQ ID NO. 25 and the MTS of human mitochondrial GLUD2 encoded by the nucleic acid sequence denoted by SEQ ID NO. 16.

In other embodiments of the presently disclosed fusion protein construct, the MTS is any one of human mitochondrial citrate synthase MTS having the amino acid sequence denoted by SEQ ID NO. 23 and the human lipoamide dehydrogenase MTS having the amino acid sequence denoted by SEQ ID NO. 24.

In some embodiments disclosed is a fusion protein comprising a HIV-1 transactivator of transcription (TAT) domain fused to human frataxin and a human mitochondria targeting sequence (MTS) of a human mitochondrial protein selected from lipoamide dehydrogenase (LAD) and citrate synthase (CS) situated between said TAT domain and said frataxin, wherein said frataxin is C-terminal to said MTS of human lipoamide dehydrogenase or human citrate synthase.

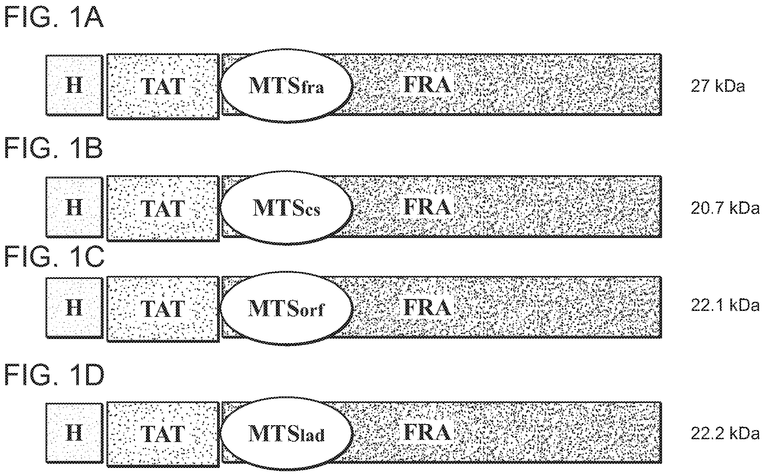

As indicated above, the fusion protein according to the presently disclosed subject matter comprises a HIV-1 transactivator of transcription (TAT) domain, located at the N-terminus of the fusion protein, N-terminal to the MTS as defined above, which in turn is situated N-terminal to the functional human mitochondrial protein (see Figure 1 for a schematic presentation).

As used herein, the term *HIV-1 transactivator of transcription (TAT)* domain refers to a protein transduction domain which is an 11-amino-acid (residues 47-57) arginine- and lysine-rich portion of the HIV-I Tat protein having the amino acid sequence YGRKKRRQRRR as set forth in SEQ ID NO. 21. TAT-fusion protein constructs are known in the art to be introduced into cultured cells, intact tissue, and live tissues and cross the blood-brain barrier (BBB). TAT fusion proteins are also known to traverse mitochondrial membranes [13].

The presently disclosed subject matter also encompasses any fragments of the above defined TAT domain. For example, a TAT domain according to the presently disclosed subject matter may comprise from about 3 to about 11 (e.g. 4-11, 5-11 , 6-11, 7-11, 8-11, 9, 10 or 11) sequential amino acid residues of the HIV-I Tat protein having the amino acid sequence YGRKKRRQRRR (SEQ ID NO. 21).

In some embodiments, the fragment of the above defined TAT domain comprise 9 sequential amino acid residues of the HIV-I Tat protein, having the amino acid sequence of RKKRRQRRR, as set forth in SEQ ID NO. 27 and encoded by the nucleic acid sequence denoted by SEQ ID NO. 1, which was used in the preparation of the fusion protein constructs exemplified below.

Thus, in this and other embodiments of the presently disclosed subject matter, the fusion protein comprises a TAT domain at its N-terminus and a functional mitochondrial protein at its C-terminus, both covalently linked (fused) to an MTS that is situated between said TAT domain and said functional mitochondrial protein. In other words, the disclosure provides a protein construct comprising an N-terminal TAT fused to N-terminal of MTS fused to N-terminal of functional protein, as schematically presented in Figure 1 for frataxin.

The fusion protein according to the presently disclosed subject matter may be prepared by any method known to a skilled artisan. By example, the fusion protein as herein defined may be prepared as exemplified below, by standard molecular biology and cloning techniques.

The term "*fusion protein*" in the context of the invention concerns a sequence of amino acids, predominantly (but not necessarily) connected to each other by peptidic bonds. The term "fused" in accordance with the fusion protein of the invention refers to the fact that the amino acid sequences of at least three different origins, namely, the TAT domain, the sequence of the heterologous mitochondrial targeting domain (MTS) and the functional mitochondrial protein, are linked to each other by covalent bonds either directly or via an amino acid linker joining (bridging, conjugating, covalently binding) the amino acid sequences. The fusion may be by chemical conjugation such as by using state of the art methodologies used for conjugating peptides.

The fusion protein in the context of the invention may also optionally comprise at least one linker covalently joining different domains of the fusion protein construct.

The term "*linker*" in the context of the invention concerns an amino acid sequence of from about 4 to about 20 amino acid residues positioned between the different fusion protein domains and covalently joining them together. For example, a linker in accordance with the invention may be 4, 5, 6, 7, 8, 9, 10, 11, 12, 13, 14, 15, 16, 17, 18, 19 or 20 amino acid long. Linkers are often composed of flexible amino acid residues, for example but not limited to glycine and serine so that the adjacent protein domains are free to move relative to one another. The term "linker" can be interchageably used with "spacer".

The design of a linker that enables proper folding of the various domains of a protein is well known in the art. A non-binding example of a linker is the amino acid sequence GSDP (Gly-Ser-Asp-Pro) as denoted by SEQ ID NO. 32, which was used in the Examples below to construct the fusion proteins His TAT MTS(cs) 81-210 FRA (denoted by SEQ ID NO. 17), His TAT MTS(fra) 81-210 FRA (denoted by SEQ ID NO. 18), His TAT MTS(lad) 81-210 FRA (denoted by SEQ ID NO. 19) and His TAT MTS(orf) 81-210 FRA (denoted by SEQ ID NO. 20) as well as several of the fusion proteins comprising OTC.

Thus in some embodiments the present disclosure relates to a fusion protein as herein defined further comprising a linker covalently linking said TAT domain to said MTS sequence.

The fusion protein in the context of the invention may also optionally comprise at least one methionine (M) residue at its N-terminus, as in the case of the exemplified fusion proteins below. The methionine is positioned N-terminal to the TAT domain.

Fusion may also be achieved by recombinant techniques, i.e. by construction of a nucleic acid sequence coding for the entire the fusion protein (coding for all segments) so that essentially all the bonds are peptidic bonds.

In order to facilitate purification of the protein constructs described herein, fusion protein constructs in accordance with the invention may also comprise an N-terminal tag (e.g. His tag as exemplified below, Glutathione S-transferase (GST), Maltose-Binding Protein (MBP), FLAG octapeptide, to name but few), which may be removed or retained in the final fusion construct. Such tags are normally cleaved off from the fusion protein upon entry to the mitochondria, along with the TAT and MTS sequences.

In some embodiments, the amino acid sequence of a fusion protein according to the invention is as set forth in SEQ ID NO. 17, namely His TAT MTS(cs) FRA, SEQ ID NO. 19, namely His TAT MTS(lad) FRA, SEQ ID NO. 20, namely, His TAT MTS(orf) FRA, SEQ ID NO. 45, namely, His TAT MTS(cs) OTC, SEQ ID NO. 46, namely, His TAT MTS(orf) OTC as well as SEQ ID NO. 47, namely, His TAT MTS(lad) OTC.

Fusion protein constructs in accordance with the invention may also be prepared without an N-terminal tag. In some embodiments, the amino acid sequence of a fusion protein according to the invention is as set forth in SEQ ID NO. 28, namely TAT MTS(cs) FRA, SEQ ID NO. 30, namely TAT MTS(lad) FRA, SEQ ID NO. 31, namely, TAT MTS(orf) FRA, SEQ ID NO. 49, namely TAT MTS(cs) OTC, SEQ ID NO. 50, namely TAT MTS(orf) OTC, as well as in SEQ ID NO. 51, namely TAT MTS(lad) OTC.

Therefore the present disclosure further encompasses a fusion protein having the amino acid sequence denoted by SEQ ID NO. 30, comprising a HIV-1 transactivator of transcription (TAT) domain having the amino acid sequence denoted by SEQ ID NO. 27 fused to human frataxin having the amino acid sequence denoted by SEQ ID NO. 26 and a mitochondria targeting sequence (MTS) of human lipoamide dehydrogenase having the amino acid sequence denoted by SEQ ID NO. 24, said MTS situated between said TAT domain and said frataxin and is linked to said TAT domain via a linker having the amino acid sequence denoted by SEQ ID NO. 32, and wherein said frataxin is C-terminal to said MTS of human lipoamide dehydrogenase.

In some embodiments the fusion protein as herein defined has the amino acid sequence denoted by SEQ ID NO. 28, comprising a HIV-1 transactivator of transcription (TAT) domain having the amino acid sequence denoted by SEQ ID NO. 27 fused to human frataxin having the amino acid sequence denoted by SEQ ID NO. 26 and a mitochondria targeting sequence (MTS) of human citrate synthase having the amino acid sequence denoted by SEQ ID NO. 23, said MTS situated between said TAT domain and said frataxin, and is linked to said TAT domain via a linker having the amino acid sequence denoted by SEQ ID NO. 32, and wherein said frataxin is C-terminal to said MTS of human citrate synthase.

The presently disclosed subject matter further provides a composition comprising a physiologically acceptable carrier and as an active ingredient a fusion protein as herein defined.

In specific embodiments the presently disclosed subject matter provides a composition comprising as an active ingredient a fusion protein having the amino acid sequence denoted by SEQ ID NO. 30, or having the amino acid sequence denoted by SEQ ID NO. 28 and a physiologically acceptable carrier.

Also provided by the presently disclosed subject matter is a pharmaceutical composition comprising a pharmaceutically acceptable carrier and as an active ingredient a fusion protein construct as herein defined.

The "*composition*" as herein defined generally comprises a buffering agent, an agent which adjusts the osmolarity thereof, and optionally, one or more pharmaceutically (or physiologically) acceptable carriers, diluents, additives and excipients as known in the art. Supplementary active ingredients can also be incorporated into the compositions. The pharmaceutically acceptable carrier can be solvent or dispersion medium containing, for example, water, ethanol, polyol (for example, glycerol, propylene glycol, and liquid polyethylene glycol, and the like), suitable mixtures thereof, and vegetable oils. Each carrier should be physiologically or pharmaceutically acceptable, as the case may be, in the sense of being compatible with the other ingredients and not injurious to the patient.

The additives may be but are not limited to at least one of a protease inhibitor, for example phenylmethanesulfonylfluoride or phenylmethylsulfonyl fluoride (PMSF), Nafamostat Mesylate, 4-(2-Aminoethyl)benzenesulfonyl fluoride hydrochloride (AEBSF), Bestatin, Pepstatin A, E-64, Leupeptin, 1, 10-Phenanthroline and any other protease inhibitor known in the art.

The "*pharmaceutical compositions*" of the presently disclosed subject matter are compositions as described above, comprising pharmaceutically acceptable carriers, diluent, adjuvant and/or excipients and/or additives as known in the art.

The presently disclosed subject matter further provides a pharmaceutical composition as herein defined for treating or alleviating a mitochondrial disorder.

The term "*mitochondrial disorder*" as encompassed by the presently disclosed subject matter refers to a group of systemic diseases caused by inherited or acquired damage to the mitochondria causing an energy shortage within those areas of the body that consume large amounts of energy such as the liver, muscles, brain, and the heart. The result is often liver failure, muscle weakness, fatigue, and problems with the heart, eyes, and various other systems.

The mitochondrial disorder may be any one of frataxin deficiency which causes or is associated with Friedreich's ataxia; a deficiency in OTC (X-linked recessive genetic disorder caused by non-conservative mutations in the OTC gene); disorder associated with LAD deficiency; or the mitochondrial metabolic disorder is Complex I deficiency (OMIM:252010). Complex I deficiency can be caused by a mutation in any of the subunits thereof. Alternatively, the Complex I deficiency is caused by a mutation in a gene selected from NDUFV1 (OMIM: 161015), NDUFV2 (OMIM:600532), NDUFS1 (OMIM: 157655), NDUFS2 (OMIM:602985), NDUFS3 (OMIM:603846), NDUFS4 (OMIM:602694), NDUFS6 (OMIM:603848), NDUFS7 (OMIM:601825), NDUFS8 (OMIM:602141), and NDUF A2 (OMIM: 602137).

In other embodiments, the mitochondrial disorder is Complex IV deficiency (Cytochrome C oxidase; OMIM:220110). Complex IV deficiency can be caused by a mutation in any of the subunits thereof. In another embodiment, the Complex IV deficiency is caused by a mutation in a gene selected from the group consisting of MTCO1 (OMIM:516030), MTCO2 (OMIM:516040), MTCO3 (OMIM:516050), COXlO (OMIM:602125), COX6B1 (OMIM: 124089), SCO1 (OMIM:603644), FASTKD2 (OMIM:612322), and SC02 (OMIM:604272).

In other embodiments, the *mitochondrial* disorder is a neurodegenerative disease. As provided herein, compositions of the present invention exhibit the ability to traverse the blood-brain barrier (BBB).

In further embodiments of the presently disclosed subject matter, the mitochondrial disorder is selected from the group consisting of encephalopathy and liver failure that is accompanied by stormy lactic acidosis, hyperammonemia and coagulopathy. In other embodiments, the mitochondrial disorder is selected from the group consisting of Ornithine Transcarbamoylase deficiency (hyperammonemia) (OTCD), Carnitine O-palmitoyltransferase II deficiency (CPT2), Fumarase deficiency, Cytochrome c oxidase deficiency associated with Leigh syndrome, Maple Syrup Urine Disease (MSUD), Medium-Chain Acyl-CoA Dehydrogenase deficiency (MCAD), Acyl-CoA Dehydrogenase Very Long-Chain deficiency (LCAD), Trifunctional Protein deficiency, Progressive External Ophthalmoplegia with Mitochondrial DNA Deletions (POLG), DGUOK, TK2, Pyruvate Decarboxylase deficiency, and Leigh Syndrome (LS). In another embodiment, the mitochondrial metabolic disorder is selected from the group consisting of Alpers Disease; Barth syndrome; beta -oxidation defects; carnitine- acyl-carnitine deficiency; carnitine deficiency; co-enzyme QlO deficiency; Complex II deficiency (OMIM:252011), Complex III deficiency (OMIM: 124000), Complex V deficiency (OMIM:604273), LHON-Leber Hereditary Optic Neuropathy; MM-Mitochondrial Myopathy; LIMM-Lethal Infantile Mitochondrial Myopathy; MMC-Maternal Myopathy and Cardiomyopathy; NARP-Neurogenic muscle weakness, Ataxia, and Retinitis Pigmentosa; Leigh Disease; FICP-Fatal Infantile Cardiomyopathy Plus, a MELAS-associated cardiomyopathy; MELAS-Mitochondrial Encephalomyopathy with Lactic Acidosis and Strokelike episodes; LDYT-Leber's hereditary optic neuropathy and Dystonia; MERRF-Myoclonic Epilepsy and Ragged Red Muscle Fibers; MHCM-Maternally inherited Hypertrophic CardioMyopathy; CPEO-Chronic Progressive External Ophthalmoplegia; KSS-Kearns Sayre Syndrome; DM-Diabetes Mellitus; DMDF Diabetes Mellitus + Deafness; CIPO-Chronic Intestinal Pseudoobstruction with myopathy and Ophthalmoplegia; DEAF-Maternally inherited DEAFness or aminoglycoside-induced DEAFness; PEM-Progressive encephalopathy; SNHL-SensoriNeural Hearing Loss; Encephalomyopathy; Mitochondrial cytopathy; Dilated Cardiomyopathy; GER-Gastro intestinal Reflux; DEMCHO-Dementia and Chorea; AMDF-Ataxia, Myoclonus; Exercise Intolerance; ESOC Epilepsy, Strokes, Optic atrophy, and Cognitive decline; FBSN Familial Bilateral Striatal Necrosis; FSGS Focal Segmental Glomerulosclerosis; LIMM Lethal Infantile Mitochondrial Myopathy; MDM Myopathy and Diabetes Mellitus; MEPR Myoclonic Epilepsy and Psychomotor Regression; MERME MERRF/MELAS overlap disease; MHCM Maternally Inherited Hypertrophic CardioMyopathy; MICM Maternally Inherited Cardiomyopathy; MILS Maternally Inherited Leigh Syndrome; Mitochondrial Encephalocardio myopathy; Multisystem Mitochondrial Disorder (myopathy, encephalopathy, blindness, hearing loss, peripheral neuropathy); NAION Nonarteritic Anterior Ischemic Optic Neuropathy; NIDDM Non-Insulin Dependent Diabetes Mellitus; PEM Progressive Encephalopathy; PME Progressive Myoclonus Epilepsy; RTT Rett Syndrome; SIDS Sudden Infant Death Syndrome; MIDD Maternally Inherited Diabetes and Deafness; and MODY Maturity-Onset Diabetes of the Young, and MNGIE.

In the above and other embodiments, the presently disclosed subject matter provides a pharmaceutical composition as herein defined for use in the treatment of Friedreich's ataxia. Other disorders associated with deficiency of frataxin or defective frataxin are described herein.

In still further embodiments, the presently disclosed subject matter provides a pharmaceutical composition for use in the treatment of Friedreich's Ataxia by intravenous administration to a subject in need thereof, said composition comprising a therapeutically effective amount of a fusion protein as herein defined.

In specific embodiments the presently disclosed subject matter provides a pharmaceutical composition for use in the treatment of Friedreich's Ataxia by intravenous administration to a subject in need thereof, said composition comprising a therapeutically effective amount of a fusion protein having the amino acid sequence denoted by SEQ ID NO. 30 or having the amino acid sequence denoted by SEQ ID NO. 28.

In further specific embodiments the presently disclosed subject matter provides a pharmaceutical composition for use in the treatment of Friedreich's Ataxia by intravenous administration to a subject in need thereof, said composition comprising a therapeutically effective amount of a fusion protein having the amino acid sequence denoted by SEQ ID NO. 30.

In still further specific embodiments the presently disclosed subject matter provides a pharmaceutical composition for use in the treatment of Friedreich's Ataxia by intravenous administration to a subject in need thereof, said composition comprising a therapeutically effective amount of a fusion protein having the amino acid sequence denoted by SEQ ID NO. 28.

The presently disclosed subject matter further provides a fusion protein according to the invention for use in a method for the treatment of a mitochondrial disorder.

In the above and other embodiments of the presently disclosed subject matter the functional human mitochondrial protein is frataxin for use in the treatment or alleviation of Friedreich's ataxia or any other disorder associated with deficiency of frataxin or defective frataxin.

As shown below in Example 7, the inventors have shown that fusion proteins comprising TAT, MTS and frataxin (TAT-MTS-FRA) were able to partially rescue cells obtained from Friedreich's ataxia patients, as well as normal cells, from oxidative stress, exhibiting a clear biological activity of the fusion protein constructs of the presently disclosed subject matter.

L-Buthionine sulphoximine (BSO) is an inhibitor of gamma-glutamylcysteine synthetase (gamma-GCS) and, consequently lowers tissue glutathione (GSH) concentrations. GSH plays an important role in cellular defense against a wide variety of toxic electrophiles via the formation of thioether conjugates. Therefore, BSO was used by the inventors to model oxidative stress, through its ability to inhibit *de novo* glutathione synthesis, thereby depleting an important component of these cells' intrinsic defenses against reactive oxygen species (ROS) and allowing for the accumulation of ROS produced by natural cell processes, known to result in cell death.

It is known that Friedreich ataxia cells are extremely sensitive to BSO-induced oxidative stress compared with normal cells because they lack Frataxin, and thus are used as an *in vitro* model of the long-term consequences of absent Frataxin.

As shown in the Examples below, oxidative stress was induced with various concentrations of BSO in cells obtained from patients as well as in normal healthy cells and the effect of the various TAT-MTS-FRA fusion proteins on cell death was measured. As can be seen in Figure 11, BSO caused cell death of normal lymphocytes as well as of cells obtained from Friedreich ataxia patients. However, cells obtained from patients were more sensitive to BSO-induced oxidative stress, consistent with previous findings. Most importantly, the various TAT-MTS-FRA fusion proteins, which were added a few hours before oxidative stress induction, were demonstrated to partially rescue both normal lymphocytes as well as patients' cells from cell death. This partial rescue was determined by both reduction in cell death and by reduction in caspase 3 activity.

Surprisingly, as also shown in Figure 11, at least two out of the three fusion proteins carrying a heterologous MTS (namely, MTSorf and MTScs) demonstrated a superior protective effect with respect to the effect demonstrated by the fusion protein carrying the native MTS.

In addition, in an independent comparative experiment shown in Figure 12, in which the effect of the various TAT-MTS-FRA fusion proteins on oxidative stress was examined, a fusion protein carrying another heterologous MTS, namely, MTSlad also demonstrated a superior protective effect with respect to the effect demonstrated by the fusion protein carrying the native MTS (Figure 12C).

The term "*treat*" or "*treatment*" or forms thereof as herein defined means to prevent worsening or arrest or alleviate or cure the disease or condition in a subject in need thereof. Thus the term *"treatment", "treating"* or *"alleviating"* in the context of the intention does not refers to complete curing of the diseases, as it does not change the mutated genetics causing the disease. This term refers to alleviating at least one of the undesired symptoms associated with the disease, improving the quality of life of the subject, decreasing disease-caused mortality, or (if the treatment in administered early enough) preventing the full manifestation of the mitochondrial disorder before it occurs, mainly to organs and tissues that have a high energy demand. The treatment may be a continuous prolonged treatment for a chronic disease or a single, several or multiple administrations for the treatment of an acute condition such as encephalopathy and liver failure that is accompanied by stormy lactic acidosis, hyperammonemia and coagulopathy.

Notably, in the case of metabolic or mitochondrial disorders there is no need to restore protein activity back to 100%, but rather raise it above a certain energetic threshold which can vary from patient to patient depending on basal protein activity.

In addition, treatment of mitochondrial disorders using replacement therapy is necessarily more complex than replacement of a cytosolic gene product and must consider not only the need to target and cross multiple membranes in mitochondria, but also the fact that many proteins in the mitochondria act as multi-component complexes which require appropriate assembly in order to integrate properly. Additionally, many of the mitochondrial gene defects cause severe neurologic symptoms as the primary or most prominent phenotype, and, as mentioned above, drug delivery across the BBB is difficult.

Therapeutic formulations may be administered in any conventional route and dosage formulation. Formulations typically comprise at least one active ingredient, as defined above, together with one or more acceptable carriers thereof. Thus, administration can be any one of intravenous, intraperitoneal, intramuscular and intratechal administration. Oral administration is also contemplated.

The term *"therapeutically effective amount"* (or amounts) of the peptide for purposes herein defined is determined by such considerations as are known in the art in order to cure or at least arrest or at least alleviate the medical condition.

As indicated above, in the case of metabolic or mitochondrial disorders it is not required to restore protein activity back to 100%. Rather, raising the protein activity to above a certain energetic threshold, which can vary from patient to patient depending on basal protein activity, can be sufficient to provide a therapeutic effect.

In some embodiments the therapeutically effective amount may be determined for each patient individually, based on the patient's *basal protein activity.* The patient's basal protein activity, or the level of protein activity may in turn be determined using any method known in the art, for example, by subjecting a biological sample obtained from a patient (e.g. blood (white cells), skin fibroblast cells, whole tissue biopsies) to an *in vitro* enzymatic assay or immunohistochemistry or any specific imaging such as PET.

In some embodiments the therapeutically effective amount according to the presently disclosed subject matter is between about 0.5 mg/kg to about 2.0 mg/kg body weight of the subject in need thereof which is the Human Equivalent Dose (HED) of an effective amount in mice of between about 7 mg/kg to about 25 mg/kg.

Thus in specific embodiments disclosed is a pharmaceutical composition as herein defined wherein the therapeutically effective amount administered is from about 0.5 mg/Kg to about 2 mg/Kg body weight of said subject.

As used herein, the term *"subject in need"* is to be taken to mean a human suffering from a mitochondrial disorder as herein defined.

In the above and other embodiments, the pharmaceutical composition for use in treating or alleviating a mitochondrial disorder according to the invention further comprises administering an additional therapeutic agent.

The term *"additional therapeutic agent"* as herein defined refers to any agent that is administered in addition to the fusion protein according to the invention in order to alleviate the symptoms associated with the disease or disorder the treatment of which is desirable. In the above and other embodiments of the disclosed subject matter, the fusion protein of the invention and said additional therapeutic agent are administered simultaneously. Alternatively or additionally, said fusion protein and said additional therapeutic agent are administered at different time points, at different intervals between administrations, for different durations of time, or in a different order.

The disclosed subject matter further provides a method for introducing a functional mitochondrial protein into mitochondria of a subject, said method comprising the step of administering to a subject in need of such treatment a therapeutically effective amount of the fusion protein as defined herein, thereby introducing a functional mitochondrial protein into the mitochondria of a subject in need thereof.

In some embodiments the functional human mitochondrial protein thereby introduced restores at least partial activity of the wild type human mitochondrial protein, for example at least 5%, at least 10%, at least 15%, at least 20%, at least 30%, at least 40%, at least 50%, at least 60%, at least 70%, at least 80%, at least 90% or up to 100% of the activity of a wild type human mitochondrial protein.

Thus, the disclosed subject matter further provides a method for restoring at least in part activity of a defective or deficient or unfunctional human mitochondrial protein in a subject in need, by administering a fusion as herein disclosed to said subject. The human mitochondrial protein may be active *per se,* or may be a member of a mitochondrial protein complex.

By another one of its aspects, the disclosed subject matter further provides a method for alleviating oxidative stress in a subject in need thereof, said method comprising the step of administering to a subject in need of such treatment a therapeutically effective amount of the fusion protein as herein defined, thereby alleviating oxidative stress in said subject.

The term *"oxidative stress*" as herein defined refers to an imbalance between the systemic manifestations of reactive oxygen species (ROS) and an ability of a biological system to readily detoxify the reactive intermediates or to repair the resulting damage. Disturbances in the normal redox state of cells can cause toxic effects through the production of peroxides and free radicals that damage all components of the cell. In addition, since some reactive oxidative species act as cellular messengers in redox signaling, accumulation of ROS can cause disruptions in normal mechanisms of cellular signaling. In humans, oxidative stress is thought to be involved in the development of various disorders and conditions, among which are cancer, Parkinson's disease, Alzheimer disease to name but a few.

It is appreciated that certain features of the presently disclosed subject matter, which are, for clarity, described in the context of separate embodiments, may also be provided in combination in a single embodiment. Conversely, various features of the invention, which are, for brevity, described in the context of a single embodiment, may also be provided separately or in any suitable sub combination or as suitable in any other described embodiment of the invention. Certain features described in the context of various embodiments are not to be considered essential features of those embodiments, unless the embodiment is inoperative without those elements.

Disclosed and described, it is to be understood that this invention is not limited to the particular examples, process steps, and materials disclosed herein as such process steps and materials may vary somewhat. It is also to be understood that the terminology used herein is used for the purpose of describing particular embodiments only and not intended to be limiting since the scope of the present invention will be limited only by the appended claims and equivalents thereof.

It must be noted that, as used in this specification and the appended claims, the singular forms "a", "an" and "the" include plural referents unless the content clearly dictates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

Unless defined otherwise, all technical and scientific terms used herein have the same meaning as commonly understood by one of ordinary skill in the art to which this invention is related. The following terms are defined for purposes of the invention as described herein.

The following Examples are representative of techniques employed by the inventors in carrying out aspects of the present invention.

### EXAMPLES

Standard molecular biology protocols known in the art not specifically described herein are generally followed essentially as in Sambrook & Russell, 2001.

### Experimental procedures

### Cell cultures

Lymphocytes (Lym 43) and fibroblasts (Fib. 78 and Fib. 65, F816) from Friedreich's ataxia patients were obtained from Coriell Cell Repositories (Camden, NJ) and grown at the recommended medium (fibroblasts in Eagle's Minimum Essential Medium (EMEM) with Earle's salts and non-essential amino acids, and lymphocytes in Roswell Park Memorial Institute (RPMI) Medium 1640 with 2m M L-glutamine, all supplemented with 15% fetal bovine serum not inactivated). Human BJAB cells (EBV-negative Burkitt's lymphoma cells; provided by Hanna Ben-Bassat, Hadassah Medical Center, Jerusalem, Israel) were grown in RPMI medium supplemented with 20% heat-inactivated fetal calf serum (FCS), 2 mM L-glutamine, 100 U/mL penicillin, and 100 µg/mL streptomycin. Normal lymphocytes [20] were grown in RPMI 1640 supplemented with 10% fetal calf serum (FCS) and antibiotics as above. HepG2 cells (ATCC: HB-8065) were grown in EMEM supplemented with 10% FCS, 2mM L-Glutamine, non-essential amino acids, antibiotics and sodium pyruvate. All culture cells were grown in 37°C with 5% CO₂. All media and supplements were purchased from Biological Industries (Bet Ha'emek, Israel). The cells were kept in a humidified atmosphere with 5% CO₂ at 37°C. All cultures were tested for mycoplasma contamination and were found to be negative.

### Cloning of the plasmids encoding the fusion proteins

### His-TAT-MTSfra-FRA

The plasmid His-TAT-LAD was prepared as previously reported [23] and was cut with BamHI and XhoII to remove the LAD sequence, thus obtaining a vector fragment encoding the His-TAT domain. The full-length frataxin was generated by PCR using a frataxin clone (purchased from Open Biosystem, Ltd., clone no. 4842134) as a template and a pair primers covering the whole sequence, including its native MTS, 5'-CGCGGATCCGTGGACTCTCGGGCGCCG-3' (forward) and 5'-ACGCTCGAGTCAAGCATCTTTTCCGGAATAGGC-3' (reverse), as denoted by SEQ ID NO. 7 and SEQ ID NO. 8, respectively. The PCR fragment was cut with BamHI and XhoII and ligated with the vector fragment, thus obtaining the plasmid encoding the His-TAT-MTSfra-FRA fusion protein.

### His-MTSlad-FRA

The plasmid encoding the His-TAT-MTSfra-FRA was cut with BamHI and BsaI to remove the MTSfra sequence. The MTSlad (the native MTS of lipoamide dehydrogenase) was obtained by PCR using the plasmid His-TAT-LAD [23] as a template and the following pair of primers: 5'-CGCGGATCCACAGAGCTGGAGTCGTGTGTA-3' (forward) and 5'-CATAGG-TGGTCTCATCTAGAGAGCCTGGGTGGCCCAAAGTTCCAGATGCGTAAGTTCT CAGAGGCA-3' (reverse) as denoted by SEQ ID NO. 9 and SEQ ID NO. 10, respectively. The PCR product was cut with BamHI and BsaI and ligated to the vector fragment thus obtaining the plasmid encoding the His-TAT-MTSlad-FRA fusion protein.

### His-TAT-MTSorf-FRA

The plasmid encoding the His-TAT-MTSfra-FRA was cut with BamHI and BsaI to remove the MTSfra sequence as described above. The MTSorf (the native MTS of the protein C6ORF66 assembly factor) was obtained by PCR using the plasmid His-TAT-ORF as a template and the following pair of primers:
5'-CGCGGATCCGGGAGCACTAGTGATTCGC-3' (forward) and 5'-CATAGGTG GTCTCATCTAGAGAGCCTGGGTGGCCCAAAGTTCCAGAAGAAGAGGGGTCTG GGAGCGA-3' (reverse), as denoted by SEQ ID NO. 11 and SEQ ID NO. 12, respectively. The PCR product was cut with BamHI and BsaI and ligated to the vector fragment thus obtaining the plasmid encoding the His-TAT-MTSorf-FRA fusion protein.

### His-TAT-MTScs-FRA

The plasmid encoding the His-TAT-MTSfra-FRA was cut with BamHI and BsaI to remove the MTSfra sequence as described above. The MTScs (the native MTS of the protein citrate synthase) was generated by synthesizing two oligonucleotides covering the MTScs sequence and the BamHI and BsaI sites at the ends: 5'-GATCCGGCTTTACTTACTGCGGCCGCCCGGCTCTTGGGAACCAAGAATGCAT CTTGTCTTGTTCTTGCAGCCCGGCATGCCAGTTCTGGAACTTTGGGCCACCC AGGCTCTC-3' (forward) and 5'-TCTAGAGAGCCTGGGTGGCCCAAAG'TTCCAG AACTGGCATGCCGGGCTGCAAGAACAAGACAAGATGCATTCTTGGTTCCCA AGAGCCGGGCGGCCGCAGTAAGTAAAGCCG-3' (reverse), as denoted by SEQ ID NO. 13 and SEQ ID NO. 14, respectively. The oligonucleotides were ligated to the vector fragment thus obtaining the plasmid encoding the His-TAT-MTScs-FRA fusion protein. In some of the assays described below the His-TAT-MTScs-FRA fusion protein construct used was obtained from Genscript (Lot #342511S03/P10011312; 2.5 mg/ml stock in 50 mM Tris-HCl, 300 mM NaCl, 10% Glycerol, pH 8.0).

All plasmids were confirmed by restriction enzymes and sequencing analyses.

**Table 1 Nucleic acid and Amino acid sequences**

| SEQ ID NO. | Sequence | Name |
|---|---|---|
| 1 | aggaagaagcggagacagcgacgaaga | nt seq. encoding TAT fragment |
| 2 | | nt seq. of MTSfra |
| 3 | | nt seq. of MTScs |
| 4 | | nt seq. of MTSorf |
| 5 | | nt seq. of MTSlad |
| 6 | | nt seq. of Mature Frataxin (FRA) |
| 7 | CGCGGATCCGTGGACTCTCGGGCGCCG | forward primer for precursor frataxin cloning |
| 8 | ACGCTCGAGTCAAGCATCTTTTCCGGAATAGGC | reverse primer for precursor frataxin cloning |
| 9 | CGCGGATCCACAGAGCTGGAGTCGTGTGTA | forward primer for MATS lad cloning |
| 10 | | reverse primer for MTS lad cloning |
| 11 | CGCGGATCCGGGAGCACTAGTGATTCGC | forward primer for MATS orf cloning |
| 12 | | reverse primer for MATS orf cloning |
| 13 | | forward oligo for MTS cs cloning |
| 14 | | reverse primer for MATS cs cloning |
| 15 | | nt seq. of Mature OTC |
| | | |
| 16 | | nt seq. of MTS of human mitochon. GLUD2 |
| 17 | | aa seq. of His TAT MTS(cs) 81-210 FRA |
| 18 | | aa seq. of HTFrataxi n [His TAT MTS(fra) 81-210 FRA] |
| 19 | | aa seq. of His TAT MTS(lad) 81-210 FRA |
| 20 | | aa seq. of His TAT MTS(orf) 81-210 FRA |
| 21 | YGRKKRRQRRR | aa seq. of TAT |
| 22 | | aa seq. of MTS (fra) |
| 23 | ALLTAAARLLGTKNASCLVLAARHAS | aa seq. of MTS (cs) |
| 24 | QSWSRVYCSLAKRGHFNRISHGLQGLSAVPLRTYA | aa seq. of MTS (lad) |
| 25 | GALVIRGIRNFNLENRAEREISKMKPSVAPRHPS | aa seq. of MTS (orf) |
| 26 | | aa seq. of Mature |
| | | frataxin |
| 27 | RKKRRQRRR | aa seq. of TAT fragment |
| 28 | | aa seq. of TAT MTS(cs) 81-210 FRA |
| 29 | | aa seq. of TAT MTS(fra) 81-210 FRA |
| 30 | | aa seq. of TAT MTS(lad) 81-210 FRA |
| 31 | | aa seq. of TAT MTS(orf) 81-210 FRA |
| 32 | GSDP | aa seq. of linker |
| 33 | | aa seq. of TAT MTS(cs) 81-210 |
| | | FRA Δ linker |
| 34 | | aa seq. of TAT MTS(fra) 81-210 FRA Δ linker |
| 35 | | aa seq. of TAT MTS(lad) 81-210 FRA Δ linker |
| 36 | | aa seq. of TAT MTS(orf) 81-210 FRA Δ linker |

Table 1 above summarizes the nucleic acid sequences (nt) of the primers used as described above (i.e. the primers denoted by SEQ ID NO. 7 - SEQ ID NO. 14), the amino acid sequence (aa) of the TAT domain (denoted by SEQ ID NO. 21), the amino acid sequence of a fragment of the TAT domain used in the present disclosure (denoted by SEQ ID NO. 27) and the nucleic acid sequence encoding therefor (denoted by SEQ ID NO. 1), the amino acid sequences of the various MTSs, namely MTS fra, MTS cs, MTS orf and MTS lad (denoted by SEQ ID NO. 22, SEQ ID NO. 23, SEQ ID NO. 25 and SEQ ID NO. 24, respectively) and the nucleic acid sequences encoding therefor (denoted by SEQ ID NO. 2, SEQ ID NO. 3, SEQ ID NO. 4 and SEQ ID NO. 5, respectively), the amino acid sequence of mature frataxin (denoted by SEQ ID NO. 26) and the nucleic acid sequence encoding therefor (denoted by SEQ ID NO. 6), as well as the nucleic acid sequences encoding the OTC protein and the MTS of human mitochondrial GLUD2 (denoted by SEQ ID NO. 15 and SEQ ID NO. 16, respectively). A four-amino acid long linker, having the amino acid sequence of GSDP is also listed in Table 1 above and is denoted by SEQ ID NO. 32.

In addition Table 1 above indicates the amino acid sequences of the various His TAT MTS 81-210 FRA constructs, which comprise the mature frataxin, namely, His TAT MTS(cs) 81-210 FRA, His TAT MTS(fra) 81-210 FRA (also denoted herein as "HTFrataxin"), His TAT MTS(lad) 81-210 FRA and His TAT MTS(orf) 81-210 FRA (denoted by SEQ ID NO. 17, SEQ ID NO. 18, SEQ ID NO. 19 and SEQ ID NO. 20, respectively). The various His TAT MTS 81-210 FRA constructs denoted by SEQ ID NO. 17, SEQ ID NO. 18, SEQ ID NO. 19 and SEQ ID NO. 20 all comprise a short peptide linker denoted by SEQ ID NO. 32, which joins the TAT and MTS domains.

Table 1 above further indicates the amino acid sequences of the various fusion constructs constructed without a His tag at their N-termini and with a short peptide linker denoted by SEQ ID NO. 32 (which joins the TAT and MTS domains). These constructs comprise TAT, a linker, MTS and mature frataxin (81-210 FRA), namely TAT MTS(cs) 81-210 FRA, TAT MTS(fra) 81-210 FRA, TAT MTS(lad) 81-210 FRA and TAT MTS(orf) 81-210 FRA, denoted by SEQ ID NO. 28, SEQ ID NO. 29, SEQ ID NO. 30 and SEQ ID NO. 31, respectively.

In addition Table 1 above indicates the amino acid sequences of the various fusion constructs constructed without a His tag and without a linker. These fusion constructs comprise TAT, MTS and mature frataxin (81-210 FRA), namely TAT MTS(cs) 81-210 FRA Δ linker, TAT MTS(fra) 81-210 FRA Δ linker, TAT MTS(lad) 81-210 FRA Δ linker and TAT MTS(orf) 81-210 FRA Δ linker, denoted by SEQ ID NO. 33, SEQ ID NO. 34, SEQ ID NO. 35 and SEQ ID NO. 36, respectively.

### Proteins expression and purification

*E. coli* BL21-CodonPlus (**λ**DE3) or HMS competent cells transformed with plasmids encoding the fusion proteins were incubated at 37°C in a saline lactose broth (SLB medium) containing kanamycine (50 µg/ml), tetracycline (12.5 µg/ml) and chloramphenicol (34 µg/ml). At an OD₆₀₀ of 0.2-0.3, 0.1% glycerol and 0.1 mM potassium glutamate were added to the culture which was then heat-shocked for 20-30 min at 42°C, after which the bacteria were grown at 37°C until an OD₆₀₀ of 0.8. Protein expression was induced by adding isopropyl-beta-D-thiogalactopyranoside (IPTG, for final concentrations see Table 2 below). After 18 hrs of incubation at 22°C, the cells were harvested by centrifugation (500 g for 15 min at 4°C).

For the purification procedure, bacteria pellets from 0.5 L culture of expressed cells were sonicated in binding buffer (PBS, pH 7.4, 0.4 M NaCl, 10% Glycerol, 1 mM phenylmethylsulphonylfluoride (PMSF) and 30 mM imidazole (Sigma-Aldrich, St. Louis, MO, USA)). The suspensions were clarified by centrifugation (35,000 g for 30 min at 4°C), and the supernatants containing the fusion proteins were purified under native conditions, using binding buffer pre-equilibrated HiTrap Chelating HP columns (Amersham-Pharmacia Biotech, Uppsala, Sweden). Columns were washed by stepwise addition of increasing imidazole concentrations. Finally, the target proteins were eluted with elution buffer (PBS, pH 7.4, 0.4 M NaCl, 10% Glycerol, 250 mM imidazole). All purification procedures were carried out using the FPLC system ÄKTA (Amersham-Pharmacia Biotech). Imidazole, NaCl and glycerol were removed by transferring the purified proteins to PBS using PD-10 desalting columns (GE Healthcare, Piscataway, NJ, USA). Aliquots of the proteins were kept frozen at -80°C until use.

**Table 2 Expression conditions of TAT-MTS-FRA fusion proteins**

| **Protein** | **Bacterial Host** | **Heat Shock** | **Temp. for induction (°C)** | **IPTG (mM)** |
|---|---|---|---|---|
| TAT-MTSfra-FRA | codonPlus | yes | 22 over night | 0.5 |
| TAT-MTScs-FRA | codonPlus | yes | 22 over night | 0.5 |
| TAT-MTSorf-FRA | HMS | yes | 22 over night | 1.0 |
| TAT-MTSlad-FRA | HMS | yes | 22 over night | 1.0 |

### Characterization of the fusion proteins

Determination of protein concentration Protein concentration was measured according to the Bradford method, using the Bradford reagent and the standard curve of BSA. Protein concentration was determined at a wavelength of 595 nm.

Separation of proteins by electrophoresis Samples from the various protein fractions (5-20 µg protein/lane) were loaded on 12% or 15% (w/v) SDS-PAGE gels. The separation of proteins was done using Sturdier Slab Gel Electrophoresis apparatus according to the manufacturer's instructions (Hoefer Sci Instruments, San Francisco, CA, USA).

Western blot analysis Proteins (5-20 µg protein/lane) were resolved on 12%-15% SDS-PAGE gels and transferred onto an Immobilon-P Transfer membrane (Millipore, Bradford, PA, USA). Western blot analysis was performed using either anti-frataxin (Abcam), or anti-His (Amersham-Pharmacia Biotech) antibodies at dilutions of 1:600 and 1:30,000, respectively, to identify the relevant proteins. Primary antibody binding was detected by blotting with a suitable secondary antibody conjugated to horseradish peroxidase (HRP) (1:10,000). Band visualization was done using an enhanced chemiluminescence kit (EZ-ECL, Biological Industries, Beit-Haemek, Israel).

### Internalization of TAT-MTS-FRA into the mitochondria of patients' fibroblasts

FA patient fibroblasts (Coriell repository, #GM03816, termed F816) were thawed and passed at least 2-3 times prior to assay performance and then incubated in the presence of the fusion protein construct TAT-MTS(cs)-FRA (20 µg/ml) comprising frataxin and citrate synthase as the mitochondrial targeting sequence (MTS) for 2, 6 and 48 hours, with fresh addition of TAT-MTS(CS)-FXN (at 20 µg/ml) after 24 hours. Vehicle was added as negative control. Cells were fractionated to cytosolic (C) and mitochondrial (M) fractions using Mitochondria isolation kit (Millipore, MIT1000). All fractions were analyzed in Western blot analysis using a monoclonal anti-frataxin antibody directed against the C-terminal region of frataxin (Abnova, cat. no. H00002395-M02, lot 08078-3F3) diluted 1:500 in blocking solution. Detection was performed using 1ml at 1 mg/ml of a goat anti mouse IgG-h+l alkaline phosphatase conjugated antibody (BETHYL, Cat#A90-116AP-16) diluted to 1:20,000 in blocking solution. Signal was developed using the ready-to-use buffered alkaline phosphatase substrate for use in immunoblotting BCIPO/NBT-Blue (Sigma, B3804).

### Aconitase activity assay

Aconitase activity assay was performed using a commercial kit (Abcam, ab109712, microplate assay kit) according to the manufacturer's instructions. The conversion of isocitrate to cis-aconitate is measured as an increase in absorbance at OD 240 nm at specific time points and calculated as activity rate (mOD/min).

### BSO experiments for inducing oxidative stress

Normal or patients' lymphocytes (4X10³ cells/100µl) were seeded in Dulbecco's Modified Eagle Medium (DMEM, Bet Ha'emek, Israel) medium without phenol red and sodium pyruvate (experimental medium) and after 3-4 hr the tested TAT-MTS-FRA fusion protein (at a final concentration of 0.1 µg/µl) was added to the cells for 5 or 24 hr. Following the incubation time with the fusion protein, L-Buthionine-sulfoximine (BSO, Sigma B2640) at different concentrations was added for an additional period of 48 hr. At the end of the incubation time, cell cultures were subjected to cell viability or cell proliferation assays, using the CellTiter-BlueTM kit (Promega, Madison, WI) according to manufacturer's instructions, as detailed below. In experiments conducted in fibroblasts, cells (3X10³ cells/100µl) were seeded in the growth medium as specified above and left for 24 hr to allow the cells to adhere. After 24 hr, the medium was changed to the experimental medium and the experiment was continued as described for lymphocytes above.

### Cell proliferation assay

10⁴/100 µl/well cells growing in suspension or 5 x 10³/100 µl/well adherent cells were seeded and treated with increasing concentrations of the fusion protein for 48-72 h, after which cellTiter-Blue® reagent (Promega, Madison, WI, USA) was added according to the manufacturer's instructions to determine cell survival. All treatments were performed in triplicates.

### In Vitro Caspase 3 Activity Assay

10⁴/100 µl/well cells growing in suspension or 5 x 10³/100 µl/well adherent cells were treated with the fusion protein (0.15 µg/µl, final concentration). Caspase3 activity within the cells was assessed by Apo-ONE® Homogeneous Caspase3/7 Assay Kit (Promega). Caspase 3 activity assays were carried in parallel to cell viability assays.

### Preparation of His-TAT-MTS-OTC constructs

Fusion protein constructs comprising ornithine transcarbamoylase (OTC) conjugated to a His-TAT-MTS fragment, namely, His-TAT-MTS(otc)-OTC, His-TAT-MTS(lad)-OTC, His-TAT-MTS(cs)-OTC and His-TAT-MTS(orf)-OTC were obtained from GenScript. These fusion protein constructs were prepared by expressing vectors harboring these constructs in *E. coli* cells and purified from inclusion bodies to about 80% purity using Ni column chromatography.

**Table 3 Nucleic acid and amino acid sequences of TAT-MTS-OTC constructs**

| SEQ ID NO. | Sequence | Name |
|---|---|---|
| 37 | | nt seq. of MTS of OTC |
| 38 | LFNLRILLNNAAFRNGHNFMVRNFRCGQPLQNKVQ | aa seq. of MTS of OTC |
| 39 | | aa seq. of mature OTC |
| 40 | | nt seq. of His-TAT-MTS(otc) -OTC Δ linker |
| | | |
| 41 | | nt seq. of |
| | | His-TAT-MTS(cs)-OTC |
| | | |
| 42 | | nt seq. of His-TAT-MTS(orf)-OTC |
| | | |
| 43 | | nt seq. of His-TAT-MTS(lad) -OTC |
| | | |
| 44 | | aa seq. of His-TAT-MTS(otc) -OTC Δ linker |
| 45 | | aa seq. of His-TAT- |
| | | MTS(cs)-OTC |
| 46 | | aa seq. of His-TAT-MTS(orf)-OTC |
| 47 | | aa seq. of His-TAT-MTS(lad) -OTC |
| | | |
| 48 | | aa seq. of TAT-MTS(otc) -OTC Δ linker |
| 49 | | aa seq. of TAT-MTS(cs)-OTC |
| 50 | | aa seq. of TAT-MTS(orf)-OTC |
| | | |
| 51 | | aa seq. of TAT-MTS(lad) -OTC |
| 52 | | aa seq. of TAT-MTS(cs)-OTC Δ linker |
| 53 | | aa seq. of TAT-MTS(orf)-OTC Δ linker |
| 54 | | aa seq. of TAT-MTS(lad) -OTC Δ linker |

Table 3 recites the amino acid sequence of the MTS of the OTC protein and the nucleotide sequence encoding therefor (denoted by SEQ ID NO. 38 and by SEQ ID NO. 37, respectively), the amino acid sequence of mature OTC (denoted by SEQ ID NO. 39), and the nucleotide sequences encoding the fusion protein constructs His-TAT-MTS(otc)-OTC Δ linker, His-TAT-MTS(cs)-OTC, His-TAT-MTS(orf)-OTC and His-TAT-MTS(lad)-OTC (denoted by SEQ ID NO. 40, denoted by SEQ ID NO. 41, SEQ ID NO. 42 and by SEQ ID NO. 43, respectively).

Table 3 also indicates the amino acid sequences of the fusion protein constructs His-TAT-MTS(otc)-OTC Δ linker, His-TAT-MTS(cs)-OTC, His-TAT-MTS(orf)-OTC and His-TAT-MTS(lad)-OTC (denoted by SEQ ID NO. 44, SEQ ID NO. 45, SEQ ID NO. 46 and by SEQ ID NO. 47, respectively).

Also recited are fusion protein constructs comprising the mitochondrial active protein OTC where the fusion protein construct does not include a His tag, namely the constructs TAT-MTS(otc)-OTC Δ linker having the amino acid sequences denoted by SEQ ID NO. 48, TAT-MTS(cs)-OTC (denoted by SEQ ID NO. 49), TAT-MTS(orf)-OTC (denoted by SEQ ID NO. 50) and TAT-MTS(lad)-OTC (denoted by SEQ ID NO. 51).

The fusion protein constructs according to the present disclosure may be prepared in the presence of a linker situated between the TAT and the MTS fragments or in its absence. Thus table 3 also recites the sequences of the fusion protein constructs termed His-TAT-MTS(otc)-OTC Δ linker, TAT-MTS(cs)-OTC Δ linker, TAT-MTS(orf)-OTC Δ linker, TAT-MTS(lad)-OTC Δ linker (having the amino acid sequences denoted by SEQ ID NO. 44, SEQ ID NO. 52, SEQ ID NO. 53 and SEQ ID NO. 54, respectively).

### Internalization of His-TAT-MTS-OTC constructs into the mitochondria

A culture of 10⁷ HepG2 cells (ATCC, #HB-8065) was seeded in T-75 flasks. The next day, TAT-MTS-OTC fusion protein constructs comprising either OTC, CS or LAD at 12µg/ml prepared as described above were added to the cells in Eagle's Minimum Essential Medium (EMEM) complete medium, or in EMEM medium supplemented with DMSO (1%) and Trehalose (30mM) or in EMEM medium supplemented with DMSO (1%), Trehalose (30mM) and Ornithine (1mM) for an incubation of 20min, 1 or 3 hours. Cells were then fractionated to cytosolic and mitochondrial fractions using Mitochondria isolation kit (Millipore, MIT1000) according to the manufacturer's instructions. Fractions were analyzed in Western blot analysis using a monoclonal antibody directed to the C terminal region of OTC (Aviva Systems Biology, cat. no. ARP41767_P050), at 1 mg/ml diluted 1:1000. Detection was performed with the goat anti rabbit IgG (H_L) secondary antibody peroxidase conjugated (Jackson AffiniPure, Code 111-035-003) at 0.8 mg/ml, diluted 1:20,000 in blocking buffer (). Signal was developed with chemiluminescence mixture (SC-2048 Santa Cruz) according to the manufacturer's instructions.

### In vitro production of citrulline by the enzymatic activity of OTC

Purified TAT-MTS-OTC fusion protein constructs comprising the MTS of OTC or CS prepared as described above were tested for their enzymatic activity as described in [40]. Briefly, proteins were diluted in a mitochondria lysis buffer (0.5% Triton, 10mM HEPES, pH 7.2 and 2mM dithiothreitol) to a concentration of 20µg in a volume of 80µl. Then the volume of each of the reaction mixtures was completed to 600µl by adding 520µl reaction mixture (5mM ornithine,15mM carbamoyl phosphate, and 270mM triethanolamine, pH 7.7). Following vortexing, each reaction mixture was divided into two separate tubes, the first tube was incubated at 37°C for 30min and the second tube served as "no-reaction" background and used to subtract endogenous signal of OTC proteins. Reactions were terminated by adding 150µl of 1:3 sulfuric acid/phosphoric acid (by volume). Citrulline production was then determined by adding 20µl of 3% 2,3-butanedione monoxime, incubating at 100°C in the dark for 15 min, and measuring absorbance at 490 nm. Commercially available OTC from *Bacillus subtilis* (Sigma, 5ng) was used as Positive Control (data not shown).

### Enzymatic activity of OTC in HepG2 cells in the presence of ammonia

HepG2 cells 2.5x10⁵ (ATCC, #HB-8065) per well were seeded in two 6-well plates. The next day, TAT-MTS-OTC fusion protein constructs comprising the MTS of OTC or CS (prepared as described above) were applied to the cells for 72 hours with replenishment after each 24 hours. The final concentration of the fusion proteins was 14µg/ml, in 3ml complete medium and the final buffer dilution was 1:85. Proteins storage buffer (1×PBS, 0.5% Sodium Lauroyl Sarcosine at pH 7.4) served as vehicle control and untreated cells as negative control. At 48 hours prior to the termination of the assay, cells were treated with 0.5% serum growth medium in the absence or in the presence of 5mM Ammonium chloride. Cells viability was monitored for 4 hours with alamarBlue Cell Viability Assay Reagent (Pierce). Fluorescence signal was obtained by excitation at 544nm and emission at 590nm.

### EXAMPLE 1

### Cloning of plasmids encoding TAT-MTS-FRA fusion proteins

Expression plasmids encoding the TAT-fusion proteins were cloned and prepared by standard molecular biology tools known in the art. For a general reference see Molecular Cloning: A Laboratory manual (2001) Joseph Sambrook and David William Russell. TAT [9] having the amino acid sequence as denoted by SEQ ID NO. 27 (encoded by the nucleic acid sequence denoted by SEQ ID NO. 1) was fused (N-terminal) to the mature human frataxin protein (having the amino acid sequence as denoted by SEQ ID NO. 26 and encoded by the nucleic acid sequence as denoted by SEQ ID NO. 6).

Various fusion constructs were prepared, which differ in their mitochondrial targeting sequence present at the N terminus of human frataxin (and thus located between the TAT and mature human frataxin described above), being either the native mitochondrial targeting sequence of frataxin (referred to herein as MTSfra, having the amino acid sequence as denoted by SEQ ID NO. 22 and encoded by the nucleic acid sequence as denoted by SEQ ID NO. 2) or other defined MTSs of human mitochondrial proteins, including lipoamide dehydrogenase (referred to herein as MTSlad, having the amino acid sequence as denoted by SEQ ID NO. 24 and encoded by the nucleic acid sequence as denoted by SEQ ID NO. 5), C6ORF66 (referred to herein as MTSorf, having the amino acid sequence as denoted by SEQ ID NO. 25 and encoded by the nucleic acid sequence as denoted by SEQ ID NO. 4) and citrate synthase (referred to herein as MTScs, having the amino acid sequence as denoted by SEQ ID NO. 23 and encoded by the nucleic acid sequence as denoted by SEQ ID NO. 3). The various TAT-MTS-FRA fusion proteins are summarized in Table 4 below and schematically presented in Figure 1.

All plasmids were cloned with His-tag at the 5'-terminus of the coding sequence and all coding sequences were under the control of the T7 promotor. All clones were confirmed by restriction enzymes and sequencing analyses.

**Table 4 The cloned plasmid constructs**

| No. | Plasmid name | Abbreviated name |
|---|---|---|
| 1 | His-TAT-MTSfra-FRA | FRA |
| 2 | His-TAT-MTSlad-FRA | (lad)FRA |
| 3 | His-TAT-MTSorf-FRA | (orf)FRA |
| 4 | His-TAT-MTScs-FRA | (cs)FRA |

| | | |
|---|---|---|
| Abbreviations: lad, Lipoamide dehydrogenase protein (E3 subunit); orf, C6ORF66 assembly factor; and cs, citrate synthase. | | |

As indicated above, the amino acid sequences of the fusion proteins obtained from the cloned plasmid constructs indicated as 1-4 in Table 4 above are denoted by SEQ ID NO. 18, SEQ ID NO. 19, SEQ ID NO. 20 and SEQ ID NO. 17, respectively.

### EXAMPLE 2

### Expression of the fusion proteins in E. coli hosts

Expression of the fusion proteins was performed in *E. coli* hosts as described below and was calibrated for optimal expression conditions. As known in the art, there are several different bacterial expression systems. Successful expression of recombinant proteins is often dependent on the strain of the bacteria expression system used. Thus, for each fusion protein prepared as described above, four different *E. coli* bacterial stains were tested: BL21-CodonPlus, BL21, Rosetta and HMS (Invitrogen, USA) and the host for expression was thereby selected. The conditions for expression were also calibrated for each of the TAT-fusion proteins, by changing several parameters, including the concentration of the inducer Isopropyl β-D-1-thiogalactopyranoside (IPTG) and length of induction growth conditions (i.e. temperature, addition of chemicals, etc.)

Upon expression, bacterial cells were disrupted and cellular sub-fractions were prepared, separating the soluble and non-soluble fractions. Analysis was performed for the whole-cell bacteria (W.C. or whole-cell extract), the soluble fraction (Sol) and insoluble fraction (Insol) on sodium dodecyl sulfate polyacrylamide gel electrophoresis (SDS-PAGE) gels in order to examine whether the fusion protein was expressed and at which sub-cellular fraction it accumulates. The goal was to obtain high expression levels of the different TAT-fusion proteins in the soluble sub-fraction of the expressing bacteria, for future purification. The different TAT-fusion proteins were also characterized by Western blots analyses using both anti-His and anti-frataxin antibodies. Table 2 above summarizes the bacterial host, IPTG concentration and temperature for production of each TAT-MTS-FRA fusion proteins carrying a different MTS sequence.

Typical expression and sub-cellular localization of each of the four fusion proteins as characterized by SDS-PAGE gels and Western blots using anti-His antibodies are demonstrated in Figure 2 and in Figure 3. Expression of the His-TAT-MTSfra-FRA fusion protein is shown in Figure 2A-2B, Expression of the His-TAT-MTSorf-FRA fusion protein is shown in Figure 2C-2D, Expression of the His-TAT-MTSlad-FRA fusion protein is shown in Figure 3A-3B and Expression of the His-TAT-MTScs-FRA fusion protein is shown in Figure 3C-3D. These experiments confirmed the full-length expression of the different TAT-fusion proteins and their identity.

It should be pointed out the anti-His antibodies recognize, most probably, the various fusion proteins with different efficacy, depending on the exposure or availability of the His sequence in the final protein preparation, for antibody interactions. Thus, expression levels of the various fusion proteins are determined based on the SDS-PAGE gels.

As can be seen in Figure 2 and in Figure 3, the TAT-MTSfra-FRA fusion protein (Figure 2A & Figure 2B) was expressed at low levels in the bacterial hosts, as compared to the other three fusion proteins carrying an heterologous MTS (Figure 2C & Figure 2D and Figure 3A-3D), even under the best calibrated conditions. Thus, using a heterologous MTS instead of the native frataxin-MTS has an advantage in its expression levels in a bacterial host. This has major implications on its future development for human use, where large quantities of the fusion protein are needed to be produced (see also below).

### EXAMPLE 3

### Purification of the TAT-MTS-FRA-fusion proteins

The soluble fractions of the expressed TAT-MTS-FRA fusion proteins were loaded onto a nickel-chelating column to affinity-purify these proteins, as detailed above. Calibration experiments were performed for each of the fusion proteins, including specific conditions for binding of the fusion protein onto the affinity column, its elution and removal of the imidazole from the final protein preparations. One typical purification run of each of the fusion proteins is demonstrated in Figure 4 (for His-TAT-MTSfra-FRA and His-TAT-MTSorf-FRA) and in Figure 5 (for His-TAT-MTSlad-FRA and His-TAT-MTScs-FRA).

For example, Figure 4A shows an image of an affinity chromatography purification profile obtained for the fusion protein TAT-MTSfra-FRA. As can be seen in Figure 4B, which is an image of SDS-PAGE analysis of the purification steps of this protein construct, the protein fraction shown in Figure 4B, lane 7, represents the fusion protein TAT-MTSfra-FRA that was eluted from the column at 250 mM imidazole.

Corresponding analyses are shown for TAT-MTSorf-FRA in Figure 4C and Figure 4D, for TAT-MTSlad-FRA in Figure 5A and Figure 5B and for TAT-MTScs-FRA corresponding analyses are shown in Figure 5C and Figure 5D.

As shown in Figure 6A, eluted proteins showed a major band of the expected size (approximately 20-27 kDa) and were >95% pure, as determined by SDS-PAGE analysis and by Western blot analyses using both anti-His (Figure 6B) and anti-Fra antibodies (Figure 6C). As can be seen in Figure 6, TAT-MTS-FRA fusion proteins carrying a heterologous MTS were full-length, intact proteins with no evidence for protein degradation (see lane 2 for TAT-MTScs-FRA, lane 3 for TAT-TSlad-FRA and lane 4 for TAT-MTSorf-FRA). However, TAT-MTSfra-FRA carrying the native MTS sequence was partially degraded (see lane 1 in Figure 6B and in Figure 6C). Thus, using a heterologous MTS sequence for the TAT-FRA fusion protein has an advantage also for keeping the fusion protein intact and stable.

In addition, when comparing the total amounts and concentrations of each of the fusion proteins, which were produced from the same starting volume of bacterial cultures, fusion proteins carrying an heterologous MTS were produced in larger amounts and at higher concentration as compared to that of fusion protein carrying the native MTS (Table 5, below). This has again major implications on its future development for human use, where large quantities of the fusion protein are needed and at high concentrations. Moreover, the stability of the produced fusion protein has an additional advantage.

**Table 5 TAT-MTS-FRA fusion proteins**

| **Protein** | **Final concentration (mg/ml)** | **Amount purified from 0.5L bacterial culture (mg)** |
|---|---|---|
| TAT-MTSfra-FRA | 0.2 | 0.6-0.7 |
| TAT-MTScs-FRA | 1.0 | 3-4 |
| TAT-MTSorf-FRA | 1.0 | 3-4 |
| TAT-MTSlad-FRA | 0.8 | 2.4-3.2 |

### EXAMPLE 4

### Internalization of TAT-MTS-FRA fusion proteins

In order to test the ability of the fusion protein to reach the mitochondria within intact cells, human BJAB cells were incubated with one of the purified fusion proteins, namely, TAT-MTSlad-FRA. After incubation, sub-cellular fractions were prepared, to separate the mitochondria and the cytosol. The mitochondria were then treated with proteinase K (Sigma P2308) to digest proteins nonspecifically adsorbed to the outer membrane, thereby ensuring that the mitochondrial extract represented only proteins within the mitochondria. Samples were then analyzed by Western blot assay for the presence of the FRA- based fusion protein, using both anti-His and anti-FRA antibodies (see Figure 7).

As shown in Figure 7A, using anti-His antibodies the results indicated the presence of the His-tagged TAT-MTSlad-FRA fusion protein within the mitochondrial fractions of the treated cells after 1 and 5 hours of incubation (lane 4 and lanes 6 & 7, respectively).

As shown in Figure 7B, using anti-FRA antibodies, the presence of a frataxin fusion protein construct inside the mitochondria is indicated. From lane 2 of Figure 7B it is evident that control cells that were not treated with the fusion protein have endogenous FRA protein, most probably, both the mature isoform and the intermediate isoform (indicated by the two arrows).

As shown in Figure 7B (lanes 4, 6, 7), upon treatment with the TAT-MTSlad-FRA fusion protein, the relative amounts of both the unprocessed fusion protein as well as of the mature protein increased with the incubation time (Figure 7B; upper and lower bands, respectively). Without wishing to be bound by theory, the increase in the upper band is explained by fusion protein construct that entered the mitochondria and was not processed.

Internalization of fusion proteins comprising frataxin into the mitochondria of cells was examined also for other TAT-MTS-FRA fusion proteins, namely, TAT-MTSfra-FRA, TAT-MTScs-FRA, and TAT-MTSorf-FRA (as well as for TAT-MTSlad-FRA), as shown in Figure 8A. In this experiment, cells were incubated for 3 hours with each of the TAT-MTS-FRA fusion proteins (at a final concentration of 0.02 µg/µl). The cells were then washed, their mitochondria were isolated and then submitted to Western blot analysis using monoclonal anti-FRA antibodies (Abnova, Taiwan; 1µLg/ml) that are directed to the C-terminus of the frataxin protein.

As demonstrated in Figure 8A, lanes 2-5, a slight size variation was observed among the various fusion protein constructs, resulting from the variation in the length of the various MTS polypeptides (see Figure 1).

As shown in Figure 8A, internalization into mitochondria was observed for all four TAT-MTS-FRA fusion proteins. Remarkably, as shown in lane 3 of Figure 8A, among the fusion proteins with a MTS that is heterologous to frataxin, the citrate synthase MTS (MTScs) was demonstrated to be delivered most efficiently into the mitochondria.

As indicated above, there is a slight variation in the lengths of the fusion protein constructs that entered the mitochondria, indicating that under the present experimental conditions (namely incubation of cells for 3 hours with each of the TAT-MTS-FRA fusion proteins) the frataxin protein was not cleaved from its respective His-TAT-MTS-frataxin construct inside the mitochondria (protein constructs that still include intact His-TAT-MTS-frataxin are termed below as "un-processed fusion construct").

Notably, Figure 8A also shows that while for the fusion proteins comprising an MTS which is heterologous to frataxin, namely the MTS of citrate synthase (cs), lipoamide dehydrogenase (lad) and C6ORF66 (orf), only a single band (representing the un-processed fusion construct) was demonstrated inside the mitochondria, two distinct bands appeared for the fusion protein comprising the native MTS of frataxin (Figure 8A, lane 2). Without wishing to be bound by theory, this may be the result of instability of the intact fusion protein comprising the TAT and native frataxin MTS regions.

The antibodies used to detect frataxin in the internalization assay presented in Figure 7B were polyclonal antibodies directed against human frataxin. These antibodies recognize the full-length, unprocessed human frataxin very well, as well as its processed products. In Figure 8 referred to below, detection was performed using monoclonal antibodies specifically directed against the C-terminus of the frataxin protein. As these monoclonal antibodies are less efficient in recognizing the protein, particularly at the amounts of protein detected in Figure 8, the results of Figure 7 and Figure 8 cannot be compared.

Control Western blot analysis using anti-E1α antibodies (Molecular Probes, Eugene, OR) at a dilution of 1:1,000) are demonstrated in Figure 8B.

### EXAMPLE 5

### His-TAT-MTS(cs)-FRA enter the mitochondria of patients' fibroblasts

Further to the above results demonstrating the ability of the frataxin fusion proteins to enter mitochondria of human BJAB cells, the internalization of a frataxin fusion protein comprising the MTS of citrate synthase into the mitochondria of fibroblasts obtained from Friedreich's ataxia patients was examined, as detailed below.

Fibroblasts obtained from Friedreich's ataxia patients were treated as described above and incubated in the presence of His-TAT-MTS(cs)-FRA (at 20µg/ml) for 2, 6 and 48 hours, with fresh addition after 24 hours. Mitochondrial and cytosolic fractions of the fibroblasts were analyzed by Western blot analysis as described above.

As demonstrated in Figure 9, the fusion protein construct His-TAT-MTS(cs)-FRA was detected in mitochondrial fractions of fibroblasts after an incubation of 2, 6 and 48 hours. The two bands detected by the anti-frataxin antibody (indicated by the upper and lower arrows in Figure 9) indicate that the fusion protein was processed inside mitochondria. Frataxin was not detected in the cytosolic fractions of the fibroblasts indicating that all of the His-TAT-MTS(cs)-FRA fusion protein construct has entered mitochondria.

### EXAMPLE 6

### Aconitase activity in fibroblasts obtained from patients following administration of His-TAT-MTS(cs)-FRA

It has been reported that the reduction in the levels of frataxin within the mitochondria has two direct effects on several tissue types, namely impaired formation of iron-sulfur (Fe-S) clusters and a rise in intracellular reactive oxygen species (ROS) [35]. The decrease in Fe-S containing proteins, such as heme, electron transport chain (ETC) complexes I-III and the Kreb's cycle protein aconitase severely impairs cellular respiration [36], which is further complicated by simultaneous oxidative damage to these mitochondrial proteins. These events all culminate in an inability of the mitochondria to fulfill the cell's energy requirements resulting in cell death [35].

In order to assess the activity of frataxin inside mitochondria, the level of aconitase activity in mitochondria obtained from Friedreich's ataxia patients was examined upon administration of His-TAT-MTS(cs)-FRA.

Aconitase activity (mOD/min) was measured by following the conversion of isocitrate to cis-aconitate, as described above, in mitochondrial fractions of fibroblasts (F816) obtained from Friedreich's ataxia patients that were treated as described above and incubated in the presence of His-TAT-MTS(cs)-FRA or vehicle for 48 hours (FXN or VEH, respectively). His-TAT-MTS(cs)-FRA was administered at 20 µg/ml and an additional dose of 20 µg/ml was administered 24 hours after the first administration). HepG2 whole cells homogenate served as positive control (POS.CON).

As shown in Figure 10, aconitase activity was higher in fibroblasts that were incubated with His-TAT-MTS(cs)-FRA (FXN, 14.5) compared to fibroblasts that were incubated in the presence of the vehicle control (VEH, 10.5). This finding is a clear demonstration that the fusion protein not only enters mitochondria, but is also active and available for its various cellular activities.

HepG2 whole cells homogenate served as positive control (POS.CON).

### EXAMPLE 7

### TAT-MTS-FRA fusion proteins partially rescue FA-patients' cells as well as normal cells from oxidative stress

As indicated above, the reduction in the levels of frataxin within the mitochondria leads to a rise in intracellular reactive oxygen species (ROS) [35].

L-Buthionine sulphoximine (BSO) is an inhibitor of gamma-glutamylcysteine synthetase (gamma-GCS) and, consequently lowers tissue glutathione (GSH) concentrations. GSH plays an important role in cellular defense or protection against a wide variety of toxic electrophiles via the formation of thioether conjugates. Therefore, BSO assay was used to inhibit de novo glutathione synthesis, depleting an important component of these cells' intrinsic defenses against reactive oxygen species (ROS) and allowing for the accumulation of ROS produced by natural cell processes, known to result in cell death [37]. The mechanism by which BSO inhibits production of GSH and results in cell death was described by Richardson, T. E. et al. [37]. Because they are lacking in Frataxin, Friedreich ataxia cells are extremely sensitive to BSO-induced oxidative stress compared with normal cells [37], and thus are used as an *in vitro* model of the long-term consequences of absent Frataxin.

Oxidative stress was induced with various concentrations of BSO in patients' cells as well as in normal healthy cells and the effect of the various TAT-MTS-FRA fusion proteins on cell death was measured. As can be seen in Figure 11, BSO caused cell death of normal lymphocytes as well as of cells obtained from Friedreich ataxia patients. However, patients' cells were more sensitive to BSO-induced oxidative stress, consistent with previous findings, showing higher percentages of cell death. Most importantly, the various TAT-MTS-FRA fusion proteins, which were added a few hours before oxidative stress induction, were demonstrated to partially rescue both normal lymphocytes as well as patients' cells from cell death. This partial rescue was determined by both reduction in cell death and by reduction in caspase 3 activity, as demonstrated in Figure 11B.

As shown in Figure 11A and Figure 11B, at least two out of the three fusion proteins carrying a heterologous MTS (namely, MTSorf and MTScs) demonstrated a superior protective effect with respect to the effect demonstrated by the fusion protein carrying the native MTS, in both patients' cells as well as in healthy cells, from BSO-induced oxidative stress.

A comparative study of the ability of the various TAT-MTS-FRA fusion proteins to rescue BSO-induced oxidative stress of patients' fibroblasts performed as detailed above is shown in Figure 12A - Figure 12D for the fusion protein constructs TAT-MTSfra-FRA, TAT-MTScs-FRA, TAT-MTSlad-FRA and TAT-MTSorf-FRA, respectively.

Interestingly, as demonstrated in Figure 12B and in Figure 12C, respectively, two of the protein constructs comprising heterologous MTS, namely the TAT-MTScs-FRA and TAT-MTSlad fusion proteins were more efficient than the fusion protein comprising the native frataxin MTS (TAT-MTSfra-FRA) in partially rescuing BSO-induced oxidative stress of patients' fibroblasts.

Similar protective effects in patients' cells induced with various concentrations of BSO were also observed for TAT-MTSorf-FRA and TAT-MTSlad fusion proteins when assayed alone (Figure 13 and Figure 14, respectively).

### EXAMPLE 8

### Pharmacodynamic and pharmacokinetic assessment of TAT-MTS-FRA fusion proteins in Friedreich's ataxia mice

In an ongoing study, in order to evaluate pharmacodynamic, pharmacokinetic (PK/PD) and safety of TAT-MTS-FRA fusion proteins in a Friedreich's ataxia preclinical mouse model, the mouse model JR# 18299 FVB; B6- Tg(FXN)1Sars *Fxn_{tm1Mkn}*/J is used in an ongoing study. The information obtained from these studies will be used for determining dose and time interval for further clinical studies. Briefly, the study requires 45 female mice, 6-8 weeks of age that are homozygous for the targeted mutation at the mouse Frataxin locus and hemizygous for the transgene. It is noted that strain 18299 is the original Sarsero mouse on a mixed genetic background, which was selected over the original C57BL/6J congenic strain due to its superior breeding performance and since no behavioral phenotyping is performed in this study.

In order to perform the preclinical study, two doses of TAT-MTScs-FRA fusion protein (at 100 µg and 400 µg) are administered twice per week for a maximal period of three weeks along with a vehicle, via tail vein injection into 18229 FVB;B6-Tg(FXN)1Sars FxntmlMkn/J mice. Mice are sacrificed 1, 4, 7, 14 or 21 days post injection and then blood is collected by cardiac puncture and various tissues are harvested (e.g. brain, heart, liver and kidney). Brain and heart tissue are processed to measure aconitase activity. Brain and heart tissue are also processed to measure frataxin levels by ELISA assay in mitochondrial extracts. Blood, liver and kidney are flash-frozen and stored.

The effect of the treatment is then assessed in the harvested tissues.

### COMPARATIVE EXAMPLE 9

### TAT-MTS-OTC protein constructs internalize into mitochondria

Fusion protein constructs comprising OTC fused to a His-TAT-MTS were prepared as described above. As shown in Figure 16, purified fusion protein constructs comprising the native MTS of OTC, the MTS of citrate synthase, the MTS of C6ORF66 and the MTS of lipoamide dehydrogenase were obtained (Figure 16A, Figure 16B, Figure 16C and Figure 16D, respectively). The size of the purified fusion protein constructs obtained was about 40 kDa as determined based on comparing their gel migration to the migration of the protein marker shown in Figure 16E,). Similar expression yields were obtained for all four fusion protein constructs that were prepared.

In order to examine the ability of the various OTC fusion protein constructs to enter mitochondria, HepG2 cells were incubated in the presence of the OTC fusion proteins constructs comprising either the native MTS of OTC or an MTS that is heterologous to OTC, namely, His-TAT-MTSotc-OTC, His-TAT-MTScs-OTC and TAT-MTSlad-OTC as described above.

First, as shown in Figure 17A, it is noteworthy that the fusion protein constructs His-TAT-MTSotc-OTC, His-TAT-MTScs-OTC and His-TAT-MTSlad-OTC (and His-TAT-MTSorf-OTC, data not shown) were soluble under the various assay conditions based on the fact that no precipitation or degradation products were detected.

Analysis of mitochondrial fractions of HepG2 cells incubated with the above constructs revealed that the OTC fusion protein construct comprising the native MTS of OTC (His-TAT-MTSotc-OTC) as well as the OTC fusion protein constructs comprising an MTS that is heterologous to the OTC protein, namely citrate synthase (cs) and lipoamide dehydrogenase (lad) were able to enter mitochondria after an incubation period of 1 hour (Figure 17A). Figure 17A also shows that prolonging the incubation period to 3 hours resulted in an increase in the level of the fusion protein construct inside the mitochondria.

Interestingly, by comparing the ability of the protein construct comprising the native MTS of OTC (His-TAT-MTSotc-OTC) to the ability of OTC fusion protein constructs comprising an MTS that is heterologous to the OTC protein (namely His-TAT-MTScs-OTC and His-TAT-MTSlad-OTC) for example upon incubation of 3 hours in EMEM, it appears that the internalization ability of the fusion protein constructs comprising an MTS that is heterologous to OTC was slightly higher than the internalization ability of the fusion protein construct comprising the native MTS of OTC.

In order to further confirm the internalization of the OTC fusion protein constructs into mitochondria, the cytosolic fractions of the cells incubated with the various His-TAT-MTS-OTC protein constructs were also analyzed. As shown in Figure 18A and Figure 18B, no fusion protein constructs were observed in any of the assayed cytosolic fractions.

### COMPARATIVE EXAMPLE 10

### In vitro enzymatic activity of OTC

As indicated above, OTC is a protein having enzymatic activity that catalyzes the reaction between carbamoyl phosphate and ornithine to form citrulline and phosphate. In order to assess the activity of the purified fusion protein constructs comprising OTC described herein above, the production of citrulline from ornithine and carbamoyl phosphate by various fusion protein constructs was evaluated, as described above.

As shown in Figure 19, the fusion protein construct His-TAT-MTScs-OTC assayed had relatively low enzymatic activity, where the fusion protein construct His-TAT-MTSotc-OTC had none. Without wishing to be bound by theory, this may be due to the fact that OTC is probably not present in the reaction mixture at its native trimeric state, which is necessary for its enzymatic activity, since it is conjugated to a His-TAT-MTS fragment.

However, as shown in Figure 19 a low level of enzymatic activity was indeed observed for the fusion protein construct comprising the MTS of CS.

### COMPARATIVE EXAMPLE 11

### Enzymatic activity of OTC in HepG2 cells suffering from ammonia stress

OTC deficiency is the most common urea cycle disorder in humans. OTC, the defective enzyme in this disorder, is the final enzyme in the proximal portion of the urea cycle, and is responsible for converting carbamoyl phosphate and ornithine into citrulline, as indicated above. In severely affected individuals, ammonia concentrations increase rapidly causing ataxia, lethargy and death without rapid intervention.

In order to test the ability of the OTC fusion protein constructs to rescue ammonia stress in cells, HepG2 cells were administered daily (for 3 days) with a fusion protein construct comprising the native MTS of OTC or with fusion protein construct comprising the MTS of CS then treated with ammonium chloride, and finally, their cell viability was evaluated using the alamarBlue indicator, as described above.

As shown in Figure 20, the level of cell viability in the presence of fusion protein constructs comprising OTC and citrate synthase as the MTS was similar to the level of cell viability for cells which were not exposed to ammonium chloride (untreated cells). Furthermore, the level of cell viability in the presence of a fusion protein construct comprising OTC and citrate synthase as the MTS was higher from the level of cell viability in cells treated with the fusion protein construct comprising the native MTS of OTC.

Taken together, the above results demonstrate that fusion protein constructs comprising TAT, MTS and OTC and in particular such fusion protein which comprise an MTS which is heterologous to OTC are able to enter mitochondria, the OTC is then processed, thereby enabling its enzymatic activity.

The results also indicated that the fusion protein constructs without ammonia were not cytotoxic (data not shown).

### COMPARATIVE EXAMPLE 12

### Kinetics of liver entry and dose determination of TAT-MTS-OTC fusion protein constructs in mice

In order to assess the kinetics of liver entry of OTC, liver levels of any of the TAT-MTS-OTC fusion protein constructs described herein are determined in OTC-deficient *spf^{ash}* mice (3 hemizygous males and 3 homozygous females at group) 4, 8, 24 and 48 hours after administration of a fusion protein construct comprising OTC (500µg/mouse). Liver pieces and other tissues (e.g. skeletal muscle, brain) are then harvested and analyzed for their OTC protein levels by Western blot analysis. In addition, the activity of OTC is examined in harvested liver, by using an OTC enzymatic assay (for example the citrulline assay described above).

Blood is collected by cardiac puncture and plasma is isolated for liver function tests (ALT/AST), amino acids (including citrulline), ammonia determination and OTC protein levels (Western blot). Urine is collected for orotic acid analysis.

Male mice are used for further studies in case the above experiments do not indicate any differences between males and females.

Then, protein levels are determined in tissues of mice administered with various doses of a TAT-MTS-OTC fusion protein construct (100-500µg/mouse) at the time point for which the highest OTC protein levels were detected in the above assay.

### COMPARATIVE EXAMPLE 13

### Phenotype protection by TAT-MTS-OTC fusion protein constructs

OTC-deficient *spf^{ash}* mice (6 males or 3 males and 3 females) are administered with OTC-shRNA knockdown rAAV according to Cunningham, S. C. et al. [41]. At a designated time-point (based on the activity of the OTC-shRNA batch and results obtained from the above experiments) dosing with a TAT-MTS-OTC fusion protein construct is commenced by administering 0, 1, 2 or 3 dose(s) per week to each tested group of mice.

Analysis is performed by first measuring baseline plasma levels of ammonia, amino acids (including citrulline) and ALT/AST as well as urinary orotic acid prior to injection of OTC-shRNA knockdown rAAV (blood is collected via tail vein nicking). Blood and urine are collected again prior to administration of a TAT-MTS-OTC fusion protein construct for the above analyses and then weekly for the duration of the experiment.

Mice are monitored and sacrificed upon observation of clinical signs of hyperammonaemia (lethargy, tremors, ataxia) or 1 month post-injection of a TAT-MTS-OTC fusion protein construct. At the termination of the experiment, liver, muscle and brain are frozen in liquid nitrogen or fixed in 4% PFA for vector copy analysis (quantitative PCR), OTC activity (in liver lysate and frozen sections) and protein levels (Western blot).

## Claims

1. A fusion protein comprising a HIV-1 transactivator of transcription (TAT) domain, a functional human mitochondrial protein and a human mitochondria targeting sequence (MTS) situated between said TAT domain and said functional human mitochondrial protein and wherein said human MTS is heterologous to said functional human mitochondrial protein, and wherein said functional human mitochondrial protein is frataxin.

2. The fusion protein according to claim 1, wherein said functional human mitochondrial protein is C-terminal to said human MTS.

3. A fusion protein according to claim 1 or claim 2, wherein said mitochondrial protein is a functional human mitochondrial protein *per se* and/or is a component of a mitochondrial multi-component complex.

4. A fusion protein according to any one of the preceding claims, wherein said MTS comprises from 15 to 40 amino acid residues, including from 3 to 5 nonconsecutive basic amino acid residues.

5. A fusion protein according to claim 4, wherein said MTS comprises from 1 to 3 or 4 or 5 serine/threonine residues.

6. A fusion protein according to any one of the preceding claims, wherein said MTS is any one of human mitochondrial citrate synthase MTS having the amino acid sequence denoted by SEQ ID NO. 23, the human lipoamide dehydrogenase MTS having the amino acid sequence denoted by SEQ ID NO. 24, the MTS of the human C60RF66 gene product having the amino acid sequence denoted by SEQ ID NO. 25 and the MTS of human mitochondrial GLUD2 encoded by the nucleic acid denoted by SEQ ID NO. 16.

7. A fusion protein according to claim 6, wherein said MTS is any one of human mitochondrial citrate synthase MTS having the amino acid sequence denoted by SEQ ID NO. 23 and the human lipoamide dehydrogenase MTS having the amino acid sequence denoted by SEQ ID NO. 24.

8. A fusion protein according to any one of the preceding claims, further comprising a linker covalently linking said TAT domain to said MTS sequence.

9. A fusion protein according to claim 1, having the amino acid sequence denoted by SEQ ID NO. 28, comprising a HIV-1 transactivator of transcription (TAT) domain having the amino acid sequence denoted by SEQ ID NO. 27 fused to human frataxin having the amino acid sequence denoted by SEQ ID NO. 26 and a mitochondria targeting sequence (MTS) of human citrate synthase having the amino acid sequence denoted by SEQ ID NO. 23, said MTS situated between said TAT domain and said frataxin, and is linked to said TAT domain via a linker having the amino acid sequence denoted by SEQ ID NO. 32, and wherein said frataxin is C-terminal to said MTS of human citrate synthase.

10. A fusion protein according to claim 1, having the amino acid sequence denoted by SEQ ID NO. 30, comprising a HIV-1 transactivator of transcription (TAT) domain having the amino acid sequence denoted by SEQ ID NO. 27 fused to human frataxin having the amino acid sequence denoted by SEQ ID NO. 26 and a mitochondria targeting sequence (MTS) of human lipoamide dehydrogenase having the amino acid sequence denoted by SEQ ID NO. 24, said MTS situated between said TAT domain and said frataxin and is linked to said TAT domain via a linker having the amino acid sequence denoted by SEQ ID NO. 32, and wherein said frataxin is C-terminal to said MTS of human lipoamide dehydrogenase.

11. A pharmaceutical composition comprising a pharmaceutically acceptable carrier or a physiologically acceptable carrier and, as an active ingredient, a fusion protein according to any one of claims 1 to 10.

12. A fusion protein according to any one of claims 1 to 10, or a pharmaceutical composition according to claim 11 for use as a medicament.

13. A fusion protein according to any one of claims 1 to 10, or a pharmaceutical composition according to claim 11, for use in treating or alleviating Friedreich's ataxia.

14. The fusion protein for use according to claim 12 or claim 13, or the pharmaceutical composition for use according to claim 12 or claim 13, wherein said fusion protein or pharmaceutical composition is for intravenous administration to said subject.

15. The fusion protein for use according to any one of claims 12 to 14, or the pharmaceutical composition for use according to any one of claims 12 to 14, wherein said fusion protein is administered in a dosage of from 0.5 mg/Kg to 2 mg/Kg body weight of said subject.

16. The fusion protein for use according to any one of claims 12 to 15, or the pharmaceutical composition for use according to any one of claims 12 to 15, wherein at least 5%, at least 10%, at least 20%, at least 30%, at least 40%, at least 50%, at least 60%, at least 70%, at least 80%, at least 90% or up to 100% of the activity of a wild type human mitochondrial protein is restored, or wherein oxidative stress is alleviated in a subject in need thereof.

## Patentansprüche

1. Fusionsprotein, das eine HIV-1-Transaktivator-der-Transkriptionsdomäne (TAT), ein funktionelles humanes mitochondriales Protein und eine humane Mitochondrien-Targeting-Sequenz (MTS), die sich zwischen der TAT-Domäne und dem funktionellen humanen mitochondrialen Protein befindet, umfasst, und wobei die humane MTS zu dem funktionellen humanen mitochondrialen Protein heterolog ist und wobei das funktionelle humane mitochondriale Protein Frataxin ist.

2. Fusionsprotein nach Anspruch 1, wobei das funktionelle humane mitochondriale Protein zu der humanen MTS C-terminal ist.

3. Fusionsprotein nach Anspruch 1 oder 2, wobei das mitochondriale Protein *per se* ein funktionelles humanes mitochondriales Protein ist und/oder eine Komponente eines mitochondrialen Multikomponentenkomplexes ist.

4. Fusionsprotein nach einem der vorhergehenden Ansprüche, wobei die MTS von 15 bis 40 Aminosäurereste umfasst, die von 3 bis 5 nicht aufeinanderfolgende basische Aminosäurereste beinhalten.

5. Fusionsprotein nach Anspruch 4, wobei die MTS von 1 bis 3 oder 4 oder 5 Serin-/Threoninreste umfasst.

6. Fusionsprotein nach einem der vorhergehenden Ansprüche, wobei die MTS eine beliebige von einer humanen mitochondrialen Citratsynthase-MTS mit der mit SEQ ID Nr. 23 bezeichneten Aminosäuresequenz, der humanen Lipoamiddehydrogenase-MTS mit der mit SEQ ID Nr. 24 bezeichneten Aminosäuresequenz, der MTS des humanen C60RF66-Genprodukts mit der mit SEQ ID Nr. 25 bezeichneten Aminosäuresequenz und der MTS von humaner mitochondrialer GLUD2, die von der mit SEQ ID Nr. 16 bezeichneten Nukleinsäure kodiert wird, ist.

7. Fusionsprotein nach Anspruch 6, wobei die MTS eine beliebige von einer humanen mitochondrialen Citratsynthase-MTS mit der mit SEQ ID Nr. 23 bezeichneten Aminosäuresequenz und der humanen Lipoamiddehydrogenase-MTS mit der mit SEQ ID Nr. 24 bezeichneten Aminosäuresequenz ist.

8. Fusionsprotein nach einem der vorhergehenden Ansprüche, das weiterhin einen Linker umfasst, der die TAT-Domäne kovalent mit der MTS-Sequenz verknüpft.

9. Fusionsprotein nach Anspruch 1 mit der mit SEQ ID Nr. 28 bezeichneten Aminosäuresequenz, das eine HIV-1-Transaktivator-der-Transkriptionsdomäne (TAT) mit der mit SEQ ID Nr. 27 bezeichneten Aminosäuresequenz, die mit humanem Frataxin mit der mit SEQ ID Nr. 26 bezeichneten Aminosäuresequenz fusioniert ist, und eine Mitochondrien-Targeting-Sequenz (MTS) von humaner Citratsynthase mit der mit SEQ ID Nr. 23 bezeichneten Aminosäuresequenz umfasst, wobei die MTS sich zwischen der TAT-Domäne und dem Frataxin befindet und mittels eines Linkers mit der mit SEQ ID Nr. 32 bezeichneten Aminosäuresequenz mit der TAT-Domäne verknüpft ist und wobei das Frataxin zu der MTS von humaner Citratsynthase C-terminal ist.

10. Fusionsprotein nach Anspruch 1 mit der mit SEQ ID Nr. 30 bezeichneten Aminosäuresequenz, das eine HIV-1-Transaktivator-der-Transkriptionsdomäne (TAT) mit der mit SEQ ID Nr. 27 bezeichneten Aminosäuresequenz, die mit humanem Frataxin mit der mit SEQ ID Nr. 26 bezeichneten Aminosäuresequenz fusioniert ist, und eine Mitochondrien-Targeting-Sequenz (MTS) von humaner Lipoamiddehydrogenase mit der mit SEQ ID Nr. 24 bezeichneten Aminosäuresequenz umfasst, wobei die MTS sich zwischen der TAT-Domäne und dem Frataxin befindet und mittels eines Linkers mit der mit SEQ ID Nr. 32 bezeichneten Aminosäuresequenz mit der TAT-Domäne verknüpft ist und wobei das Frataxin zu der MTS von humaner Lipoamiddehydrogenase C-terminal ist.

11. Pharmazeutische Zusammensetzung, die einen pharmazeutisch akzeptablen Trägerstoff oder einen physiologisch akzeptablen Trägerstoff und als einen Wirkbestandteil ein Fusionsprotein nach einem der Ansprüche 1 bis 10 umfasst.

12. Fusionsprotein nach einem der Ansprüche 1 bis 10 oder pharmazeutische Zusammensetzung nach Anspruch 11 zur Verwendung als ein Arzneimittel.

13. Fusionsprotein nach einem der Ansprüche 1 bis 10 oder pharmazeutische Zusammensetzung nach Anspruch 11 zur Verwendung beim Behandeln oder Lindern von Friedreich-Ataxie.

14. Fusionsprotein zur Verwendung nach Anspruch 12 oder 13 oder pharmazeutische Zusammensetzung zur Verwendung nach Anspruch 12 oder 13, wobei das Fusionsprotein oder die pharmazeutische Zusammensetzung zur intravenösen Verabreichung an den Probanden ist.

15. Fusionsprotein zur Verwendung nach einem der Ansprüche 12 bis 14 oder pharmazeutische Zusammensetzung zur Verwendung nach einem der Ansprüche 12 bis 14, wobei das Fusionsprotein in einer Dosierung von 0,5 mg/kg bis 2 mg/kg Körpergewicht des Probanden verabreicht wird.

16. Fusionsprotein zur Verwendung nach einem der Ansprüche 12 bis 15 oder pharmazeutische Zusammensetzung zur Verwendung nach einem der Ansprüche 12 bis 15, wobei mindestens 5 %, mindestens 10 %, mindestens 20 %, mindestens 30 %, mindestens 40 %, mindestens 50 %, mindestens 60 %, mindestens 70 %, mindestens 80 %, mindestens 90 % oder bis zu 100 % der Aktivität eines humanen mitochondrialen Wildtyp-Proteins wiederhergestellt wird oder wobei oxidativer Stress bei einem Probanden gelindert wird, der dieser Linderung bedarf.

## Revendications

1. Protéine de fusion comprenant un domaine de transactivation transcriptionnelle (TAT) du HIV-1, une protéine mitochondriale humaine fonctionnelle et une séquence de ciblage mitochondrial humaine (MTS) située entre ledit domaine TAT et ladite protéine mitochondriale humaine fonctionnelle et dans laquelle ladite séquence MTS humaine est hétérologue à ladite protéine mitochondriale humaine fonctionnelle, et dans laquelle ladite protéine mitochondriale humaine fonctionnelle est la frataxine.

2. Protéine de fusion selon la revendication 1, dans laquelle ladite protéine mitochondriale humaine fonctionnelle se trouve en position C-terminale par rapport à ladite séquence MTS humaine.

3. Protéine de fusion selon la revendication 1 ou la revendication 2, dans laquelle ladite protéine mitochondriale est une protéine mitochondriale humaine fonctionnelle en elle-même et/ou est un composant d'un complexe multicomposants mitochondrial.

4. Protéine de fusion selon l'une quelconque des revendications précédentes, dans laquelle ladite séquence MTS comprend de 15 à 40 résidus acides aminés, parmi lesquels de 3 à 5 résidus acides aminés basiques non consécutifs.

5. Protéine de fusion selon la revendication 4, dans laquelle ladite séquence MTS comprend de 1 à 3 ou 4 ou 5 résidus sérine/thréonine.

6. Protéine de fusion selon l'une quelconque des revendications précédentes, dans laquelle ladite séquence MTS est l'une quelconque parmi la séquence MTS de la citrate synthase mitochondriale humaine ayant la séquence d'acides aminés désignée par la SEQ ID N° 23, la séquence MTS de la lipoamide déshydrogénase humaine ayant la séquence d'acides aminés désignée par la SEQ ID N° 24, la séquence MTS du produit génique humain C60RF66 ayant la séquence d'acides aminés désignée par la SEQ ID N° 25, et la séquence MTS de la GLUD2 mitochondriale humaine codée par l'acide nucléique désigné par la SEQ ID N° 16.

7. Protéine de fusion selon la revendication 6, dans laquelle ladite séquence MTS est l'une quelconque parmi la séquence MTS de la citrate synthase mitochondriale humaine ayant la séquence d'acides aminés désignée par la SEQ ID N° 23 et la séquence MTS de la lipoamide déshydrogénase humaine ayant la séquence d'acides aminés désignée par la SEQ ID N° 24.

8. Protéine de fusion selon l'une quelconque des revendications précédentes, comprenant en outre un segment de liaison qui lie de manière covalente ledit domaine TAT à ladite séquence MTS.

9. Protéine de fusion selon la revendication 1, ayant la séquence d'acides aminés désignée par la SEQ ID N° 28, comprenant un domaine de transactivation transcriptionnelle (TAT) du HIV-1 ayant la séquence d'acides aminés désignée par la SEQ ID N° 27 fusionné à de la frataxine humaine ayant la séquence d'acides aminés désignée par la SEQ ID N° 26 et à une séquence de ciblage mitochondrial (MTS) de la citrate synthase humaine ayant la séquence d'acides aminés désignée par la SEQ ID N° 23, ladite séquence MTS étant située entre ledit domaine TAT et ladite frataxine et étant liée audit domaine TAT par l'intermédiaire d'un segment de liaison ayant la séquence d'acides aminés désignée par la SEQ ID N° 32, et dans laquelle ladite frataxine se trouve en position C-terminale par rapport à ladite séquence MTS de la citrate synthase humaine.

10. Protéine de fusion selon la revendication 1, ayant la séquence d'acides aminés désignée par la SEQ ID N° 30, comprenant un domaine de transactivation transcriptionnelle (TAT) du HIV-1 ayant la séquence d'acides aminés désignée par la SEQ ID N° 27 fusionné à de la frataxine humaine ayant la séquence d'acides aminés désignée par la SEQ ID N° 26 et à une séquence de ciblage mitochondrial (MTS) de la lipoamide déshydrogénase humaine ayant la séquence d'acides aminés désignée par la SEQ ID N° 24, ladite séquence MTS étant située entre ledit domaine TAT et ladite frataxine et étant liée audit domaine TAT par l'intermédiaire d'un segment de liaison ayant la séquence d'acides aminés désignée par la SEQ ID N° 32, et dans laquelle ladite frataxine se trouve en position C-terminale par rapport à ladite séquence MTS de la lipoamide déshydrogénase humaine.

11. Composition pharmaceutique comprenant un véhicule acceptable au plan pharmaceutique ou un véhicule acceptable au plan physiologique et, en tant que substance active, une protéine de fusion selon l'une quelconque des revendications 1 à 10.

12. Protéine de fusion selon l'une quelconque des revendications 1 à 10, ou composition pharmaceutique selon la revendication 11, destinée à être utilisée comme médicament.

13. Protéine de fusion selon l'une quelconque des revendications 1 à 10, ou composition pharmaceutique selon la revendication 11, destinée à être utilisée pour traiter ou atténuer l'ataxie de Friedreich.

14. Protéine de fusion destinée à être utilisée selon la revendication 12 ou la revendication 13, ou composition pharmaceutique destinée à être utilisée selon la revendication 12 ou la revendication 13, dans laquelle ladite protéine de fusion ou ladite composition pharmaceutique est destinée à être administrée par voie intraveineuse audit sujet.

15. Protéine de fusion destinée à être utilisée selon l'une quelconque des revendications 12 à 14, ou composition pharmaceutique destinée à être utilisée selon l'une quelconque des revendications 12 à 14, dans laquelle ladite protéine de fusion est administrée à un dosage allant de 0,5 mg/kg à 2 mg/kg de masse corporelle dudit sujet.

16. Protéine de fusion destinée à être utilisée selon l'une quelconque des revendications 12 à 15, ou composition pharmaceutique destinée à être utilisée selon l'une quelconque des revendications 12 à 15, dans laquelle au moins 5 %, au moins 10 %, au moins 20 %, au moins 30 %, au moins 40 %, au moins 50 %, au moins 60 %, au moins 70 %, au moins 80 %, au moins 90 % ou jusqu'à 100 % de l'activité d'une protéine mitochondriale humaine de type sauvage est rétablie ou dans laquelle le stress oxydatif est atténué chez un sujet qui en a besoin.
